# EUROPEAN PATENT SPECIFICATION

(11) **EP 4 407 012 B1**
(45) Date of publication and mention of the grant of the patent: **10.06.2026**
(21) Application number: 24150237.6
(22) Date of filing: 03.01.2024
(51) Int. Cl.: C09K 9/02, C08G 61/12, C08L 65/00, C09D 165/00, C09K 11/06, G02F 1/1516, C07D 333/16, C07D 333/18, C07D 495/04, C07D 409/14

(54) **ELECTROCHROMIC DEVICE CONTAINING HIGH TRANSPARENCY ELECTROCHROMIC POLYMERS**
ELECTROCHROME VORRICHTUNG ENTHALTEND ELEKTROCHROME POLYMERE MIT HOHER TRANSPARENZ
DISPOSITIF ÉLECTROCHROMIQUE CONTENANT DES POLYMÈRES ÉLECTROCHROMES À TRANSPARENCE ÉLEVÉE

(30) Priority: 04.01.2023 US 202318093287; 24.12.2023 US 202318395603
(43) Date of publication of application: 31.07.2024
(73) Proprietor: Ambilight Inc, Grand Cayman, KY1-1003 (KY)
(72) Inventor: Mei, Jianguo, West Lafayette, 47906 (US); Wang, Zhiyang, West Lafayette, 47906 (US); Pandit, Vaidehi, Woburn, 01801 (US); You, Liyan, West Lafayette, 47906 (US)
(74) Representative: Marks & Clerk LLP

(56) References cited:
- HSIAO SHENG-HUEI ET AL: "Fluorescent and electrochromic polymers from 2,8-di(carbazol-9-yl)dibenzothiophene and itsS,S-dioxide derivative", DYES AND PIGMENTS, ELSEVIER APPLIED SCIENCE PUBLISHERS BARKING, GB, vol. 134, 29 June 2016 (2016-06-29), pages 51 - 63, XP029708707, ISSN: 0143-7208, DOI: 10.1016/J.DYEPIG.2016.06.043
- JEONG DONG-CHEOL ET AL: "Effects of Substituent on Binaphthyl Hinge-Containing Conductive Polymers", MACROMOLECULES, vol. 45, no. 24, 10 December 2012 (2012-12-10), US, pages 9571 - 9578, XP093073488, ISSN: 0024-9297, DOI: 10.1021/ma301899e
- LIU JIAN ET AL: "Electrochromic polymer achieving synchronous electrofluorochromic switching for optoelectronic application", ORGANIC ELECTRONICS, vol. 51, 18 September 2017 (2017-09-18), pages 295 - 303, XP085320386, ISSN: 1566-1199, DOI: 10.1016/J.ORGEL.2017.09.022
- KOCAEREN AYSEL AYDIN: "Synthesis and electrochromic performance of a novel polymer based on an oxidative polymer derived from carbazole and thiophene", JOURNAL OF POLYMER RESEARCH, SPRINGER NETHERLANDS, DORDRECHT, vol. 23, no. 4, 8 March 2016 (2016-03-08), pages 1 - 10, XP035643812, ISSN: 1022-9760, [retrieved on 20160308], DOI: 10.1007/S10965-016-0961-1
- KOCAEREN AYSEL AYDIN: "Substituent and heteroatom effects on the electrochromic properties of similar systems", JOURNAL OF POLYMER SCIENCE PART A: POLYMER CHEMISTRY, vol. 50, no. 4, 28 October 2011 (2011-10-28), pages 615 - 621, XP055194200, ISSN: 0887-624X, DOI: 10.1002/pola.25047

## Description

### CROSS-REFERENCE TO RELATED APPLICATIONS

This application is a Continuation-In-Part application of Non-Provisional Application No. 18/093,287, filed on January 4, 2023, which is a Continuation-In-Part application of Non-Provisional Application No. 17/748,383, filed on May 19, 2022, which is a Continuation-In-Part application of Non-Provisional Application No. 17/668,300, filed on February 9, 2022.

### TECHNICAL FIELD

The present disclosure is related to a new type of electrochromic polymers that comprise meta-conjugated linkers and aromatic moieties, which present a high transparency in the visible light region in the neutral state. The polymers become highly absorbing in the visible light and near-infrared region and thus colored when their films are being oxidized. A device incorporating such conjugated electrochromic polymer films with a high optical contrast and a high transmittance is also disclosed.

### BACKGROUND

Electrochromic devices allow to adjust light transmittance and control solar-heat gain. In comparison with inorganic-based electrochromic devices made through the vacuum sputtering process, polymer-based electrochromic windows can be manufactured through roll-to-roll coating and lamination. It thus renders a low-cost production and manufacturing flexibility. Polymer based electrochromic devices are typically composed of conjugated electrochromic polymers (ECPs), which feature fully conjugated polymer backbone made of sp² hybridized carbons. Conventionally, ECPs typically have strong absorbance in the visible light region and are thus colored in their neutral state. When they are oxidized, their absorption is shifted toward near-infrared (near-IR) region and they become transmissive in the visible light region. However, the oxidized polymers still have weak absorption in the visible light region, leading to residue colors. The problem becomes more severe when the polymer films are thick. As a result, it negatively impacts optical contrast of the polymers. Furthermore, it limits the highest optical transmittance a conjugated electrochromic polymer can achieve. In addition, conventional ECPs in the neutral state blocks visible light through the film and allow near-IR light passing through; While in the transmissive state, it allows visible light passing through and blocks near-IR light. This combination is not effective for thermal management and control the solar-heat gain (SHG). SHG describes the way radiation from the sun is turned into heat through a window product. HSIAO SHENG-HUEI ET AL: "Fluorescent and electrochromic polymers from 2,8-di(carbozal-9-yl)dibenzothiophene and its S,S-dioxide derivative", DYES AND PIGMENTS, ELSEVIER APPLIED SCIENCE PUBLISHERS, vol. 134, 29 June 2016, pages 51-63, XP029708707, reports the synthesis and characterization of two carbazole-endcapped monomers and their derived polymers PSCz and PSO₂Cz. Dilute solutions of PSCz and PSO₂Cz prepared by chemical oxidative coupling exhibited fluorescent and solvatochromic behaviour.

### SUMMARY

The present disclosure is related to a new type of electrochromic polymer and the devices that uses the polymer.

In one aspect, an electrochromic device is provided. The electrochromic device includes: a first insulating substrate; a first conducting layer disposed over the first insulating substrate; an electrochromic layer disposed over the first conducting layer, wherein the electrochromic layer comprises an electrochromic polymer having a polymer backbone comprising one or more meta-conjugated linkers (MCLs) and one or more aromatic moieties (Ars), wherein each of the one or more MCLs is partially conjugated with one of the one or more Ars at a meta position of the one or more MCLs; an electrolyte layer disposed over the electrochromic layer; a second conducting layer disposed over the electrolyte layer; and a second insulating substrate disposed over the second conducting layer. A thickness of the electrochromic layer is from 10 nm to 1500 nm resulting in transmittance of 85%-99.9% at a wavelength of 550 nm at a neutral state of the electrochromic layer. For example, a thickness of the electrochromic layer 106 from 10 nm to 1500 nm yields a transmittance of 85%, 87%, 90%, 92%, 95%, 96%, 97%, 98%, 99%, 99.9% or between any two of the above numbers. The electrochromic device 100 has transmittance of 60% or more at a wavelength of 550 nm at a bleached state of the device. For example, by tuning the material and thickness of the electrochromic layer, the electrochromic device 100 may have at its bleached state transmittance of 60%, 65%, 70%,75%, 80%, 85%, 90%, 92%, 95%, 96%, 97%, 98%, or 99%, or between any two of the above numbers.

In some embodiments, the electrochromic layer has transmittance of 40%-0.1% at a wavelength of 550 nm at an oxidized state of the electrochromic layer. For example, the electrochromic layer at the oxidized state has transmittance at a wavelength of 550 nm of 40%, 35%, 30%, 25%, 20%, 15%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1%, or between any two of the above numbers.

In some embodiments, the electrochromic layer has an optical contrast of 60% or more. For example, the electrochromic layer may have an optical contrast of 60%, 65%, 70%, 75%, 80%, 85%, 90%, 95%, 96%, 97%, 98%, 99%, or between any two of the above numbers.

In some embodiments, when the electrochromic device further includes the ion storage layer 110, which is disposed between the electrolyte layer 108 and the second conducting layer, and the ion storage layer has transmittance of 80% or more at a wavelength of 550 nm. In some embodiments, the ion storage layer may include (1) one or more oxides of metal elements in Group 4-12, or (2) a mixture of the oxides, or (3) one of the oxides doped by a different metal oxide, or (4) a transition-metal complex, or (5) one or more of redox-active polymers including redox active nitroxyl, galvinoxyl radical polymers and conjugated polymers.

In some embodiments, the ion storage layer includes ITO particles, wherein the ion storage layer has transmittance of 90% or more at a wavelength of 550 nm. In some embodiments, the ITO particles may be nanoparticles having a size of 1-900 nm.

In some embodiments, at least one of the first conducting layer and the second conducting layer includes ITO, aluminum zinc oxide (AZO), fluorine doped tin oxide (FTO), silver nanowires, graphene, carbon nanotube, metal mesh based transparent conductive electrodes, silver-nanoparticle ink, or an organic conductive polymer.

In some embodiments, the electrochromic device has an optical contrast of 60% or more. For example, the electrochromic device may have an optical contrast of 60%, 65%, 70%, 75%, 80%, 85%, 90%, 95%, 96%, 97%, 98%, 99%, or between any two of the above numbers.

In some embodiments, a color of the electrochromic layer at an oxidized state is varied by varying/adjusting a conjugation length of the one or more MCLs and the one or more Ars.

In some embodiments, the electrochromic layer includes a blend of different electrochromic polymers without an intermediate color.

The electrochromic layer includes an electrochromic polymer. The electrochromic polymer consists of: a polymer backbone comprising one or more meta-conjugated linkers (MCLs) and one or more aromatic moieties (Ars). Each of the one or more MCLs is partially conjugated with one of the one or more Ars at a meta position of the one or more MCLs. A thickness of the electrochromic polymer is from 10 nm to 1500 nm resulting in transmittance of 85%-99.9% at a wavelength of 550 nm at a neutral state of the electrochromic layer. For example, a thickness of the electrochromic layer 106 from 10 nm to 1500 nm yields a transmittance of 85%, 87%, 90%, 92%, 95%, 96%, 97%, 98%, 99%, 99.9% or between any two of the above numbers.

In some embodiments, the electrochromic polymer disclosed in this application consists of a polymer backbone comprising one or more meta-conjugated linkers (MCLs) and one or more aromatic moieties (Ars). Each of the one or more MCLs is partially conjugated with the one or more Ars at meta positions of the one or more MCLs to form the polymer backbone of an electrochromic polymer. In some embodiments, the disclosed electrochromic polymer is anodically-coloring electrochromic polymer (AC-ECP), becoming colored when it is oxidized.

In some embodiments, the disclosed electrochromic polymer has an energy bandgap equal to or higher than 2.9 eV and less than 4.0 eV in the neutral state. In some embodiments, the absorption maxima ( 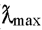 , the wavelength at which the polymer has its strongest photon absorption) are less than 410 nm in the neutral state. In some embodiments, the disclosed electrochromic polymer is colorless in the neutral state, while it is colored and visible and near-infrared absorbing in the oxidized state. The oxidized electrochromic polymer has an absorption coefficient larger than 10⁴ cm⁻¹ in the visible and/or near-IR region and thus colored in the oxidized state.

In spite of their high bandgaps, the disclosed electrochromic polymers still have relatively low oxidation potential in the ranges of 0.1-1.5 V inclusive versus Ag/AgCl electrode in some embodiments.

The MCL comprises at least one of an aromatic structure, or a fused aromatic structure, or the combinations thereof. The aromatic structure comprises a benzene or heterocyclic structure. The fused aromatic structure comprises a fused benzene structure or a fused heterocyclic structures or a fused benzene and heterocyclic structure.

In some embodiments, for the disclosed electrochromic polymers, the one or more MCLs and the one or more Ars are arranged in an alternative or random fashion with a general formula of 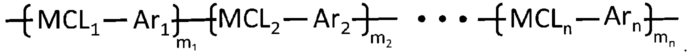 In the structures here, n is an integer greater than 0 and each of m₁, m₂, ... , mₙ is an integer equal to or greater than 0 with at least one of m₁, m₂, ... , mₙ is greater than 0. The one or more MCLs (or the one or more Ars) can be the same as or different from each other.

The one or more MCLs and corresponding meta-positions comprise one of the following formulas: 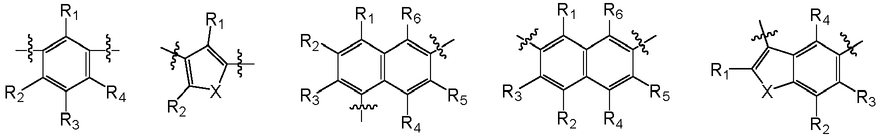 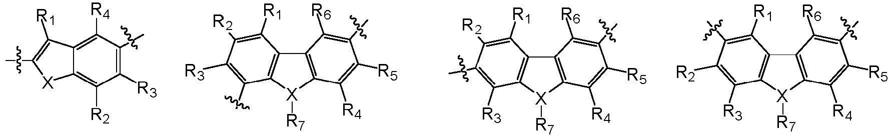 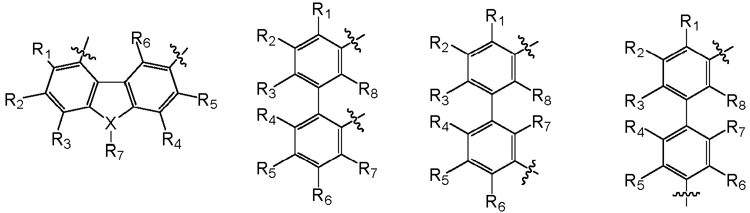 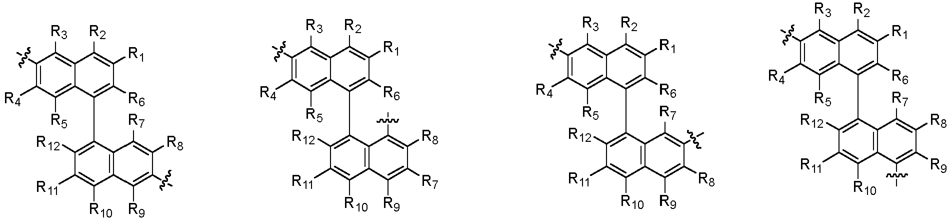 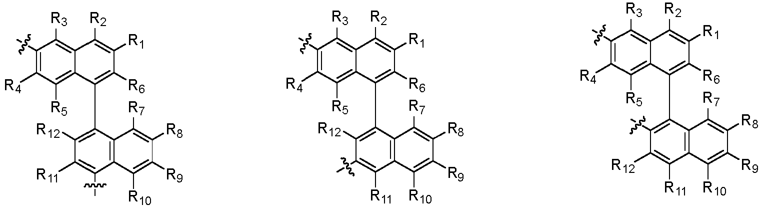 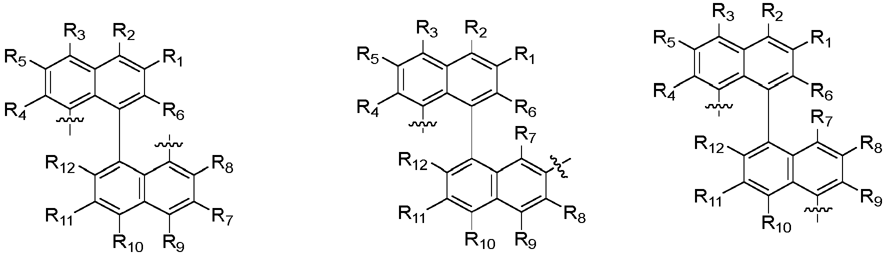 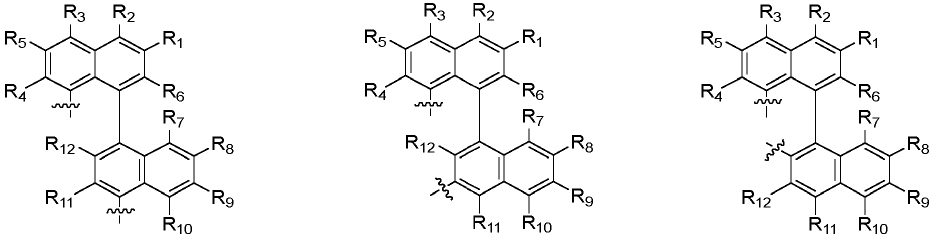 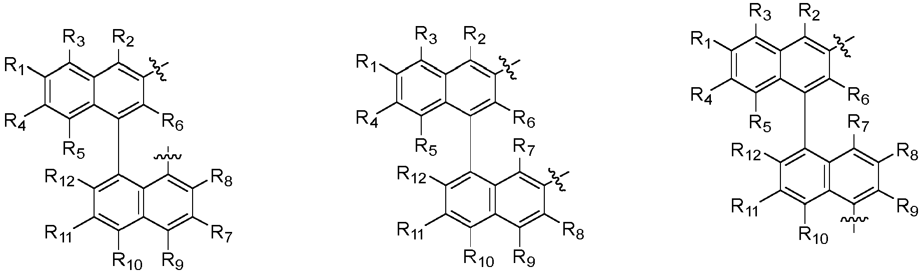 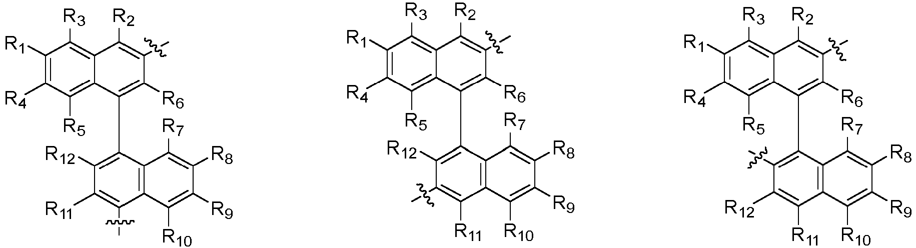 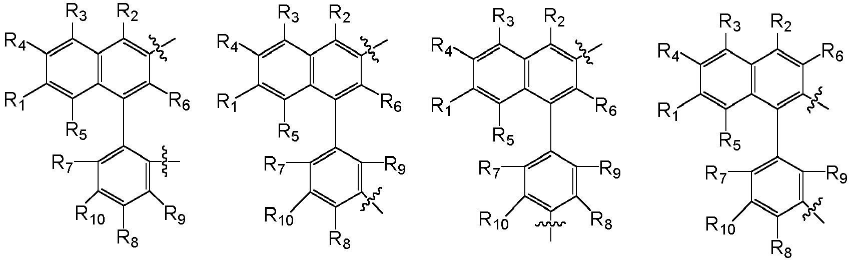 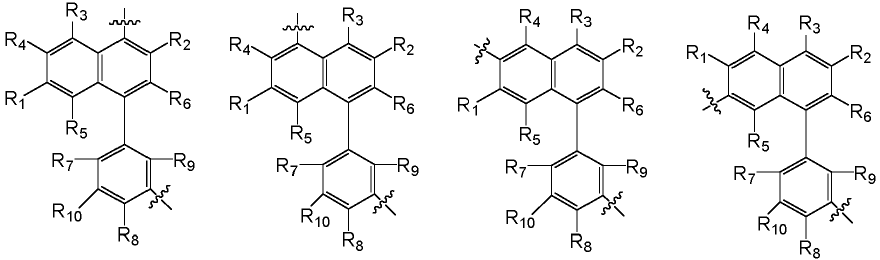 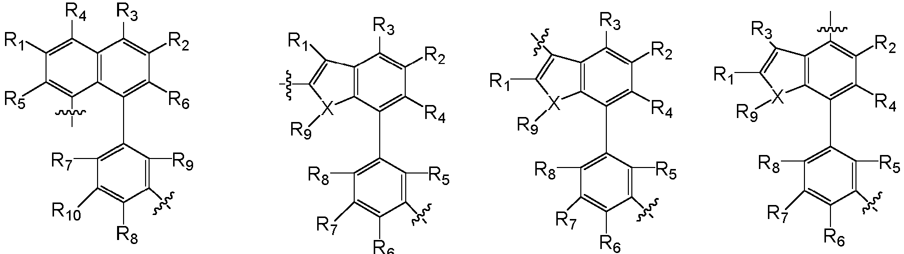 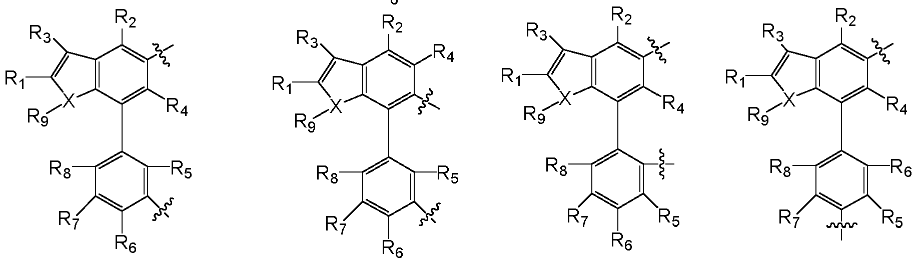 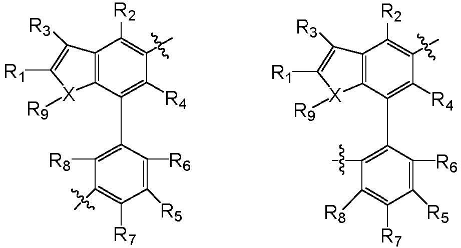 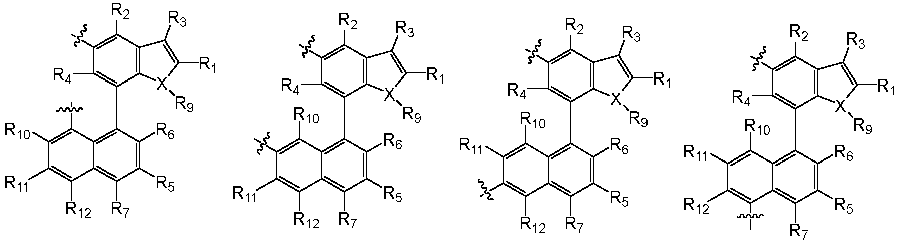 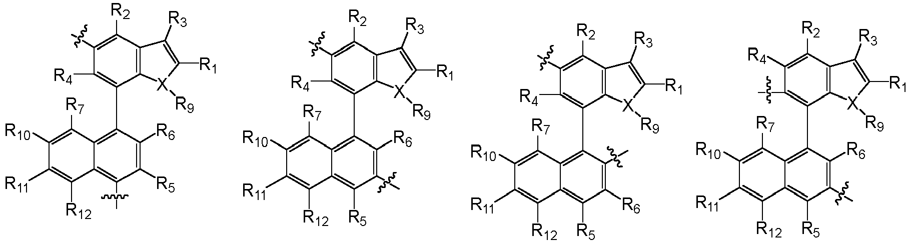 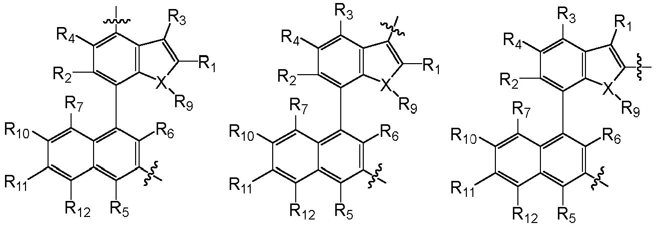 wherein each of the wavy lines represents meta-positions to link adjacent the one or more Ars; X is S, Se, N, C, or O; R₁ - R₁₂ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl.

The one or more Ars comprise one of a thiophene-based unit, a furan-based unit, a selenophene-based unit, or a pyrrole-based unit with a formula of 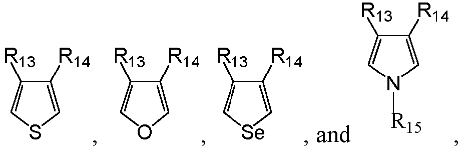 or any combination thereof, wherein each of R₁₃ , R₁₄ and R₁₅ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl.

In some embodiments, the thiophene-based unit comprises a formula of 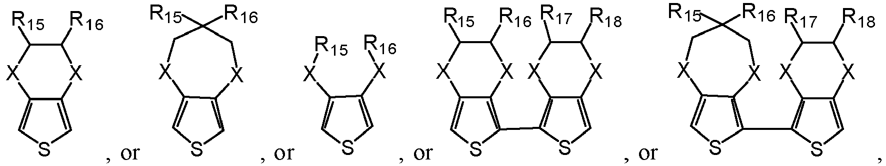 or 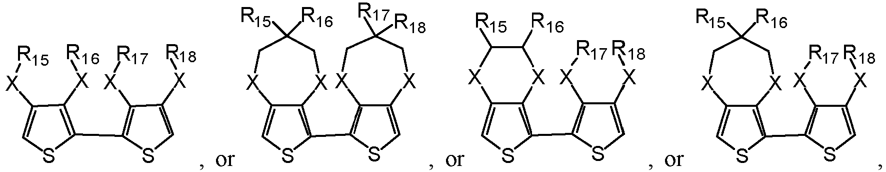 or 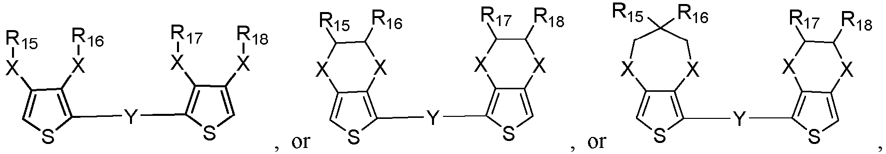 or 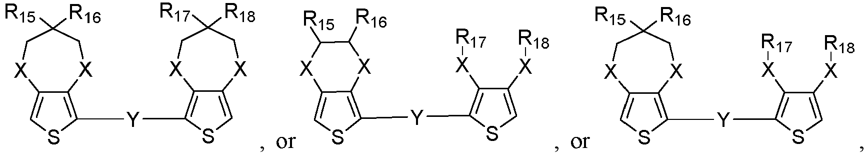 or a combination thereof,
wherein X is S, Se, N, C, or O; each of R₁₅ - R₁₈ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl. Y is any one or more of Ars, or aromatic structures, or fused aromatic structures, or a combinations thereof.

In some embodiments, X in the thiophene-based unit is O.

In some embodiments, the disclosed electrochromic polymers comprise a formula of 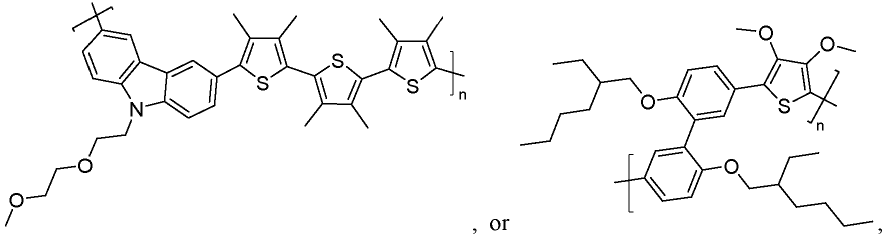 or 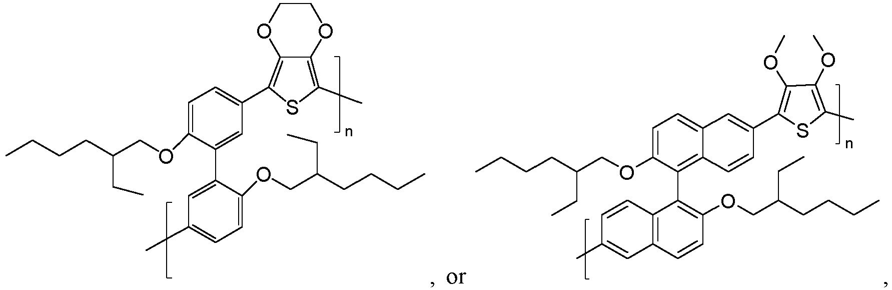 or 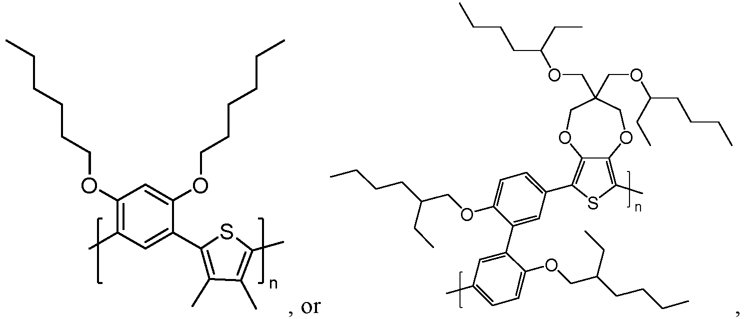 or 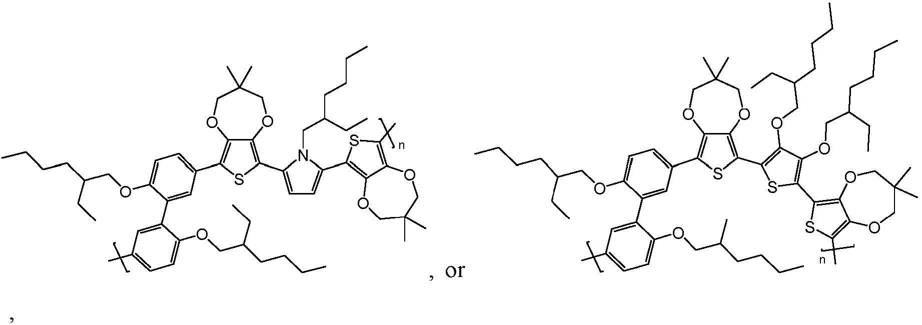 or 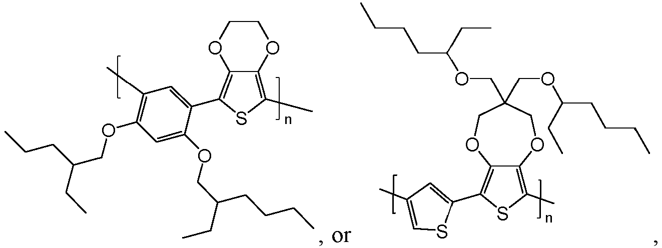 or 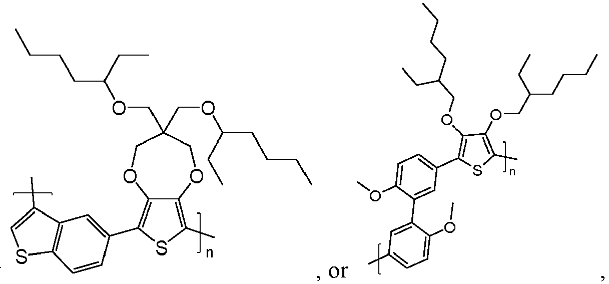 or 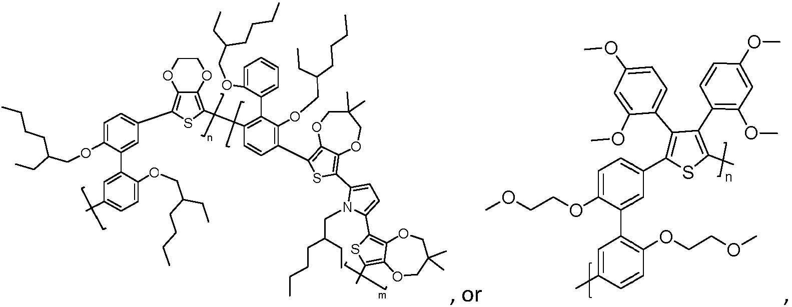 or 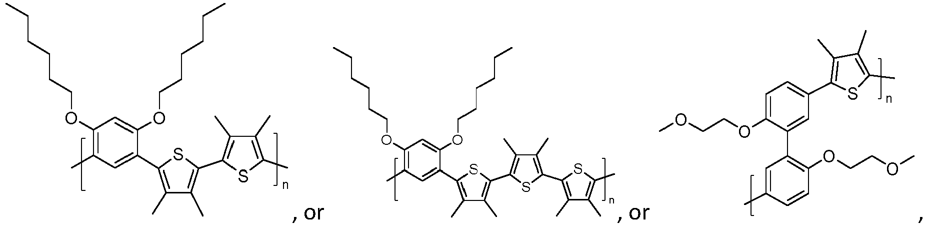 or 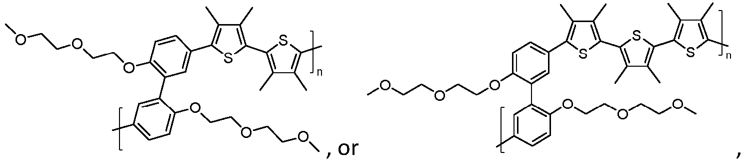 or 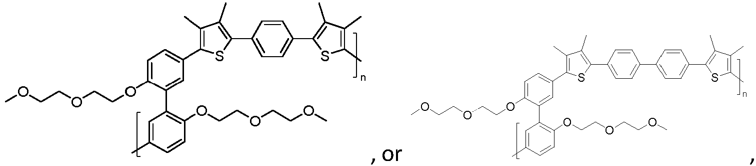 or 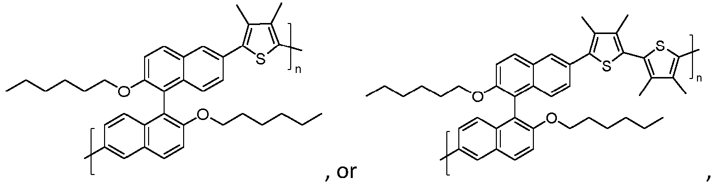 or 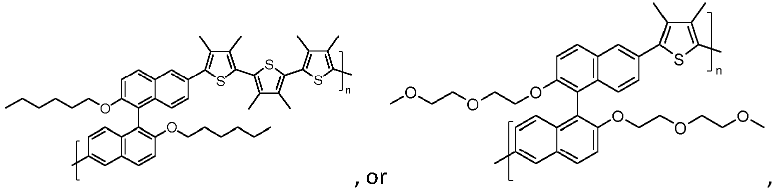 or 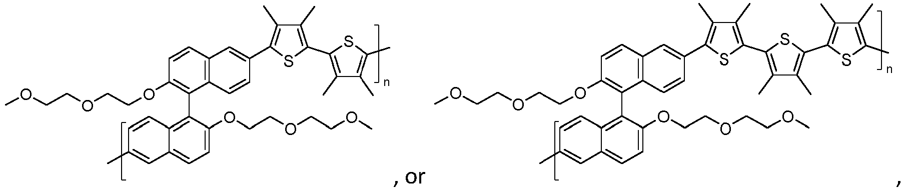 or 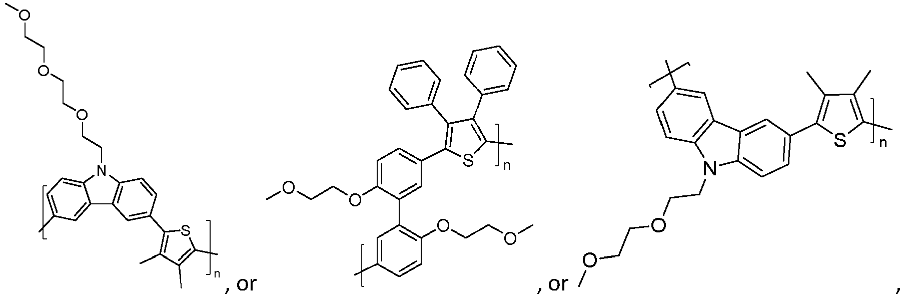 or 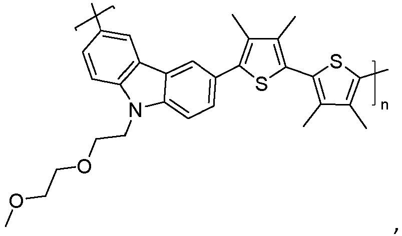 or 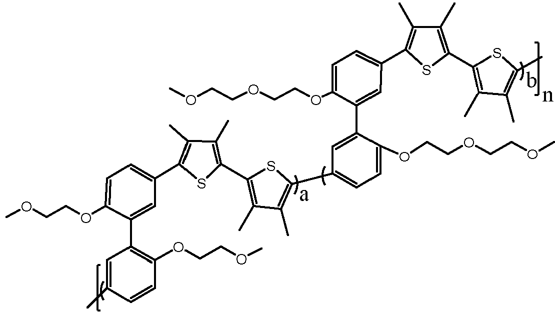 wherein n, and m are integers greater than 0, a and b are integers equal to or greater than 0 with at least one of a and b is greater than 0.

In some embodiments, the electrochromic layer has a transmittance of 40%-0.1% at a wavelength of 550 nm at an oxidized state of the electrochromic layer.

In some embodiments, the electrochromic layer has an optical contrast of 60% or more.

In some embodiments, each of the one or more MCLs and corresponding meta positions comprise one of the following formulas: 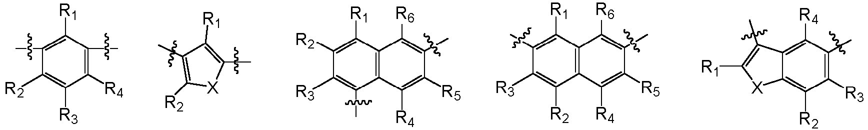 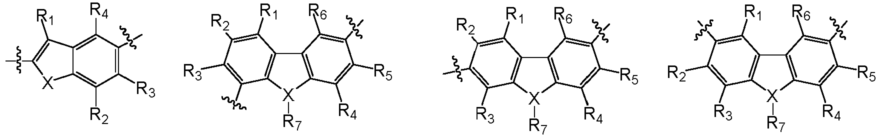 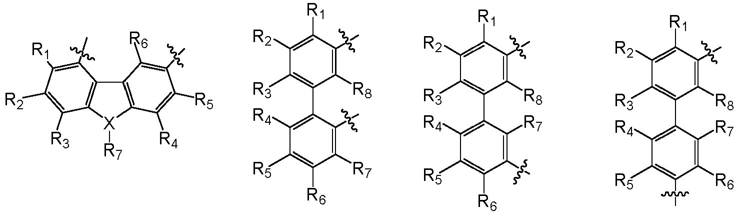 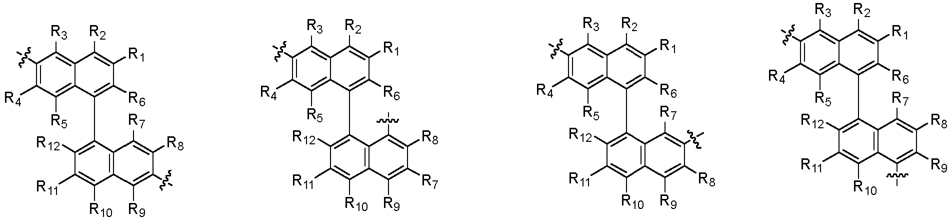 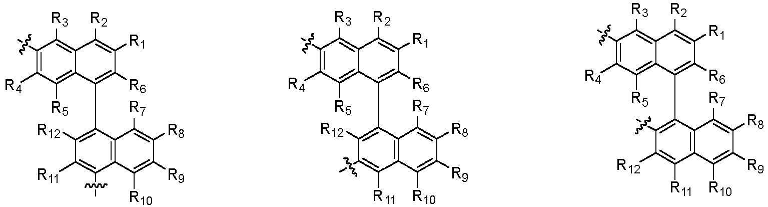 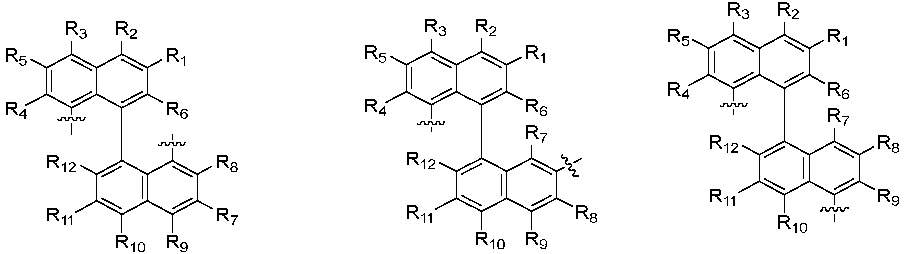 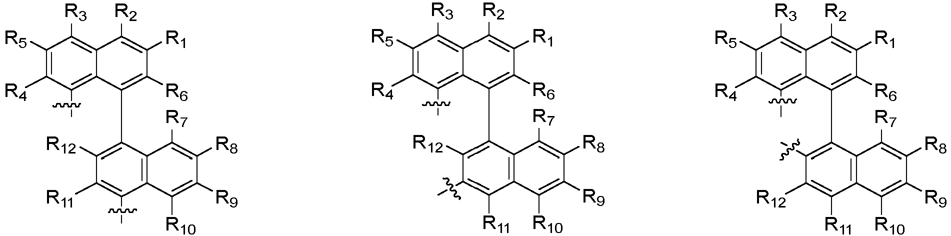 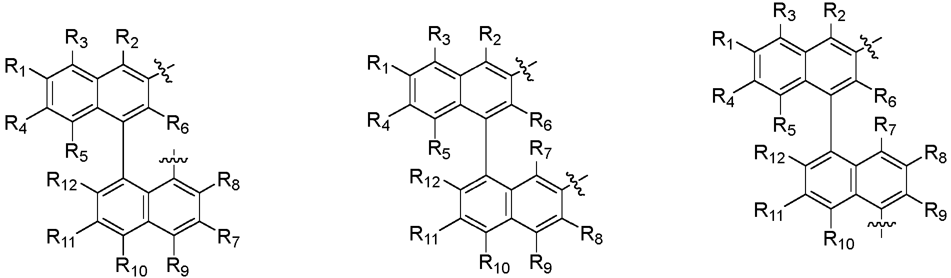 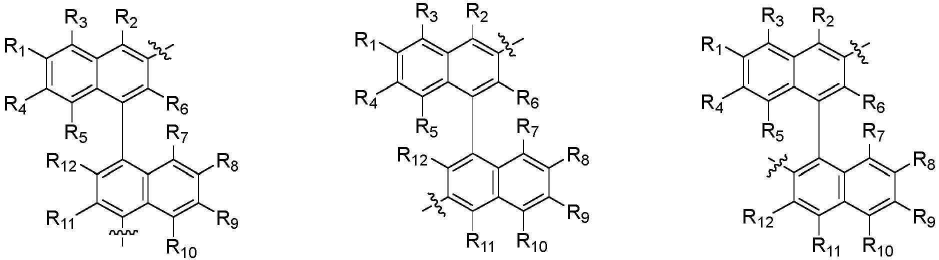 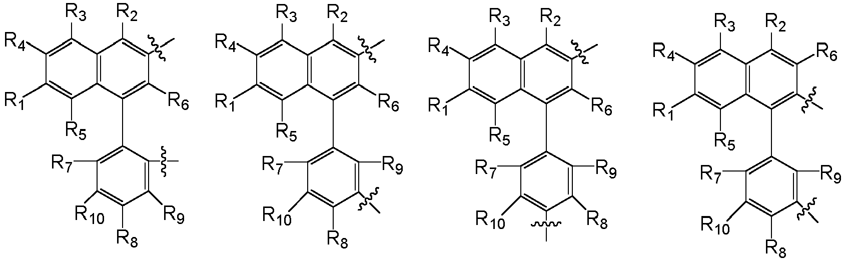 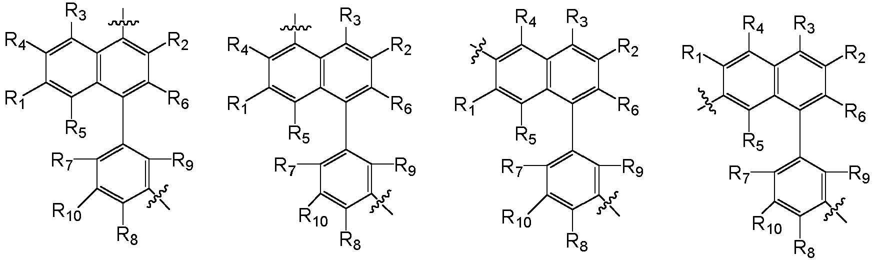 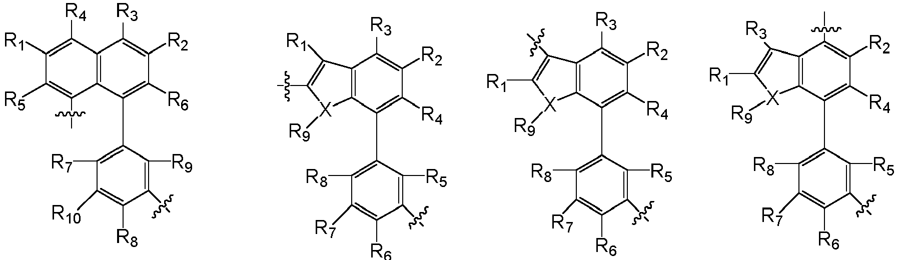 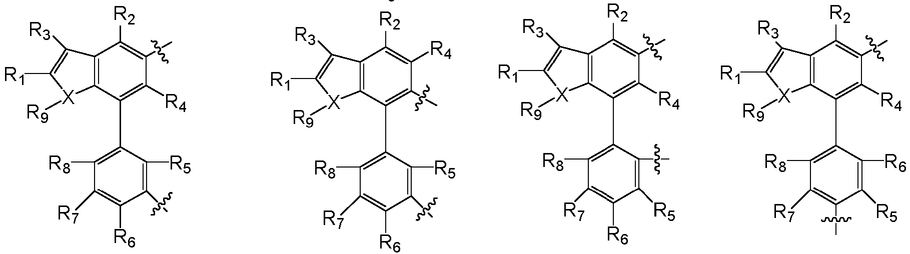 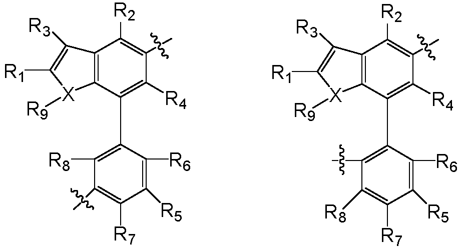 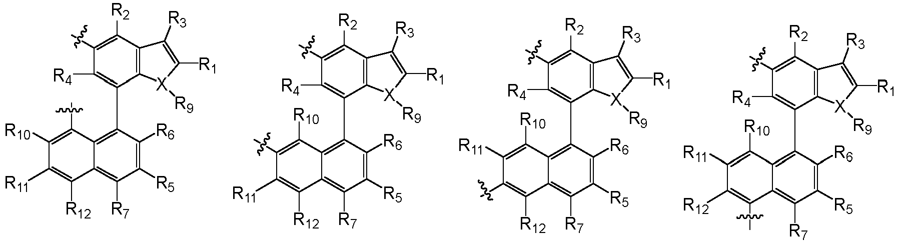 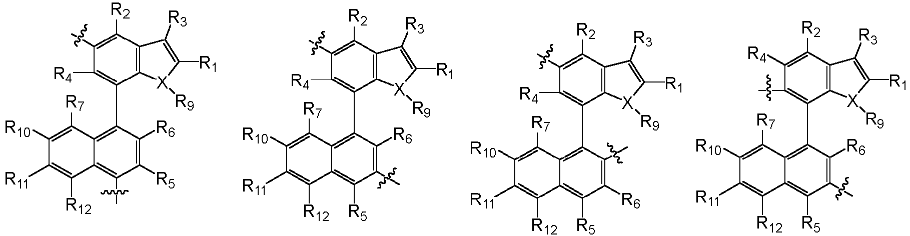 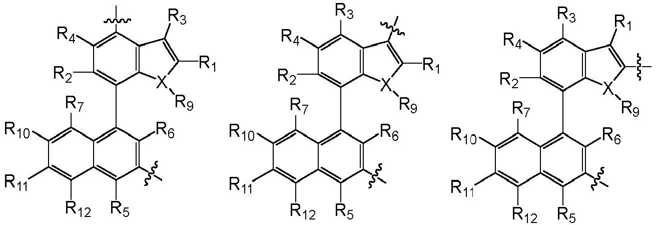 wherein X is S, Se, N, C, or O; each of R₁-R₁₂ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl; and each of the wavy lines represents one of the meta positions.

In some embodiments, wherein each of the one or more Ars comprises one of a thiophene-based unit, a furan-based unit, a selenophene-based unit, or a pyrrole-based unit with one of the following formulas: 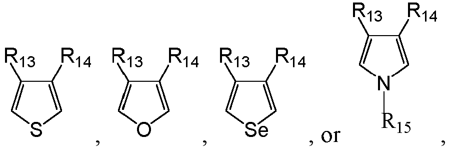 wherein each of R₁₃ ,R₁₄ and R₁₅ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl.

In some embodiments, the thiophene-based unit comprises one of the following formulas: 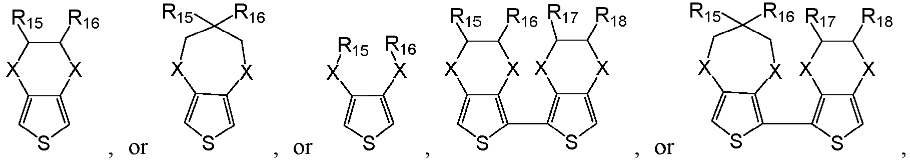 or 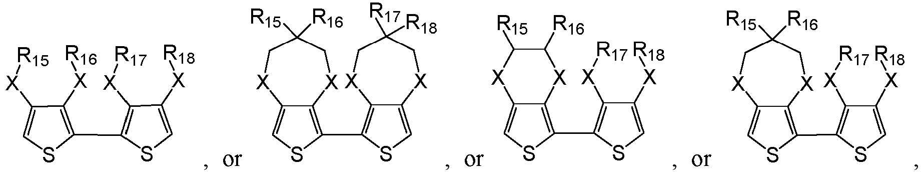 or 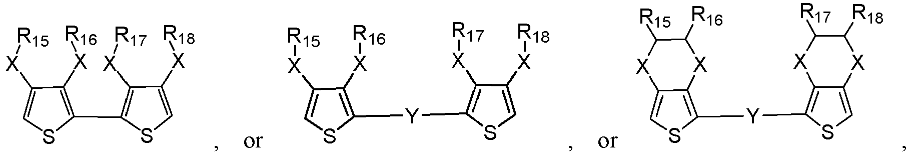 or 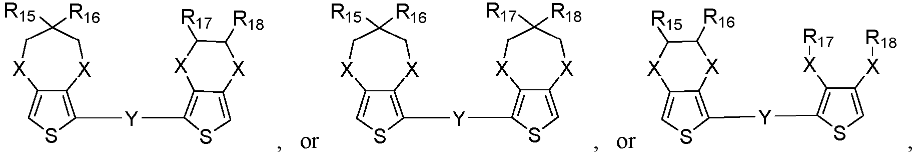 or 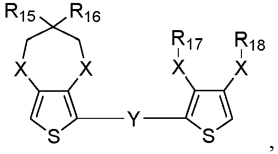 wherein X is S, Se, N, C, or O; each of R₁₅ - R₁₈ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl; Y is any one or more of Ars, or aromatic structures, or fused aromatic structures, or a combinations thereof.

### BRIEF DESCRIPTION OF THE DRAWINGS

Certain features of various embodiments of the present technology are set forth with particularity in the appended claims. A better understanding of the features and advantages of the technology will be obtained by reference to the following detailed description that sets forth illustrative embodiments, in which the principles of the invention are utilized, and the accompanying drawings below. For the purpose of illustrating the invention, the drawings show aspects of one or more embodiments of the invention. However, it should be understood that the present invention is not limited to the precise arrangements and instrumentalities shown in the drawings.
FIGS. 1(A)-(B) are diagrams representing the different color changing mechanisms of the disclosed ECP (FIG. 1(A)) compared to conventional ECP (FIG. 1(B)).
FIG. 2 is the CV data of an exemplary solid-state device using an example ECP-1, according to one embodiment.
FIG. 3 is the switching kinetics of the exemplary solid-state device using the example ECP-1 at 545 nm, according to one embodiment.
FIG. 4 are the absorbance spectra of the exemplary ECP-1 thin film at different voltages, according to one embodiment.
FIG. 5 is the CV data of an exemplary solid-state device using another example ECP-2, according to one embodiment.
FIG. 6 is the switching kinetics of the exemplary solid-state device using the example ECP-2 at 550nm, according to one embodiment.
FIG. 7 are the absorbance spectra of the exemplary ECP-2 thin film at different voltages, according to one embodiment.
FIG. 8(A) illustrates calculated neutral state UV-Vis spectra of the disclosed meta-conjugated polymers with three representative MCLs, according to some embodiments.
FIG. 8(B) illustrates calculated oxidized state UV-Vis spectra of the disclosed meta-conjugated polymers with three representative MCLs, according to some embodiments.
FIG. 9(A) illustrates absorption spectro-electrochemistry from neutral state transitioning to oxidized state of the CBZ-Blend with a film thickness of 300 nm, according to some embodiments. From the bottom line to the top line, the potential is respectively 0.6 v, 0.65 v, 0.7 v, 0.75 v, 0.8 v, 0.85 v, 0.9 v, 0.95 v, 1.0 v versus Ag/AgCl.
FIG. 9(B) illustrates Beer-Lambert plots of CBZ-Blend in the neutral state (dashed line) and oxidized state (solid) at 550 nm.
FIG. 9(C) illustrates the transmittance of CBZ-Blend at 550 nm in neutral and oxidized states as a function of film thickness, according to some embodiments. Experimental results are in dots and calculated results are in sloid and dashed lines.
FIG. 9(D) illustrates the transmittance of CBZ-Blend in both neutral and oxidized states of the EC layer at different thicknesses, according to some embodiments.
FIG. 9(E) illustrates the transmittance of CBZ-Blend in both neutral (bleached) and oxidized states of the EC layer under 10000 cycles, according to some embodiments.
FIG. 10 depicts a cross-section view of an electrochromic device, according to one example embodiment of the present disclosure.

### DETAILED DESCRIPTION OF EMBODIMENTS

In the following description, certain specific details are set forth in order to provide a thorough understanding of various embodiments of the invention. However, one skilled in the art will understand that the invention may be practiced without these details. Moreover, while various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way.

Unless the context requires otherwise, throughout the present specification and claims, the word "comprise" and variations thereof, such as "comprises" and "comprising" are to be construed in an open, inclusive sense, that is as "including, but not limited to." Recitation of numeric ranges of values throughout the specification is intended to serve as a shorthand notation of referring individually to each separate value falling within the range inclusive of the values defining the range, and each separate value is incorporated in the specification as it was individually recited herein. Additionally, the singular forms "a" "an", and "the" include plural referents unless the context clearly dictates otherwise.

Reference throughout this specification to "one embodiment" or "an embodiment" means that a particular feature, structure, or characteristic described in connection with the embodiment is included in at least one embodiment of the present invention. Thus, the appearances of the phrases "in one embodiment" or "in an embodiment" in various places throughout this specification are not necessarily all referring to the same embodiment, but maybe in some instances. Furthermore, the particular features, structures, or characteristics may be combined in any suitable manner in one or more embodiments.

The present disclosure is related to a new type of electrochromic polymers. The electrochromic polymer disclosed in this application consists of a polymer backbone comprising one or more meta-conjugated linkers (MCLs) and one or more aromatic moieties (Ars). Each of the one or more MCLs is partially conjugated with the one or more Ars at meta positions of the one or more MCLs to form the polymer backbone of an electrochromic polymer. In some embodiments, the electrochromic polymer disclosed in this application consists of a repeat unit comprising one or more MCLs and one or more Ars, where meta-conjugation is introduced along the polymer backbone through the use of the MCLs. In some embodiments, the electrochromic polymer is anodically-coloring electrochromic polymer (AC-ECP), becoming colored when it is oxidized.

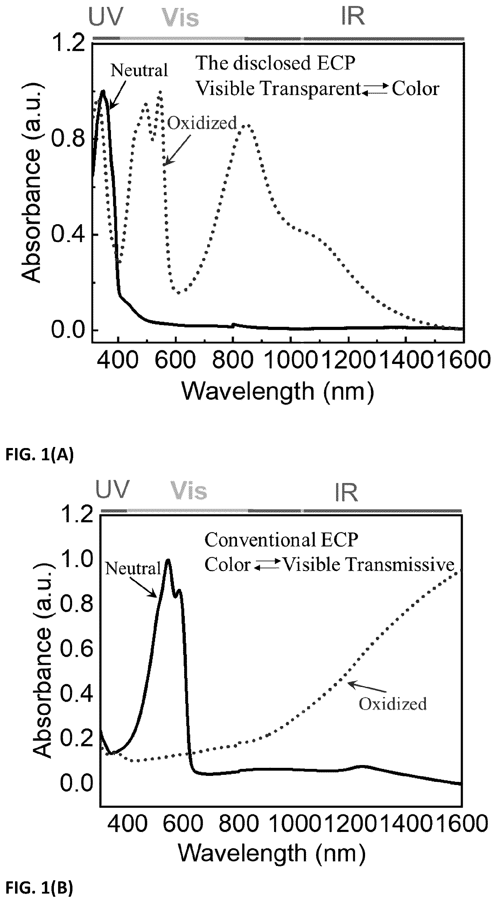

As illustrated in FIG. 1, conventional conjugated ECPs (FIG. 1(B)) are fully conjugated and have strong absorbance in the visible light region and are thus colored in their neutral state, while when oxidized (oxidized state), their absorption is shifted toward near-IR region and they become transmissive. However, the oxidized polymers still have weak absorption in the visible light region, leading to residue colors. On the other hand, as illustrated for one example disclosed ECP in FIG. 1(A), the ECP exhibits substantially no absorption after 450 nm in the neutral state and has several absorption peaks in visible light range and the near infrared range in the oxidized state, demonstrating coloring in the visible light range and near-infrared absorbing.

The disclosed electrochromic polymers allows passing or blocking of visible light and near-IR light to be synchronized, which is in one embodiment very useful in an electrochromic window for the management of solar heat gain. The disclosed electrochromic polymers are transparent in the neutral state, and are colored and IR-absorbing in the oxidized state, which are highly desired in order to achieve a high optical contrast, a high transmittance and a synergistic solar-heat gain.

The disclosed electrochromic polymers are transparent in the visible light region in the neutral state and are colored in the oxidized state. For example, the disclosed electrochromic polymers may have a transmittance of at least 60% in the visible light range (e.g., 450-750 nm) in the neutral state. In some embodiments, the disclosed electrochromic polymers may have a transmittance of at least 65%, 70%, 75%, 80%, 85%, 90%, 92%, 95%, 98%, or above in the range of 450-750 nm in the neutral state. In some embodiments, the disclosed electrochromic polymers are transparent in the visible light range in the neutral state. In the oxidized state, the disclosed electrochromic polymers have absorption in the visible light range (e.g., about 360 to 750 nm) and the near-IR range (e.g., about 750 to 1600 nanometers), thereby being colored and near-infrared absorbing.

The disclosed electrochromic polymer has UV absorption and energy bandgap. An energy bandgap is the energy difference between the valence band of electrons and the conduction band. It is the minimum change in energy required to excite an electron up to a state in the conduction band where it can participate in conduction. Absorption onset ( 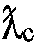 ) is the wavelength at higher than which the polymer has no photon absorption. The energy bandgap can be calculated as 1240/wavelength of absorption onset. In some embodiments, the electrochromic polymers disclosed in this application have an absorption onset at equal to or less than 450 nm in the neutral state. In some embodiments, the disclosed electrochromic polymer has an absorption onset at equal to or less than 440 nm, 430 nm, 420nm, 410nm, 405nm, or 400 nm in the neutral state. In some embodiments, the absorption maxima ( 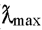 , the wavelength at which the polymer has its strongest photon absorption) are less than 420 nm in the neutral state. In some embodiments, the absorption maxima are less than 410 nm or 405 nm or 400 nm in the neutral state. In some embodiments, the disclosed electrochromic polymer has an energy bandgap equal to or higher than 2.8 eV and less than 4.0 eV in the neutral state. In some embodiments, the disclosed electrochromic polymer has an energy bandgap equal to or higher than 2.9, 3.0 or 3.1 eV and less than 4.0 eV in the neutral state. In some embodiments, the disclosed electrochromic polymer is colorless (e.g., no absorbance in 400-750 nm or 410-750 nm or 420-750 nm) or yellow (e.g., tailing absorption in 400-500 nm, or 410-500nm, or 420-500 nm or 400-480 nm, or 410-480 nm, or 420-480 nm or 400-450 nm, or 410-450 nm or 420-450 nm) in the neutral state and is colored and visible and near-IR absorbing in the oxidized state. The oxidized electrochromic polymer has an absorption coefficient larger than 10⁴ cm⁻¹ in the visible and/or near-IR region and thus colored in the oxidized state.

Due to substantial lack of absorbance in the visible light range in the neutral state and high absorbance in the visible light range in the oxidized state, the disclosed electrochromic polymers demonstrate high optical contrast and high optical transmittance when comparing with conventional ECPs. In spite of their high bandgaps, the disclosed electrochromic polymers have relatively low oxidation potential in the ranges of 0.1-1.5 V inclusive versus Ag/AgCl electrode in some embodiments. In some embodiments, the disclosed electrochromic polymers have low oxidation potential in the ranges of 0.1-1 V inclusive versus Ag/AgCl electrode. The relatively low oxidation potential can benefit cycling durability of ECPs. Thus, the disclosed electrochromic polymers can be successfully incorporated into a device with a good cycling stability/reliability and a high optical contrast.

The MCL comprises at least one of an aromatic structure, or a fused aromatic structure, or the combinations thereof. The aromatic structure comprises a benzene or heterocyclic structure. The fused aromatic structure comprises a fused benzene structure or a fused heterocyclic structures or a fused benzene and heterocyclic structure. In some embodiments, the MCL comprises at least one of benzene, or naphthalene, or five-membered heterocycle, or benzene fused five-membered heterocycle, or a combination of these structures. Side chains or aromatic side chains can also be introduced onto the MCL to adjust its performance, for example, solubility or processibility or stability.

In some embodiments, the one or more MCLs and the one or more Ars are arranged in an alternative or random fashion with a general formula of 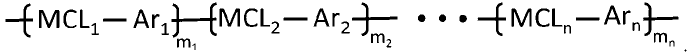 In the structure here, n is an integer higher than 0 and each of m₁, m₂, ... , mₙ is an integer equal to or higher than 0 with at least one of m₁, m₂, ... , mₙ is higher than 0. The one or more Ars are aromatic moieties, which may include one or more aromatic structures. Each of the one or more MCLs (or Ars) can be the same as or different from each other.

Meta-conjugation is introduced in the polymer backbone through the use of the one or more MCLs. Each of the one or more MCLs is partially conjugated in the polymer backbone by connecting with the one or more Ars through its meta-positions. For example, the meta-positions are two positions of the aromatic structure or a fused aromatic structure of the MCLs. When the meta-positions are connected, the pi electrons from an aromatic structure or a fused aromatic structure cannot be fully delocalized to another adjacently-connected unit through p-orbitals.

In some embodiments, an aromatic structure of the MCLs comprises a benzene structure or a five-membered heterocyclic structure, and the aromatic structure of the MCLs is substituted at meta-positions, which are the 1- and 3- positions on the aromatic structure. In some embodiments, a fused aromatic structure of the MCLs comprises naphthalene, and the fused aromatic structure is substituted at meta-positions, which are the 1- and 3-, or 1- and 4-, or 1- and 6- positions on naphthalene. In some embodiments, a fused aromatic structure of the MCLs comprises benzene fused with a five-membered heterocycle, and the fused aromatic structure is substituted at meta-positions, which are the 1- and 3-, or 1- and 5- positions on the benzene fused heterocycle.

Example structures of the one or more MCLs and corresponding meta-positions include one of the followings: 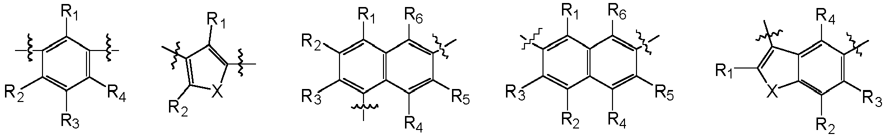 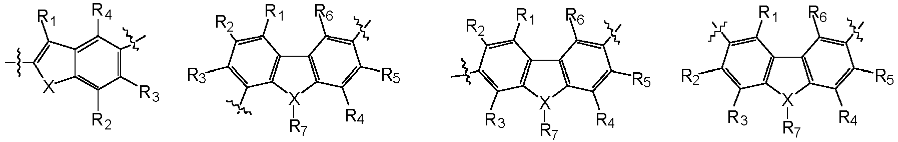 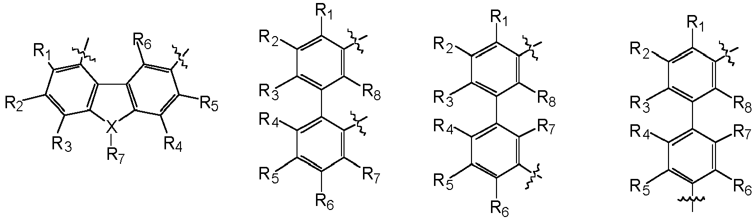 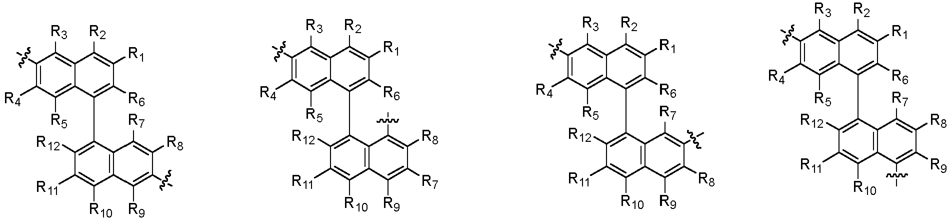 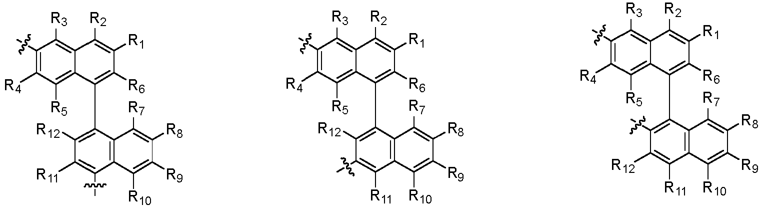 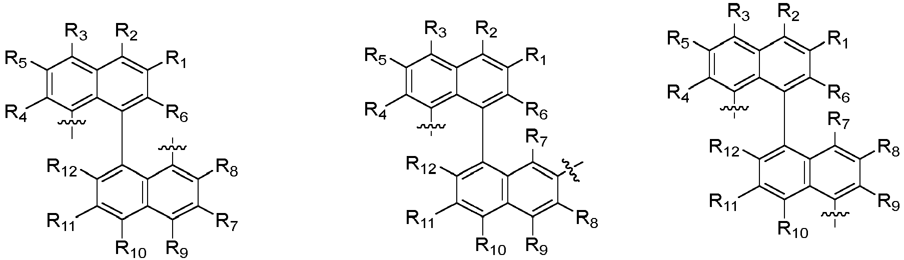 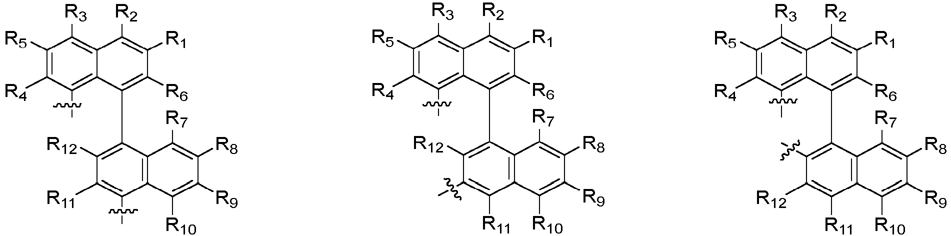 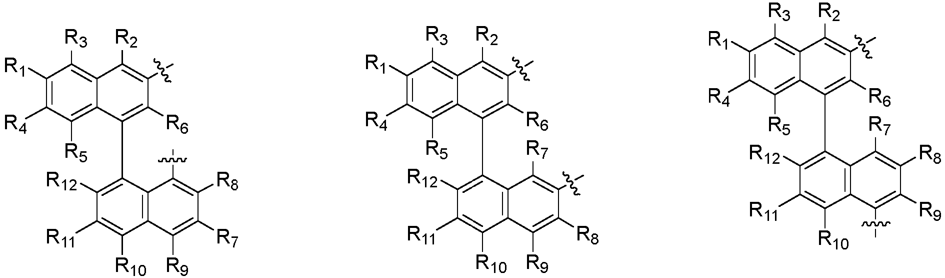 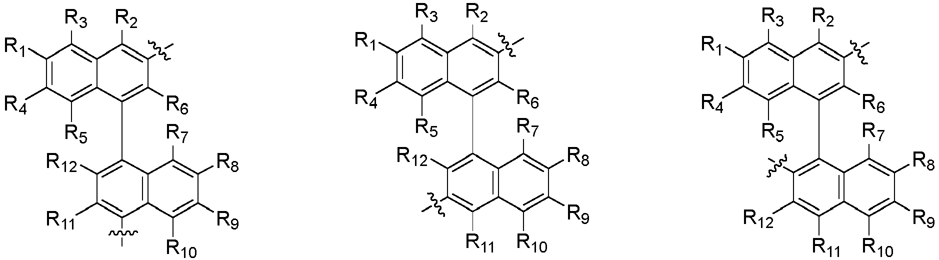 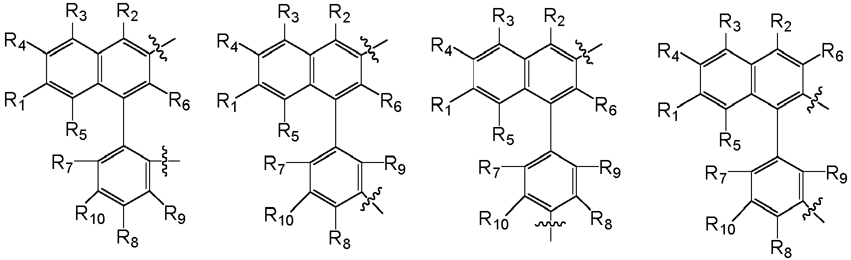 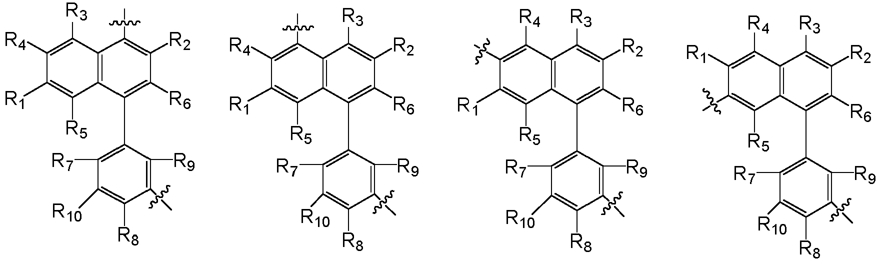 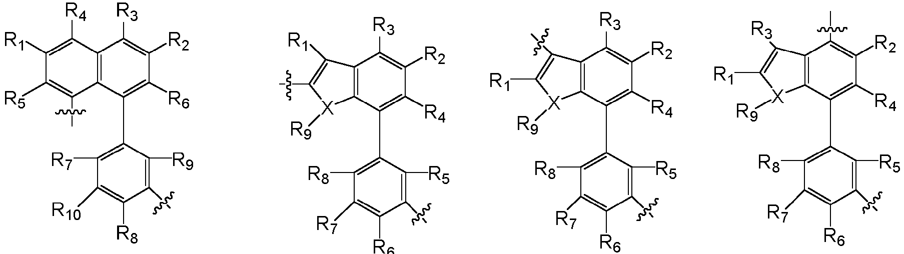 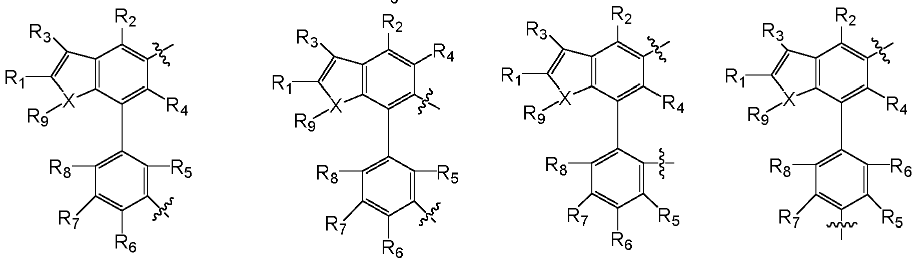 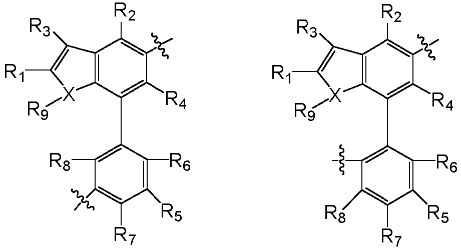 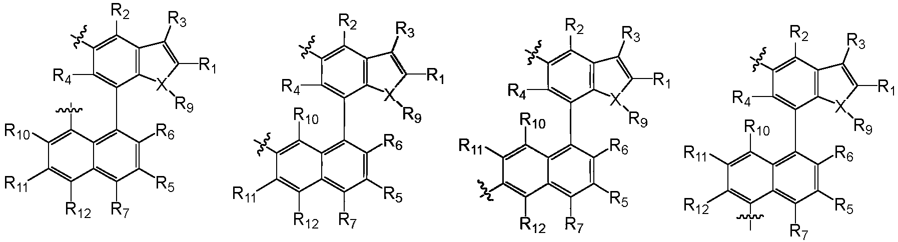 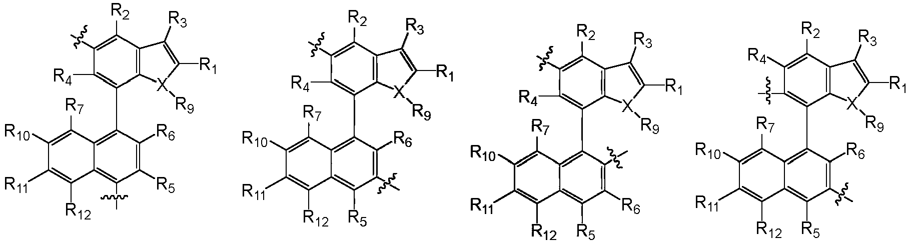 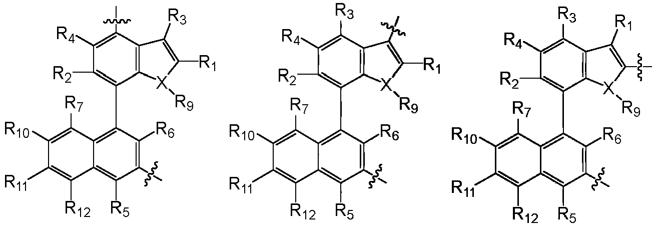 wherein X is S, Se, N, C, or O; R₁ - R₁₂ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl; and the wavy lines represent the meta-positions.

The one or more Ars include any one of a thiophene-based unit, a furan-based unit, a selenophene-based unit, or a pyrrole-based unit with a formula of 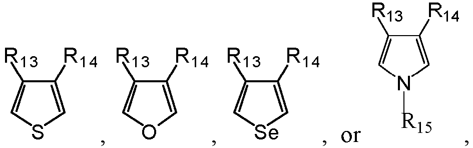 or any combination thereof. In the structures above, each of R₁₃ ,R₁₄ and R₁₅ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl.

An example thiophene-based unit may include, but is not limited to, the formula of 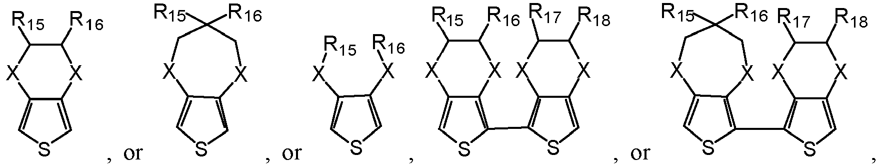 or 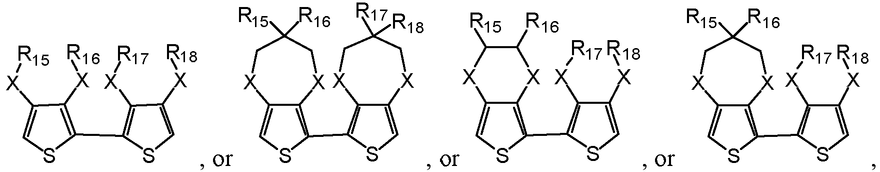 or 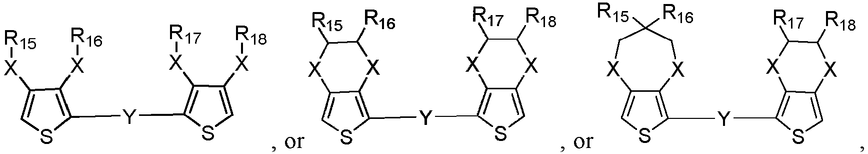 or 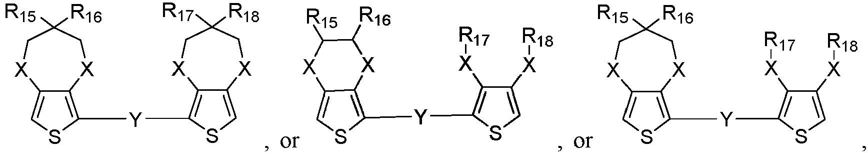 or a combination thereof.
In the structures above, X is S, Se, N, C, or O; each of R₁₅ - R₁₈ is independently selected from the following substituents, including, but not limited to, hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl. Y is any one or more of Ars, or aromatic structures, or fused aromatic structures, or a combinations thereof.

In some embodiments, the X in the thiophene-based unit is O.

By introducing meta-conjugation into the electrochromic polymer backbone, the electronic conjugation along the polymer backbone is interrupted and leads to a high bandgap (>2.0 eV). Thus, the disclosed electrochromic polymer appears highly transmissive (or even transparent) in the neutral state. Oxidation of the ECP results in a lower bandgap (<1.5 eV), and the absorbance of the polymer is red-shifted from UV region to visible and near-IR region. Thus, the polymer becomes highly colored.

The one or more Ars include one or more aromatic structures or fused aromatic structures. By controlling the types and amounts of the Ars, the redox potentials of the disclosed electrochromic polymer can be easily tuned while maintaining its high transparency within the visible light range in the neutral state. For example, more electron-rich units (e.g., dioxythiophenes) can be introduced onto the backbone to make the polymer more favorable to be oxidized, thereby decreasing its onset potential and improving its electrochemical stability and electrochromic cycling stability. The redox potentials of the disclosed electrochromic polymer can also be adjusted by varying substituents on MCLs (e.g., introducing alkoxy side chains).

The disclosed electrochromic polymers can be dissolved in a solvent, for example, toluene or p-xylene, which can be used for solution-processable film casting processes. By controlling the concentration of the polymer solution, a polymer thin film with a controllable thickness can be obtained. Furthermore, the excellent solubility makes the disclosed electrochromic polymers compatible with various casting methods, for example, spin-coating, spray-coating, and drop-casting. Manufacturing friendly process makes its extended applications feasible.

Examples are shown in the following.

### Embodiments

### Example 1 ECP-1

In some embodiments, the disclosed ECP-1 has a formula of

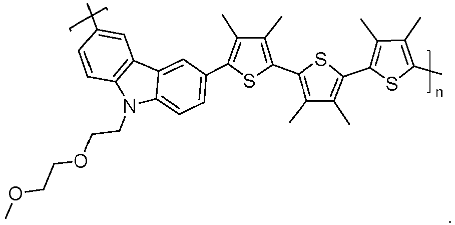

The ECP-1 is synthesized by preparing a carbazole-containing reaction unit and then polymerizing it with a dimer unit. The detail method includes the following steps:

### Step 1: preparing a carbazole-containing reaction unit (compound 2).

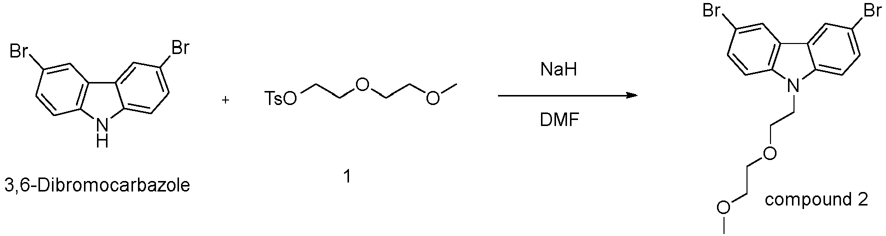

3,6-Dibromocarbazole is dissolved in DMF. Subsequently, 1.2 eq of NaH is added, and the mixture is stirred for 2 hours. Then 1.2 eq compound 1 is added into the reaction , and the mixture is stirred overnight. After that water is added into the reaction to precipitate out the solid. The suspension is filtered to get the desired product compound 2 as a white solid.

### Step 2: polymerization: carbazole-containing reaction unit polymerizing with a dimer unit.

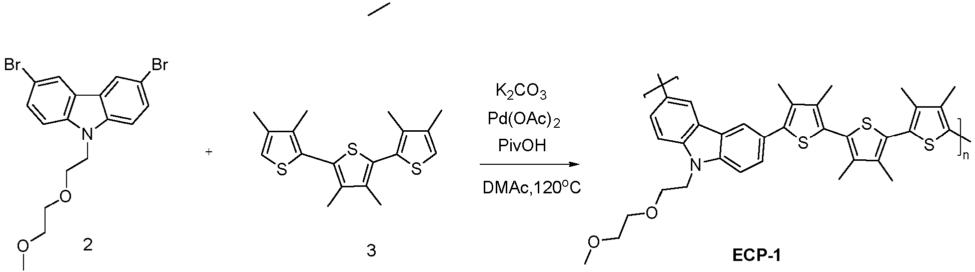

Compound 2 (1 eq), compound 3 (1 eq), K₂CO₃ (2.6 eq), PivOH(0.3 eq), Pd(OAc)₂, (0.02 eq) are added into a Schenk tube. Then vacuum(3-5min) and refill the tube with nitrogen. Repeat this procedure for three times. Subsequently, nitrogen degassed solvent Dimethylacetamide (DMAc) is added, and the mixture is heated to 120°C and last for 14 hours. Then the mixture is poured into methanol to precipitate out the crude polymer solid. Filter to get the solid and re-dissolve the solid into chloroform and wash with water for three times. The chloroform solution is added into large amount of methanol and precipitate out the polymer. The suspension is filtered to get the desired product polymer ECP-1.

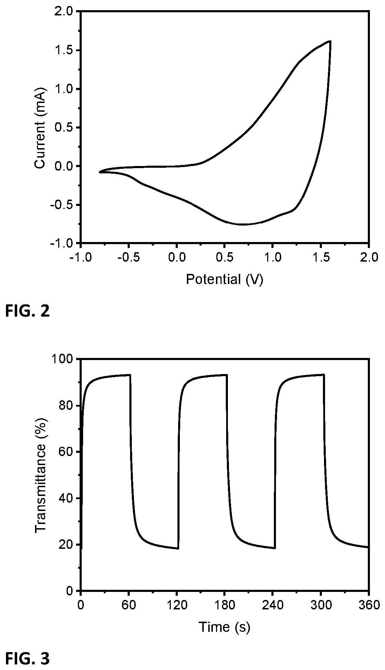

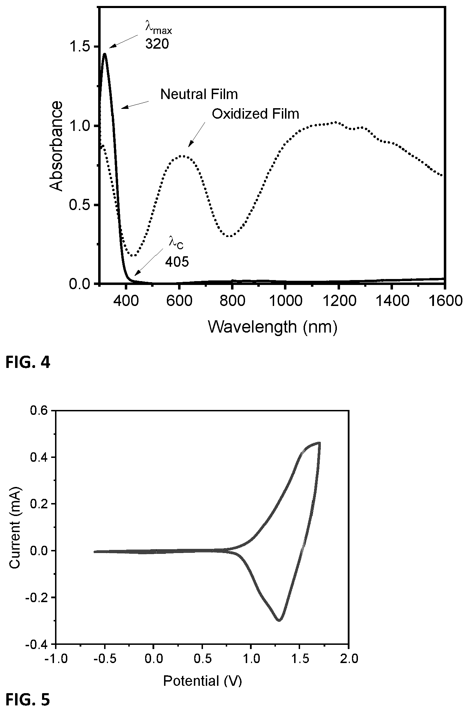

The obtained ECP-1 has an oxidation potential of around 0.75 V (vs. Ag/AgCl) and an energy bandgap of higher than 3.0 eV. The ECP-1 is fabricated into a solid-state ECD with ECP-1 used as the electrochromic layer, 0.2M of LiTFSI in PEGDA as the electrolyte, and VOx as the ion storage layer. The solid-state ECD can be stably switched between -0.5 V to 1.5 V (FIG. 2). The neutral state and oxidized absorbance spectra of the ECP-1 are shown in FIG. 4 with 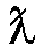 c of 405 nm and 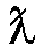 max of 320 nm. The solid-state ECD shows a high transparency with transmittance as high as 93% in the neutral state (FIG. 3), and switches to a bright blue color when ECP-1 is oxidized with one absorption peak at about 614 nm and another boarder absorption band at the near-IR region, around 900-1100 nm (FIG. 4). The optical contrast of the solid-state ECD is about 75% (FIG. 3).

### Example 2 ECP-2

In some embodiments, the disclosed ECP-2 has a formula of

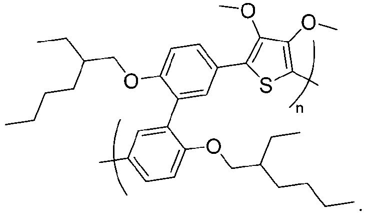

The ECP-2 is synthesized by first preparing a substituted benzene reaction unit and then polymerizing it with an acyclic dioxythiophene (AcDOT) unit. The detail method includes the following steps:

### Step 2-1: preparing a benzene-containing reaction unit (compound 4) by two steps.

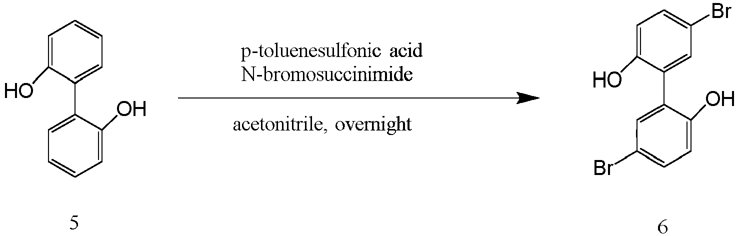

Compound 5 and p-toluenesulfonic acid are dissolved in acetonitrile. Subsequently, N-bromosuccinimide is added, and the mixture is agitated overnight. The suspension is filtered to get the desired product. The product compound 6 is a white solid.

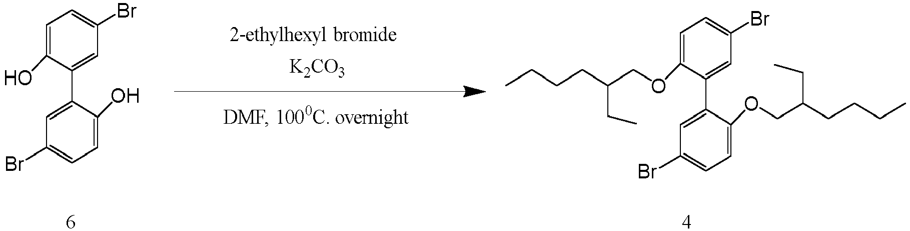

Compound 6 is dissolved in DMF under N₂. K₂CO₃ is added to the solution, and the reaction mixture is stirred for 15 mins, after which 2-ethylhexyl bromide is added. The reaction mixture is stirred at 100⁰C overnight. The reaction is stopped and cooled down to room temperature. The solvent is removed in vacuum, and the residue is dissolved in diethyl ether. The organic phase is washed with water, and the aqueous phases are extracted with ethyl acetate. The combined organic phases are dried, and the volatiles is removed by vacuum. The crude is passed through a small silica column, and the solvent is dried in vacuum to get the compound 4 as a yellow oil..

### Step 2-2: polymerization: The polymerization method is similar to that in step 1-2 with the reaction units of the substituted benzene reaction unit (compound 4) and AcDOT (compound 8) with a structure of

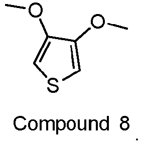

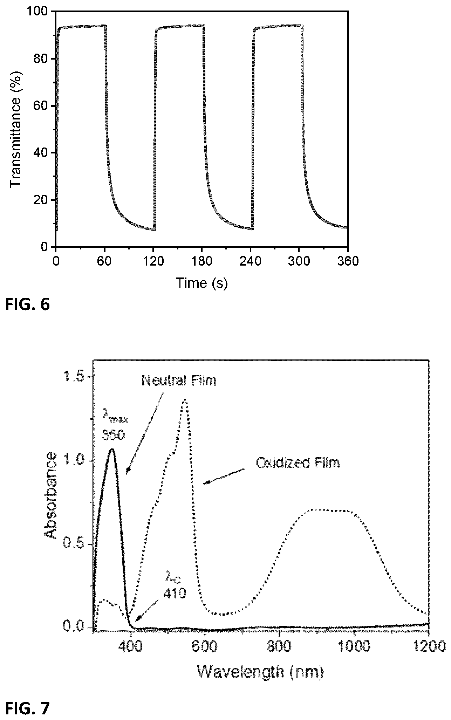

The obtained ECP-2 has a oxidation potential around 0.95 V (vs. Ag/AgCl) and an energy bandgap higher than 3.1 eV. The ECP-2 is fabricated into a solid-state ECD with ECP-2 used as the electrochromic layer, 1M of LiPF₆ in PEGMEA as the electrolyte, and VOₓ as the ion storage layer. The solid-state ECD can be stably switched between -0.6 V to 1.7 V (FIG. 5). The neutral state and oxidized state absorbance spectra of the ECP-2 are shown in FIG. 7 with 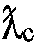 of 410 nm and 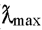 of 350 nm. The solid-state ECD shows high transparency with transmittance as high as 94% at neutral state at 550 nm (FIG. 6), and switches to bright red color when ECP-2 is oxidized with one absorption peak at around 546 nm and another broader absorption band at the wavelength around 800-1100 nm (FIG. 7). The optical contrast of the solid-state ECD is 87% (FIG. 6).

### Example 3 ECP-3

In some embodiments, the disclosed ECP-3 has a formula of

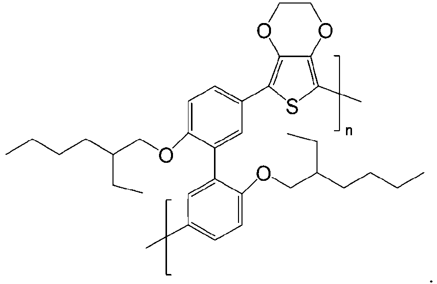

ECP-3 is synthesized by preparing a benzene-containing reaction unit and polymerizing it with a ProDot unit. The detail method includes the following steps:
Step 3-1: the same as Step 2-1
Step 3-2: polymerization: The polymerization method is similar to that in step 1-2 with the different reaction units of benzene-containing reaction unit (compound 4) and 3, 4-Ethylenedioxythiophene (EDOT, compound 9) with a structure of

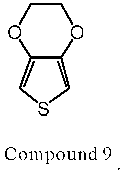

### Example 4 ECP -4

In some embodiments, the disclosed ECP-4 has a formula of

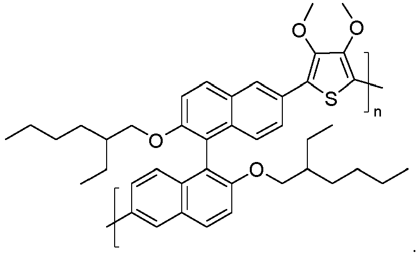

The ECP-4 is synthesized by preparing a naphthalene-containing reaction unit and then polymerizing it with an AcDOT unit. The detail method includes the following steps:

### Step 4-1: preparing naphthalene-containing reaction unit (compound 10) by two steps.

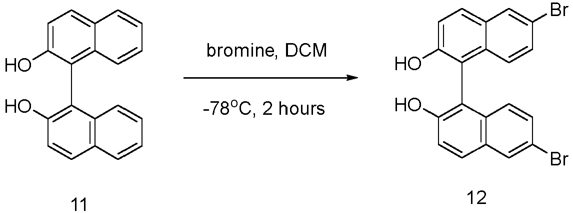

To a solution of compound 11 in dichloromethane was added dropwise a solution of bromine in dichloromethane over 15 minutes at -78°C. The reaction mixture is stirred for 2 hours at -78°C and then warmed gradually to room temperature and stay at room temperature for an additional 2 hours. The excess bromine was quenched by saturated aqueous sodium sulfite solution and stirred for 2 hours at room temperature. After extraction with dichloromethane, the combined organic layer was washed with brine, dried over sodium sulfate, and concentrated in vacuum.

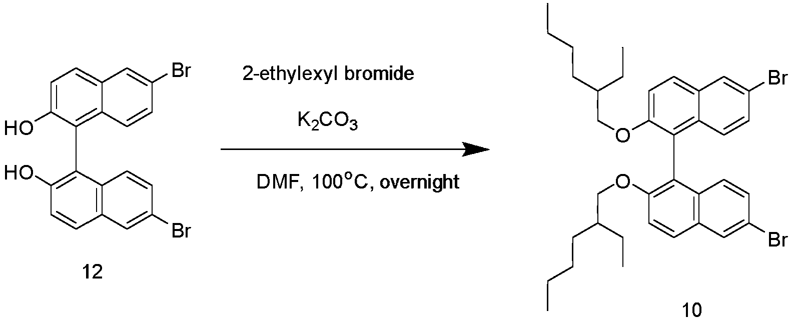

Compound 12 is dissolved in DMF under N₂, K₂CO₃ is added to the solution, and the reaction mixture is stirred for 15 minutes, after which 2-ethylexyl bromide is added. The reaction mixture is stirred at 100⁰C overnight. The reaction is stopped and cooled down to room temperature. The solvent is removed in vacuum, and the residue is dissolved in diethyl ether. The organic phase is washed with water, and the aqueous phases are extracted with ethyl acetate. The combined organic phases are dried by vacuum.

Step 4-2: polymerization: The polymerization method is similar to that in step 1-2 with the different reaction units of the naphthalene-containing reaction unit (compound 10) and AcDOT (compound 8).

### Example 5 ECP-5

In some embodiments, the disclosed ECP-5 has a formula of

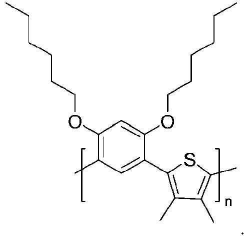

The ECP-5 is synthesized by a similar polymerization method to that in step 1-2 with the different reaction units of 1,5-dibromo-2,4-bis(hexyloxy)benzene and 3,4-dimethylthiophene.

In some embodiments, the disclosed ECP has a formula of 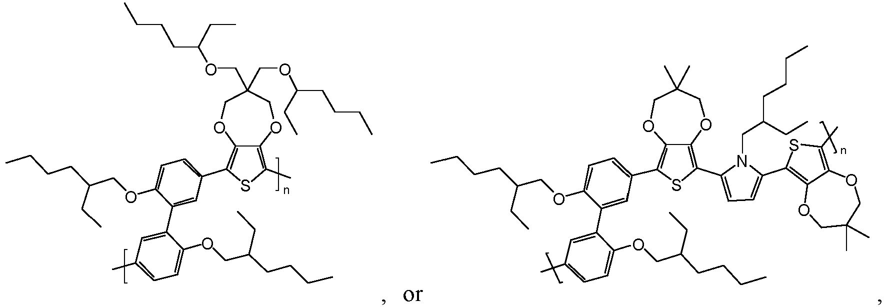 or 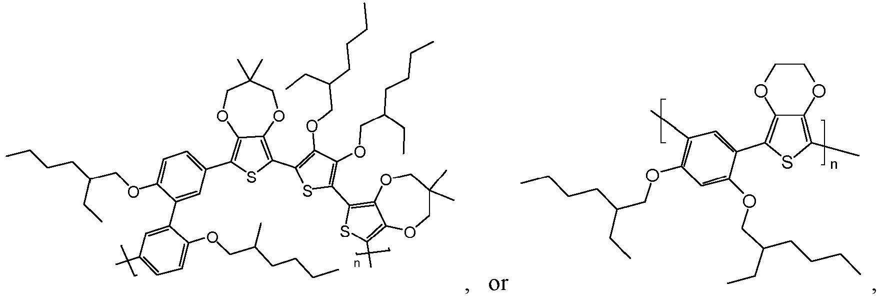 or 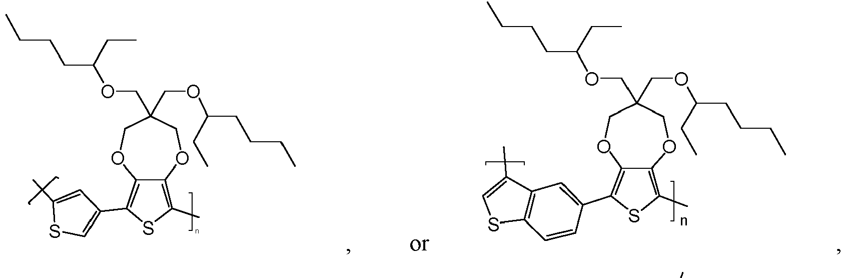 or 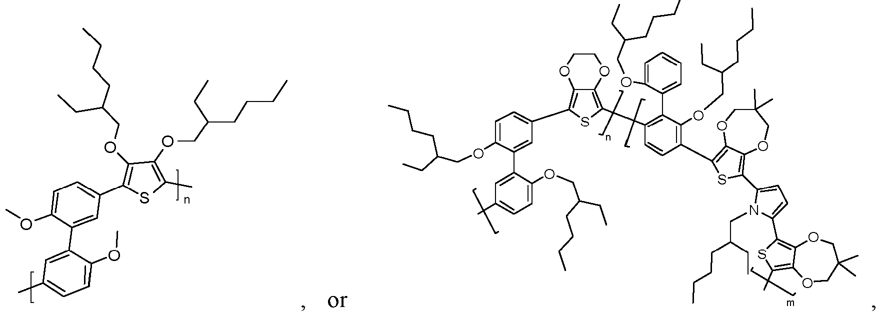 or 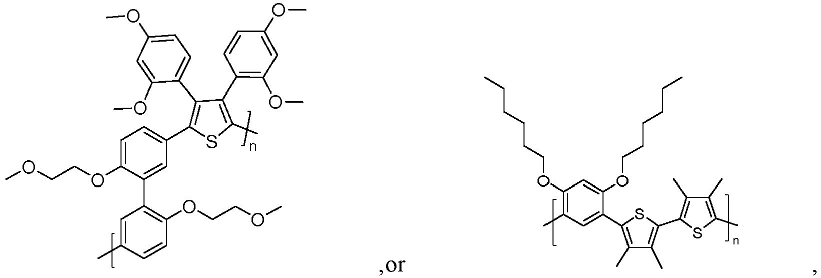 or 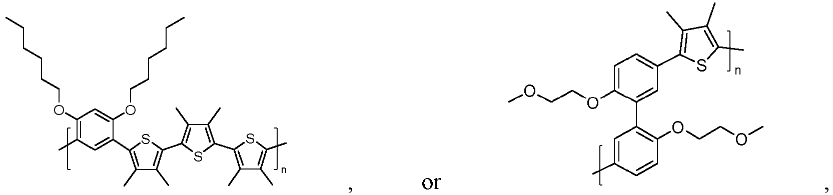 or 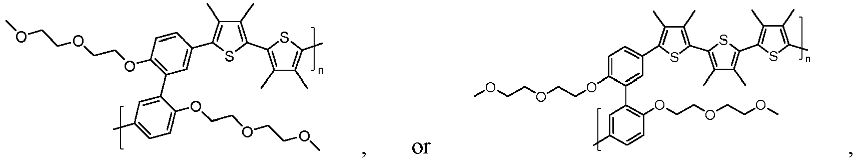 or 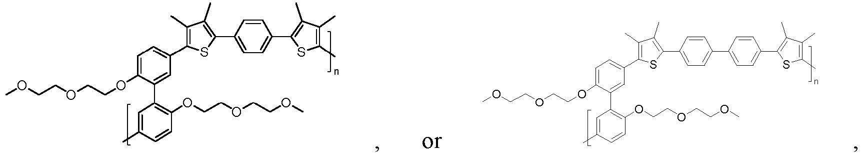 or 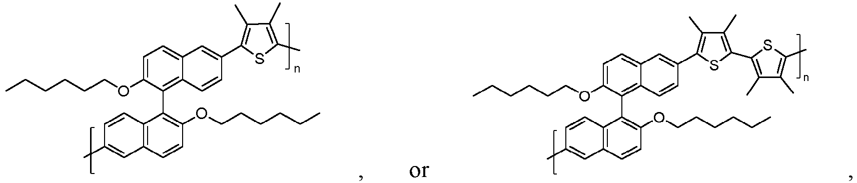 or 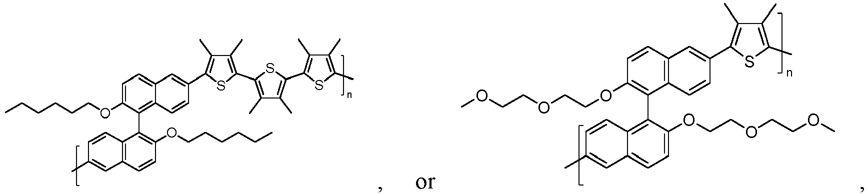 or 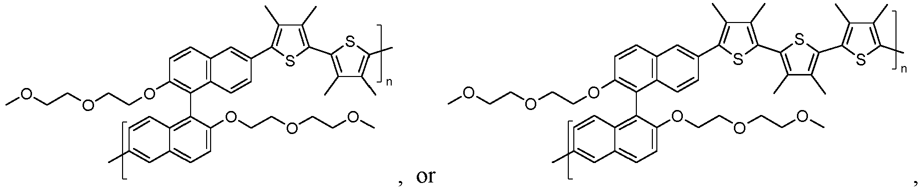 or 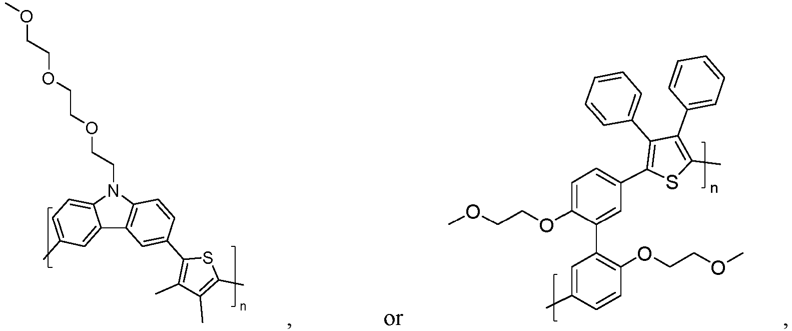 or 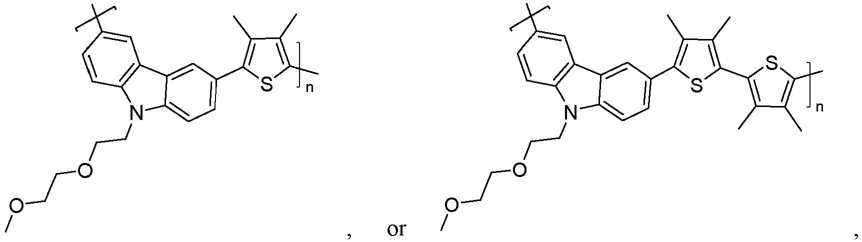 or 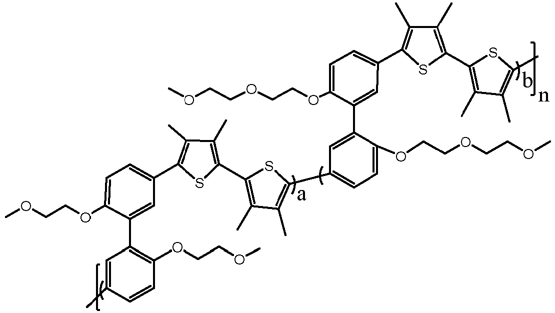 wherein n and m are integers greater than 0, a and b are integers equal to or greater than 0 with at least one of a and b is greater than 0.

In another aspect, the disclosed polymers can have fluorescent emission and can be applied to fluorescent products.

In conventional conjugated electrochromic polymers, the formation of polaron and bipolaron upon electrochemical doping lowers the energy of the optical transition, resulting in red-shift of the absorption from the visible region to the near-IR region and manifesting as color-to-transmissive change. Consequently, their doped state possesses residue absorption throughout the visible region. As the film thickness increases, this residue absorption becomes more severe, and the residue color appears. Thus, conjugated electrochromic polymers exhibit relatively low optical contrast and contrast ratio, a major factor that limits the further adoption of polymer-based ECDs in applications.

In contrast to conventional conjugated ECPs which undergo color-to-transmissive change, the disclosed novel ECPs have transparent-to-colored change. The disclosed polymers exhibit higher energy band gaps so that they absorb light in the UV region without absorption in the visible region in their neutral state, resulting in a transparent state, and in some embodiments, almost 100%transparent. For example, polymers containing chromophore group triarylamine attain transparent-to-colored electrochromic switching. Some conventional small molecules based on ethylenedioxythiophene derivatives that can switch from a transparent to a colored state. However, such designs also give rise to specific challenges. Firstly, these polymers usually exhibit poor switching stability. This is due to the fact that the charges formed in the doped state are unable to be delocalized along the polymer chain, resulting in limited stability and durability. Secondly, electrochromic devices based on organic small molecules are usually in the solution phase, thus the color change depends on the diffusion of molecules onto the electrode, leading to slow switching speeds, intermediate colors, and hampering the applications in flexible devices.

The disclosed electrochromic polymers exhibit almost 100% transmittance (e.g., 85%-99.9%) in the neutral state while showing high absorption in the oxidized state, leading to the highest recorded optical contrast and contrast ratio. The polymer backbone includes or consists of meta-conjugated linkers (MCL) and aromatic moieties (Ars) as described above. The MCL connects the aromatic moieties at the meta position, interrupting the charge delocalization. Therefore, the band gap of the disclosed polymer is increased by meta-conjugation so that the absorption of the neutral polymer could be centered in the UV region to achieve an almost 100% transparent state. On the other hand, the MCL and aromatic moieties provide the conjugation to accomplish low oxidation potential for the transparent-to-colored switching and high switching stability. The color of the polymers can be easily controlled by adjusting the conjugation length of MCL and aromatic moieties. In some embodiments, the disclosed polymers may be made from MCLs, such as carbazole, biphenyl, and binaphthanlene, and thiophenes as aromatic moieties. The disclosed polymers based on this inventive concept show wide color tunability and good electrochromic properties, including optical contrast of over 95% and switching stability of more than 10000 cycles.

### Examples

In the disclosed polymer, each meta-conjugated polymer contains aromatic comonomers which are connected by MCL at meta-position. We design a series of polymers containing carbazole (CBZ), biphenyl (BP), and binaphthalene (BNP) as MCL and varying the number of thiophenes (T1, T2 and T3) to change the length of the aromatic moieties. The structures of these designs are shown below.

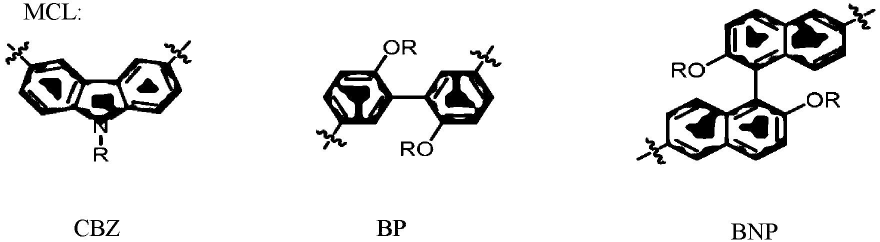

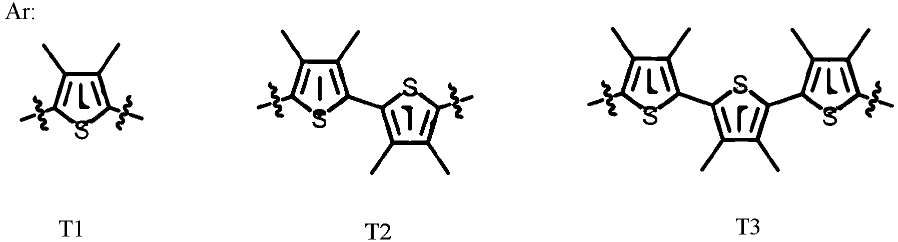

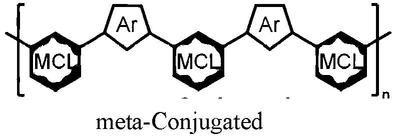

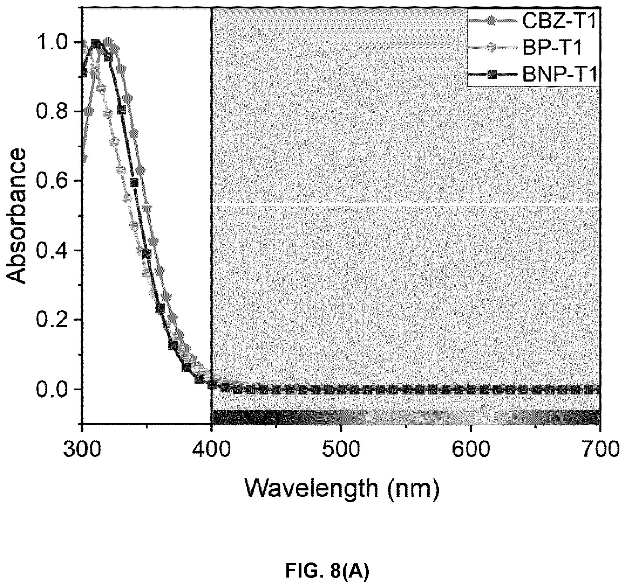

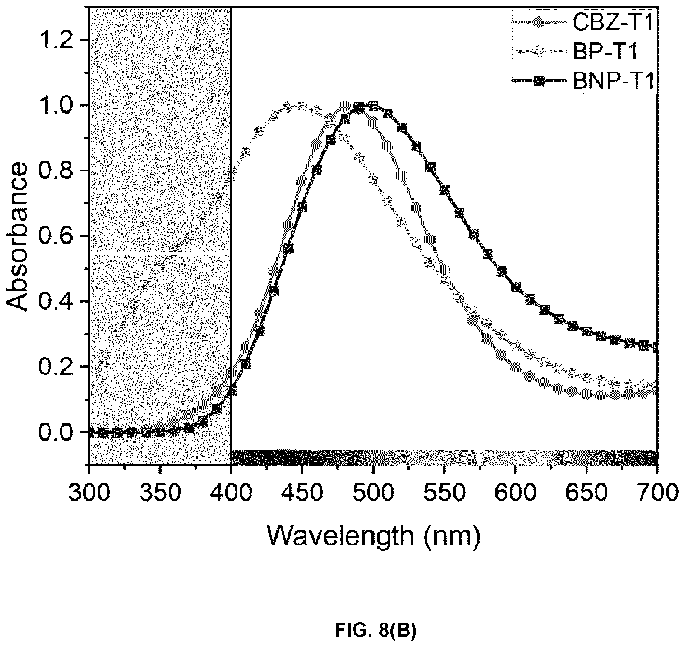

In order to guide experiment and probe the design paradigm from a molecular orbital perspective, density functional theory (DFT) calculations are performed on these meta-conjugated polymers and theoretical spectra are generated for the neutral and radical cation states. The absorption spectra of CBZ-T1, BP-T1, and BNP-T1 in their neutral states exhibit almost 100% transparency in the visible region, with notable absorption occurring solely in the UV region (FIG. 8(A)). While CBZ-T1 and BNP-T1 manifest a slightly red-shifted absorption onset compared to BP-T1, all three polymers maintain absorption wavelengths below 400 nm. In the radical cation (oxidized) states, the absorption in the UV region decreases, leading to an increase in absorption within the visible region (FIG. 8(B)). This transparent-to-colored electrochromism can be further explained by the geometric change of the polymer from neutral to radical cation states. The polymers have a non-planar structure in their neutral state with torsional angles around 50 to 60 degrees between the MCL and adjacent thiophene. This non-planar structure and significant torsional hindrance impede charge delocalization, increasing the neutral polymer's band gap, so polymer absorbs in solely in UV region. Whereas, in radical cation state, the polymer is planarized with the decrease in torsional angles 25 to 40, and thus charges could delocalize along the polymer chain, and the absorption undergoes a red shift to the visible region for coloration. In contrast with BP-T1, the radical cation absorption of CBZ-T1 and BNP-T1 is more red-shifted due to enlarged conjugation from the BNP unit.

The length of aromatic moieties has impact on the optical properties of the polymer. In their neutral states, CBZ-T1, CBZ-T2, and CBZ-T3 exhibit nearly identical absorption spectra, suggesting that the number of thiophene units does not affect the band gap of the polymers in the neutral states. Based on calculation, the torsional angles between MCL and thiophene remain nearly equivalent across polymers as the number of thiophene increases, thus resulting in their corresponding absorption in the UV region to achieve transparency. However, in the radical cation state, increasing the number of thiophene units leads to a red shift in the spectra. Consequently, the radical cation polymers display distinct colors, specifically orange, purple, and blue, corresponding to CBZ-T1, CBZ-T2, and CBZ-T3, respectively. This color variation can be attributed to the significant torsional angle change between thiophenes. In the case of moving from polymers with one thiophene (CBZ-T1) to two thiophene (CBZ-T2), a change in torsional angle (~5 degrees) has been observed. A similar trend has been observed while moving from 2T to 3T with torsional angles ~15 degrees, respectively. MCL could hinder the charge delocalization of the neutral polymer and result in almost 100% transparency. The color of the polymer in radical cation state could be tuned by adjusting/varying the conjugation length of the polymer.

### Example synthesis of electrochromic polymers

The MCL monomers of carbazole, biphenyl and binaphthalene are synthesized with different enlarged conjugation. Different sidechains are added on the MCL to adjust the solubility and polarity of the polymers. Then the oligomer 3,4-dimethylthiophene T1, T2 and T3 are made. After getting the monomers, Direct Arylation Polymerization (DArP) is applied to make the nine meta-conjugated transparent electrochromic polymers. Each meta-conjugated polymer solution is spin coated onto an ITO glass as working electrode and placed in a cuvette for the electrochemical and optical measurements. The location of the oxidized state absorption peaks indicates the red-shifting trend upon incorporation of longer aromatic moieties. The disclosed techniques allow rationally shifting of the absorption peak, which allows access to a large variety of colors throughout the visible region. In some embodiments, the oxidized polymers with different length of aromatic moieties display distinct colors, specifically orange, purple, and blue, corresponding to T1, T2, and T3 for BP and CBZ polymers, respectively. The CIELAB color coordinates of all polymers in their neutral and oxidized states are obtained. The neutral state polymers possess *L*a*b** values close to (100, 0, 0), which is completely transparent. In oxidized state, these polymers cover a wide range in the color space, which provide the potential for color mixing. By varying a conjugation length of the one or more MCLs and the one or more Ars, the disclosed electrochromic polymer can be controlled to design for a wide range of colors. In addition, by blending different disclosed electrochromic polymers with different colors with different ratios, another new batch of colors can be produced, which greatly enriches the color library. Furthermore, unlike the conventional electrochromic polymer blends, due to the close oxidation potentials from the disclosed electrochromic polymers, the disclosed electrochromic polymer blends do not have any intermediate color issue which is on the other hand commonly observed by the conventional electrochromic polymer blends. In some embodiments, due to its high transmittance, the disclosed electrochromic polymers or blends have a high optical contrast and good stability with a big color library without any intermediate color. This disclosed electrochromic polymer/device can be used in a variety of applications including smart windows and glasses, biosensors, electronic papers, displays, Augmented Reality (AR), Virtual Reality (VR), Mixed Reality (MR), patterned electrochromic displays, curtain wall and sunroof.

The electrochemical properties of the polymers are evaluated by cyclic voltammetry and differential pulse voltammetry (DPV). The polymers exhibit quasi-reversible oxidations. Upon examination of the polymers via DPV, CBZ-T1, CBZ-T2 and CBZ-T3 exhibit one peak indicating the formation of radical cation. However, polymers containing BP and BNP unit show two peaks as the second peak corresponding to the formation of dication. This oxidation renders the electrochemistry irreversible and causes the formation of a new absorption peak in the visible range. It is worth noting that all polymers exhibit relatively low oxidation onset potential (CBZ polymers about 0.6-0.8 V vs Ag/AgCl; BP polymers about 0.8-1.0 V vs Ag/AgCl; BNP polymers about 0.8-1.0 V vs Ag/AgCl), which is attributed to the conjugation from aromatic moieties. The low oxidation onset potential is beneficial for the electrochemical stability of polymers since undesirable side reactions could be avoided such as water oxidation. Overall, CBZ polymers possess a lower oxidation onset potential and a better reversibility at higher potential, although these meta-conjugated polymers are quite similar in their characteristics.

The lower energy absorption peak is a result of the electronic transition from the singularly occupied molecular orbital with an electronic spin down (Sβ) to the lowest unoccupied molecular orbital (Lβ). Therefore, the disclosed meta-conjugated polymer is capable for the modulation of both visible light and near IR synchronously.

The disclosed techniques allow the color tunability of meta-conjugated electrochromic polymers and their low oxidation potentials. The techniques can also be used to provide black color electrochromism by blending vibrantly colored chromophores whose collective absorption entirely covers the visible spectrum. The inventors discover that blending of the disclosed polymers can produce desired colors. In some embodiments, CBZ-T1 (orange) and CBZ-T3 (blue) meta-conjugated polymers are used for blending to obtain transparent-to-black electrochromics. When making blends, the absorption coefficients of the polymers in their oxidized states are employed to determine the proper ratios of the polymers to blend to obtain the black color. Beer-Lamber plots of the polymer films show the nearly identical absorption coefficient of CBZ-T1 and CBZ-T3 in oxidized states, thus the mass ratio of the CBZ-Blend is determined as 1:1. Other mass ratios of blending or blending of different disclosed polymers can be used to make other desirable colors.

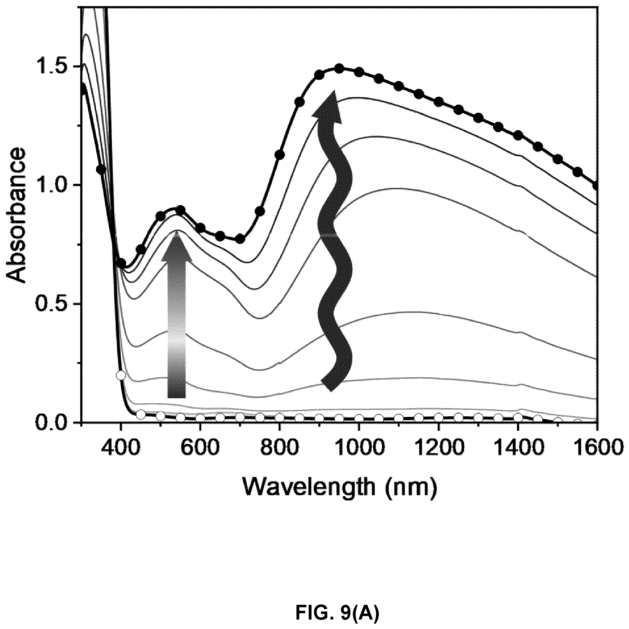

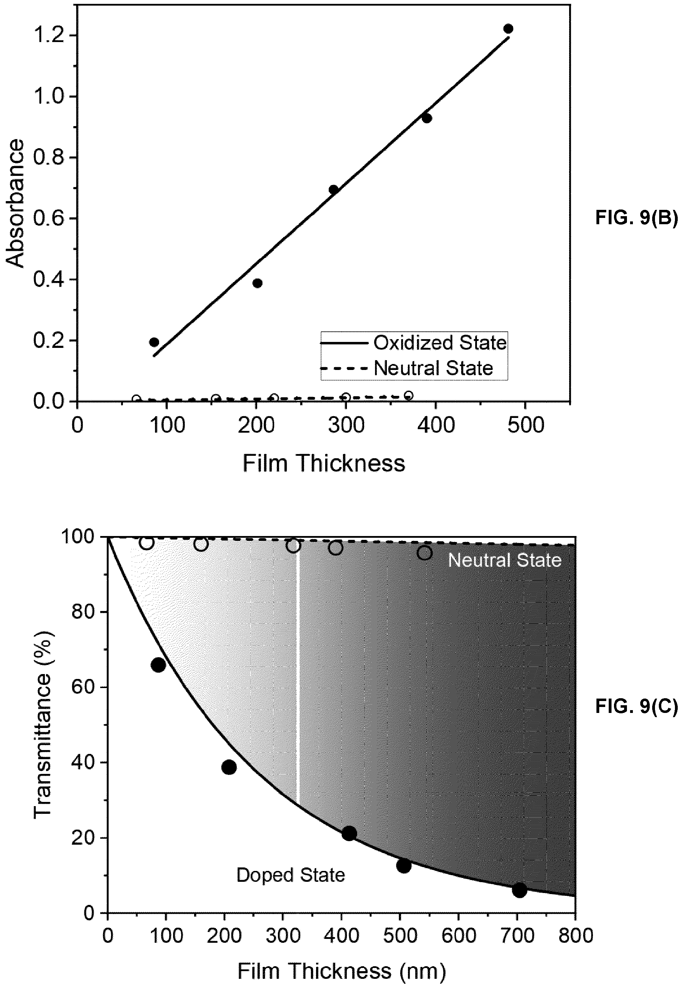

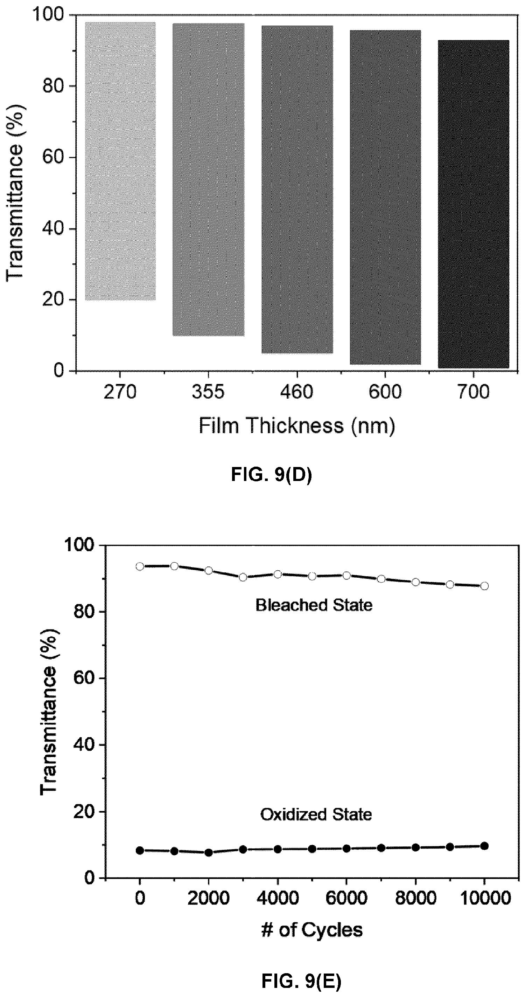

The absorption spectro-electrochemistry of the CBZ-Blend with a film thickness of 300 nm is shown in Fig. 9(A). The neutral film possesses an absorption onset at 400 nm indicating almost 100% transparency in visible region. Oxidation of CBZ-Blend to the radical cation state gives rise to two broad absorptions in visible and near-IR region, with λmax at 550 and 950 nm, demonstrating the synchronized modulation of light and heat. A number of CBZ-Blend films with different film thickness are prepared and the absorption coefficient is derived from the Beer-Lambert plot as shown in FIG. 9(B). The neutral (bleached) state absorption coefficient for CBZ-Blend is approximated to be 5 × 10² cm⁻¹, which is 2 orders of magnitude lower than the oxidized (colored) state value, 3.7 × 10⁴ cm⁻¹. The transmittance of the neutral and oxidized states are plotted as a function of film thickness using the respective absorption coefficient values as shown in FIG. 9(C). When the film is thin, the transmittance of both the neutral state and the oxidized state can be nearly 100%. Since the neutral state absorption coefficient is close to zero, the transmittance remains nearly 100% and undergoes minimal decay as the film becomes thicker. In some embodiments, the optical contrast between the neutral and oxidized states can reach nearly 100%, e.g., 80%, 85%, 90%, 95%, 96%, 97%, 98%, 99%, or more, or between any of the above values. FIG. 9(D) illustrates the transmittance of CBZ-Blend in both neutral and oxidized states of the electrochromic layer at different thicknesses according to some embodiments. The optical contrast is equal to the difference of transmittance in both neutral and oxidized states. For different film thicknesses (270-700 nm) measured in neutral state, the optical contrasts shown in FIG. 9(D) are respectively 78%, 87.7%, 92.1%, 93.3%, and 92%. The contrast ratio is calculated by the ratio of transmittance at the neutral state versus the transmittance at the oxidized state. For different film thicknesses (270-700 nm) measured in neutral state, the contrast ratios shown in FIG. 9(D) are respectively 4.9%, 9.8%, 19.4%, 47.65%, and 93%. As shown in FIG. 9(D), the optical contrast of the disclosed electrochromic film increases with the film thickness. When the disclosed EC film thickness increases, the response time would be increased. When the film thickness is greater than 1500nm, the disclosed EC device can have a great optical contrast, e.g., about 96% with transmittance of 96% in the neutral state and about 0.06% in the oxidized state of the EC layer, the device can have slow response time, e.g., about 1 minute. When the film thickness increases, the transmittance of the EC layer in the neutral state can decrease to some minor degree. However, the transmittance of the EC layer in the oxidized state can greatly decrease, thus with the film thickness increases, the optical contrast (transmittance difference between the neutral state and the oxidized state) of the EC layer greatly increases. However, limited by the increased response time with the thickness increasement, in some embodiments, the film thickness of the disclosed EC layer is limited to 1500nm or less. In some embodiments, the film thickness of the disclosed EC layer is limited to 1200 nm, 1000 nm, 900 nm, 800 nm, 700 nm, 600 nm, 500 nm, 400nm, 300 nm, 200 nm, or 100 nm, to obtain the response time of less than 1 minute.

The disclosed meta-conjugated electrochromic polymer layers exhibit ultrahigh optical contrast and fast switching speed. They also exhibit high stability, including photostability and electrochromic switching stability. The photostability is examined by exposing the encapsulated polymer films to a solar simulator matching a standard air mass 1.5 illuminant. Their absorption spectra are measured and the maximum absorption as a function of irradiation time is plotted. The results indicate the disclosed polymers stable even when used with other materials, such as ITO, electrolyte, and ion storage layer (e.g., nano ITO particles). To reveal the cycling stability of the polymer thin film, 10,000 CV switching cycles are applied in a 3-electrode set up, where the voltage is applied from -0.2 V to 1.0 V at 80 mV/s. The transmittance of neutral and colored state is recorded every 1000 cycles. The optical contrast of the polymer decreased by 10%, suggesting that the meta-conjugated polymers are suitable for long-term performance.

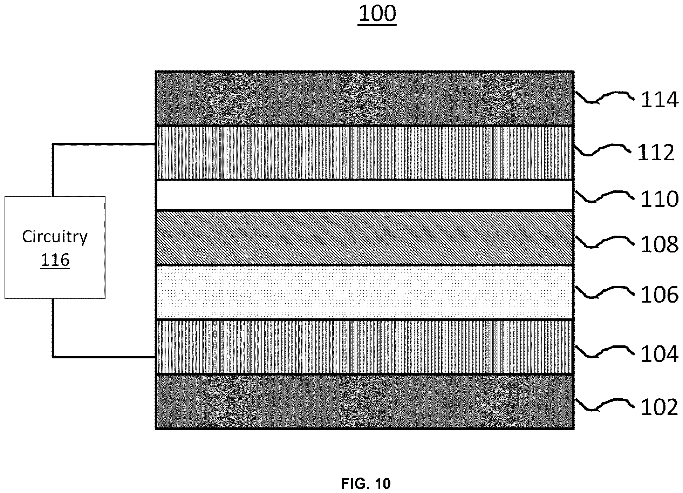

As shown in FIG. 10, an electrochromic device 100 according to some example embodiments may have a first insulating substrate 102, a first conducting layer 104 disposed over the first insulating substrate 102, an electrochromic layer 106 disposed over the first conducting layer 104, an electrolyte layer 108 disposed over the electrochromic layer 106 , a second conducting layer 112 disposed over the electrolyte layer 108, a second insulating substrate 114 disposed over the second conducting layer 112, and circuitry 116 to operate the electrochromic device 100. In some embodiments, the electrochromic device 100 may further includes an ion storage layer 110 disposed between the second conducting layer 112 and the electrolyte layer 108. The electrochromic layer 106 may include an electrochromic polymer as disclosed above. For example, the electrochromic polymer includes or consists of a polymer backbone including one or more meta-conjugated linkers (MCLs) and one or more aromatic moieties (Ars), wherein each of the one or more MCLs is partially conjugated with one of the one or more Ars at a meta position of the one or more MCLs. In some embodiments, a thickness of the electrochromic layer 106 is from 10 nm to 1500 nm resulting in transmittance of 85%-99.9% at a wavelength of 550 nm at a neutral state of the electrochromic layer. For example, a thickness of the electrochromic layer 106 from 10 nm to 1500 nm yields a transmittance of 85%, 87%, 90%, 92%, 95%, 96%, 97%, 98%, 99%, 99.9% or between any two of the above numbers. The electrochromic device 100 has transmittance of 60% or more at a wavelength of 550 nm at a bleached state of the device. For example, by tuning the material and thickness of the electrochromic layer 106, the electrochromic device 100 may have at its bleached state transmittance of 60%, 65%, 70%, 75%, 80%, 85%, 90%, 92%, 95%, 96%, 97%, 98%, or 99%, or between any two of the above numbers.

In some embodiments, the electrochromic layer has transmittance of 40%-0.1% at a wavelength of 550 nm at an oxidized state of the electrochromic layer. For example, the electrochromic layer at the oxidized state has transmittance at a wavelength of 550 nm of 40%, 35%, 30%, 25%, 20%, 15%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1%, or between any two of the above numbers.

In some embodiments, the electrochromic layer 106 has an optical contrast of 60% or more. For example, the electrochromic layer 106 may have an optical contrast of 60%, 65%, 70%, 75%, 80%, 85%, 90%, 95%, 96%, 97%, 98%, 99%, or between any two of the above numbers.

In some embodiments, when the electrochromic device 100 includes the ion storage layer 110, the ion storage layer 110 has transmittance of 80% or more at a wavelength of 550 nm. In some embodiments, the ion storage layer 110 may include (1) one or more oxides of metal elements in Group 4-12, or (2) a mixture of the oxides, or (3) one of the oxides doped by a different metal oxide, or (4) a transition-metal complex, or (5) one or more of redox-active polymers including redox active nitroxyl, galvinoxyl radical polymers and conjugated polymers.

In some embodiments, the ion storage layer 110 includes ITO particles, wherein the ion storage layer has transmittance of 90% or more at a wavelength of 550 nm. In some embodiments, the ITO particles may be nanoparticles having a size of 1-900 nm.

In some embodiments, at least one of the first conducting layer 104 and the second conducting layer 112 includes ITO, aluminum zinc oxide (AZO), fluorine doped tin oxide (FTO), silver nanowires, graphene, carbon nanotube, metal mesh based transparent conductive electrodes, silver-nanoparticle ink, or an organic conductive polymer.

In some embodiments, the electrochromic device 100 has an optical contrast of 60% or more. For example, the electrochromic device 100 may have an optical contrast of 60%, 65%, 70%, 75%, 80%, 85%, 90%, 95%, 96%, 97%, 98%, 99%, or between any two of the above numbers.

In some embodiments, a color of the electrochromic layer 106 at an oxidized state is varied by varying a conjugation length of the one or more MCLs and the one or more Ars.

In some embodiments, the electrochromic layer 106 includes a blend of different electrochromic polymers without an intermediate color. The structures of the polymer(s) in the electrochromic layer 106 are explained above and will not be repeated for brevity.

This disclosure further provides an electrochromic device that can be switch between transparent and black. The electrochromic materials that can switch reversibly between black and transmissive states holds great significance for various commercial and military applications. For example, the inventors discover that CBZ-T1 and CBZ-T3 possess similar absorption coefficient, therefore mix CBZ-T1 (transparent-to-orange switching) and CBZ-T3 (transparent-to-blue switching) at mass ratio of 1:1 to obtain transparent-to-black electrochromic device. The configuration of the transparent-to-black electrochromic device is similar to the electrochromic device 100 explained above. The device is assembled in a two-electrode configuration by using CBZ-T1 and CBZ-T3 mixture as electrochromic layer and nano ITO particle as ion storage layer (at 1.5 µm). In the spectro-electrochemistry study, the potential of the device is increased from -0.6 to 2.4 V. As the potential is increased, transmittance in the visible region decrease due to the oxidation of CBZ-T1 and CBZ-T3 mixture, and the device switches from transparent state to black state. The potential is increased until no further changes in transmittance are observed and the optical contrast is 88% (1-89%). The transmittance spectra are referenced to air, which means the transmittance loss includes glass, ITO, electrolyte layer, and ion storage layer. The CIE *L*a*b** color coordinates at different voltage are investigated. As the potential increases, the lightness (L*) in CIE *L*a*b** color coordinates decrease from 95 to 36, with *a** and *b** maintaining close to 0, indicating the transparent-to-black color switching without intermediate color. To reveal the cycling stability of the device, 10000 CV switching cycles are applied and the transmittance spectra are measured. The transmittance at 550 nm of the transparent state and colored state of the device indicates that the optical contrast dropped slightly from its original value of 85% to 78% (shown in FIG. 9(E)), representing the best cycling stability of black electrochromic device with such high optical contrast.

In summary, meta-conjugated polymers that enable transparent-to-colored electrochromic switching with a wide color range, ultra-high optical contrast, low oxidation potential, and excellent switching stability have been provided in this disclosure. A transparent-to-black electrochromic device based on polymer blending has been successfully obtained with the optical contrast above 91% and contrast ratio of 91%, representing the best performing black electrochromics. This approach to access transparent electrochromic polymers opens up promising prospects in future electrochromic innovations.

The disclosed electrochromic device can be utilized in various applications, such as smart windows and glasses, biosensors, electronic papers, displays, Augmented Reality (AR), Virtual Reality (VR), Mixed Reality (MR), patterned electrochromic displays, curtain wall and sunroof. Note that for conventional EC devices, the bleached state of the device is corresponding to the oxidized state of EC layer. However, for the disclosed EC device, the bleached state of the device is corresponding to the neutral state of EC layer.

## Claims

1. An electrochromic device, comprising:
a first insulating substrate;
a first conducting layer disposed over the first insulating substrate;
an electrochromic layer disposed over the first conducting layer, wherein the electrochromic layer comprises an electrochromic polymer having a polymer backbone comprising one or more meta-conjugated linkers (MCLs) and one or more aromatic moieties (Ars), wherein each of the one or more MCLs is partially conjugated with one of the one or more Ars at a meta position of the one or more MCLs;
an electrolyte layer disposed over the electrochromic layer;
a second conducting layer disposed over the electrolyte layer; and
a second insulating substrate disposed over the second conducting layer,
wherein a thickness of the electrochromic layer is from 10 nm to 1500 nm resulting in transmittance of 85%-99.9% at a wavelength of 550 nm at a neutral state of the electrochromic layer;
wherein the electrochromic device has transmittance of 60% or more at a wavelength of 550 nm at a bleached state of the device;
wherein each of the one or more MCLs and corresponding meta positions comprise one of the following formulas: 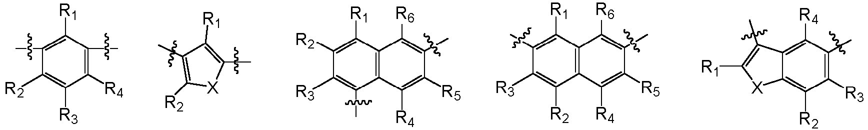 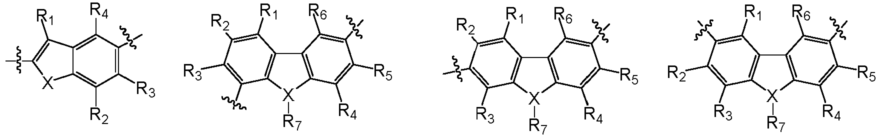 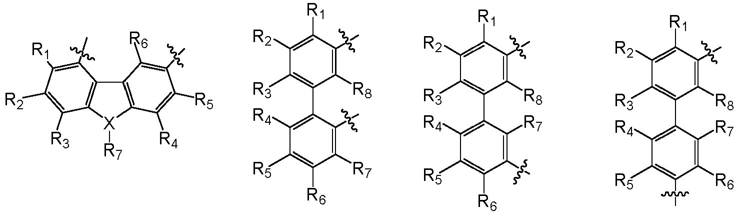 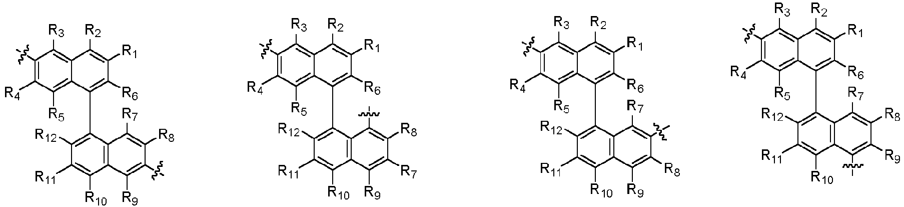 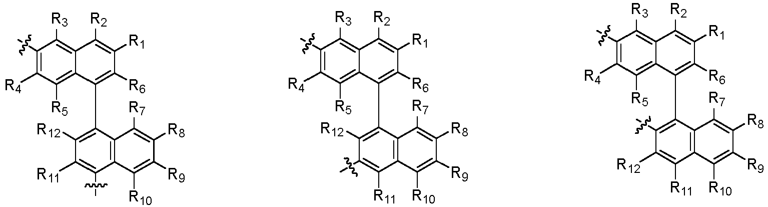 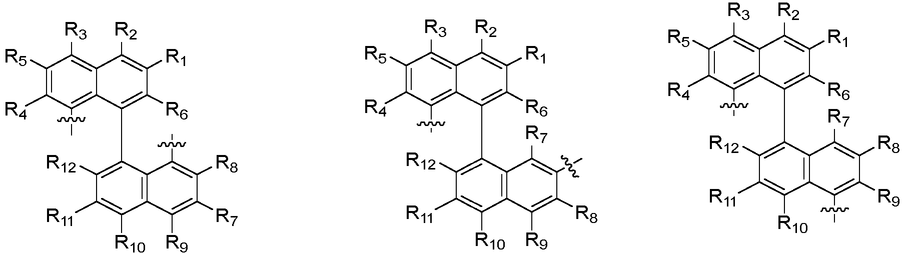 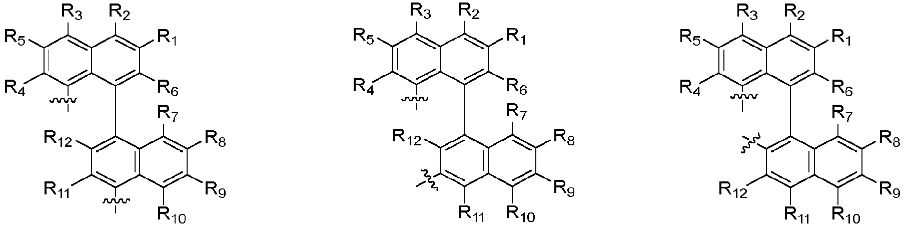 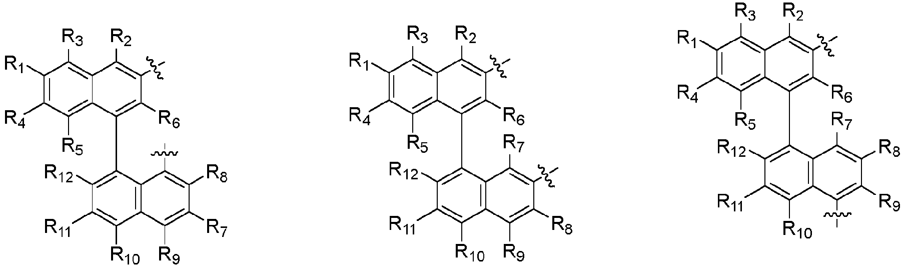 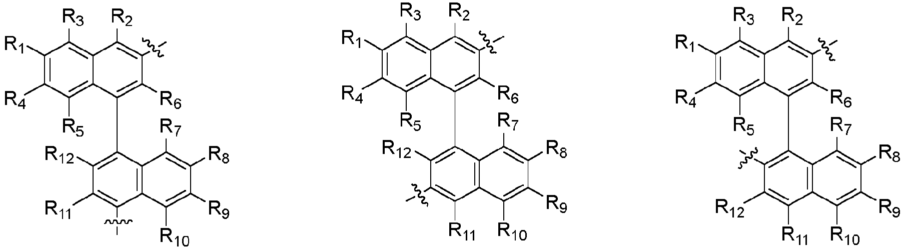 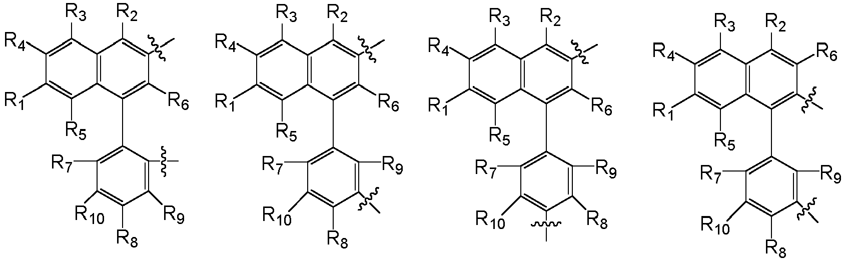 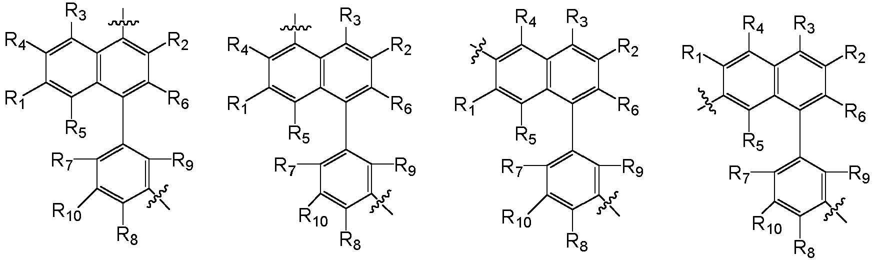 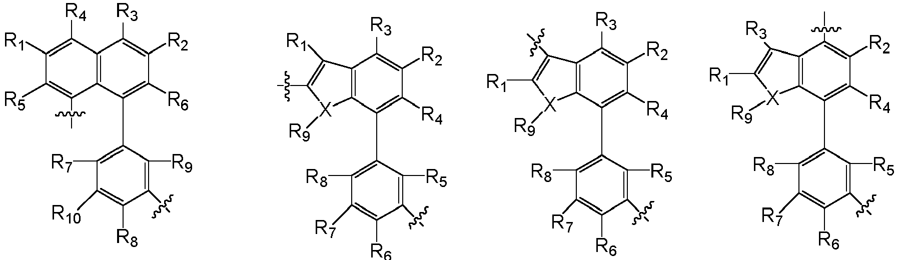 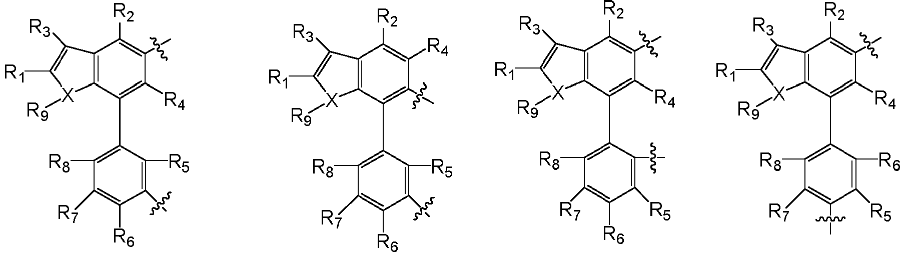 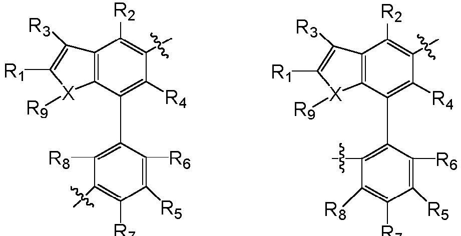 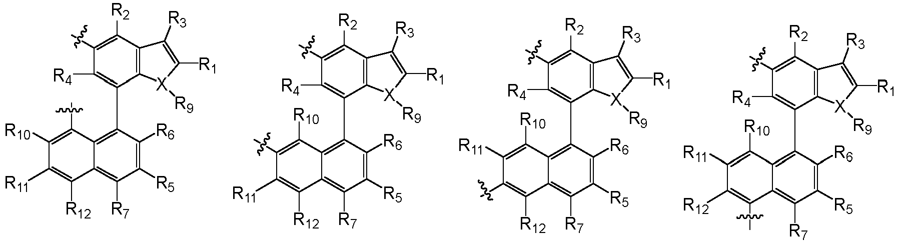 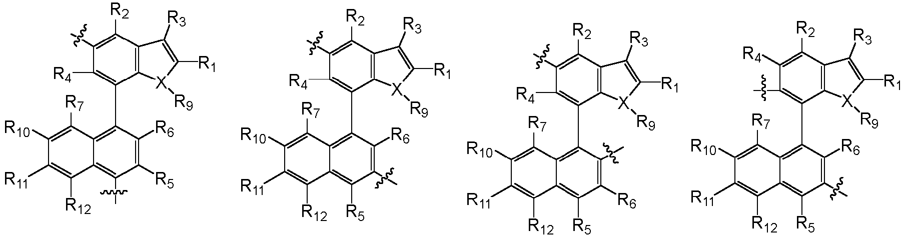 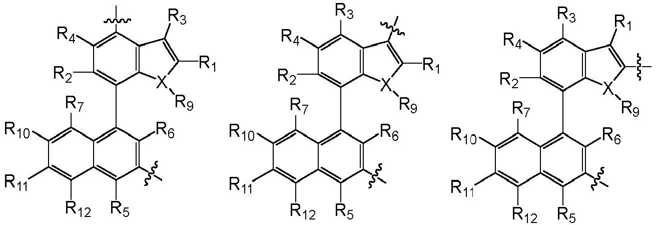 wherein X is S, Se, N, C, or O; each of R₁-R₁₂ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl; and each of the wavy lines represents one of the meta positions.
and
wherein each of the one or more Ars comprises one of a thiophene-based unit, a furan-based unit, a selenophene-based unit, or a pyrrole-based unit with one of the following formulas: 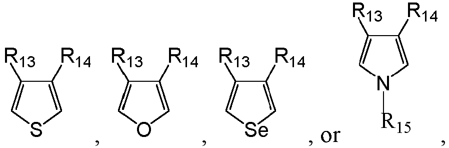 wherein each of R₁₃ ,R₁₄ and R₁₅ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl.

2. The electrochromic device of claim 1, wherein the electrochromic layer has transmittance of 40%-0.1% at a wavelength of 550 nm at an oxidized state of the electrochromic layer.

3. The electrochromic device of claim 1, further comprising an ion storage layer, wherein the ion storage layer is disposed between the electrolyte layer and the second conducting layer and has transmittance of 80% or more at a wavelength of 550 nm.

4. The electrochromic device of claim 3, wherein the ion storage layer comprises one or more oxides of metal elements in Group 4-12, or a mixture of the oxides, or one of the oxides doped by a different metal oxide, or a transition-metal complex, or one or more of redox-active polymers including redox active nitroxyl, galvinoxyl radical polymers and conjugated polymers.

5. The electrochromic device of claim 3, wherein the ion storage layer comprises indium tin oxide (ITO) particles, wherein the ion storage layer has transmittance of 90% or more at a wavelength of 550 nm.

6. The electrochromic device of claim 1, wherein at least one of the first conducting layer and the second conducting layer comprises ITO, aluminum zinc oxide (AZO), fluorine doped tin oxide (FTO), silver nanowires, graphene, carbon nanotube, metal mesh based transparent conductive electrodes, or silver-nanoparticle ink or an organic conductive polymer.

7. The electrochromic device of claim 1, wherein the electrochromic layer has an optical contrast of 60% or more.

8. The electrochromic device of claim 1, wherein the electrochromic device has an optical contrast of 60% or more.

9. The electrochromic device of claim 1, wherein a color of the electrochromic layer at an oxidized state is varied by varying a conjugation length of the one or more MCLs and the one or more Ars.

10. The electrochromic device of claim 1, wherein the electrochromic layer comprises a blend of the electrochromic polymers without an intermediate color.

11. The electrochromic device of any one of the preceding claims, wherein the thiophene-based unit comprises one of the following formulas: 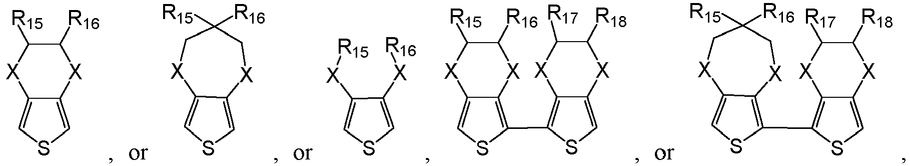 or 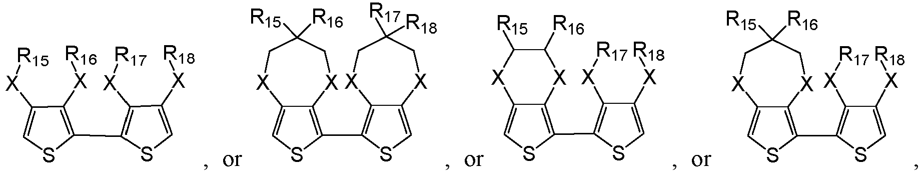 or 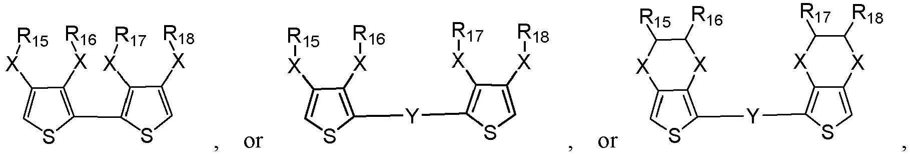 or 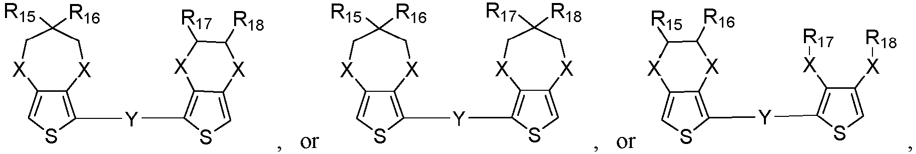 or 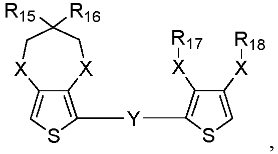 wherein X is S, Se, N, C, or O; each of R₁₅ - R₁₈ is independently selected from one of hydrogen, C₁-C₃₀ alkyl, C₂-C₃₀ alkenyl, C₂-C₃₀ alkynyl, C₂-C₃₀ alkylcarbonyl, C₁-C₃₀ alkoxy, C₃-C₃₀ alkoxyalkyl, C₂-C₃₀ alkoxycarbonyl, C₄-C₃₀ alkoxycarbonylalkyl, C₁-C₃₀ alkylthio, C₁-C₃₀ aminylcarbonyl, C₄-C₃₀ aminylalkyl, C₁-C₃₀ alkylaminyl, C₁-C₃₀ alkylsulfonyl, C₃-C₃₀ alkylsulfonylalkyl, C₆-C₁₈ aryl, C₃-C₁₅ cycloalkyl, C₃-C₃₀ cycloalkylaminyl, C₅-C₃₀ cycloalkylalkylaminyl, C₅-C₃₀ cycloalkylalkyl, C₅-C₃₀ cycloalkylalkyloxy, C₁-C₁₂ heterocyclyl, C₁-C₁₂ heterocyclyloxy, C₁-C₃₀ heterocyclylalkyloxy, C₁-C₃₀ heterocyclylaminyl, C₅-C₃₀ heterocyclylalkylaminyl, C₂-C₁₂ heterocyclylcarbonyl, C₃-C₃₀ heterocyclylalkyl, C₁-C₁₃ heteroaryl, or C₃-C₃₀ heteroarylalkyl; Y is any one or more of Ars, or aromatic structures, or fused aromatic structures, or a combinations thereof.

12. The electrochromic device of claim **1,** wherein the electrochromic polymer comprises a formula of 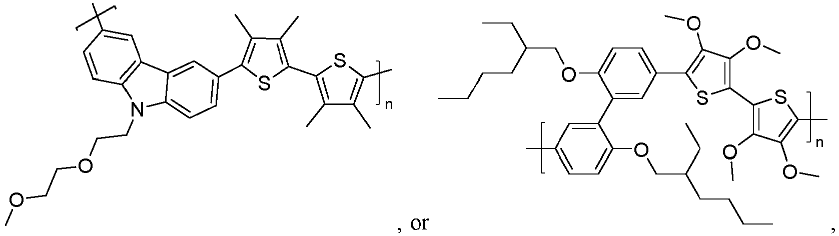 or 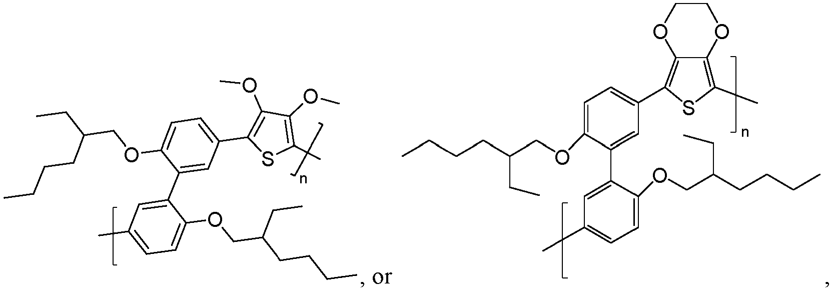 or 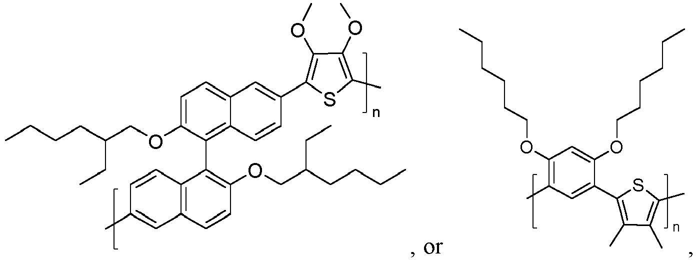 or 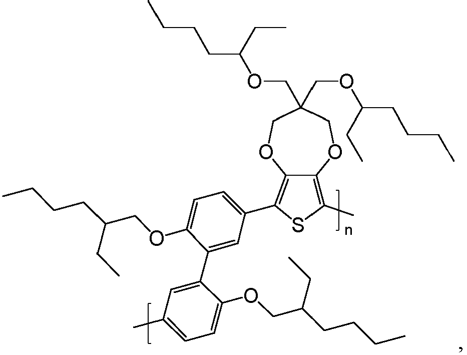 or 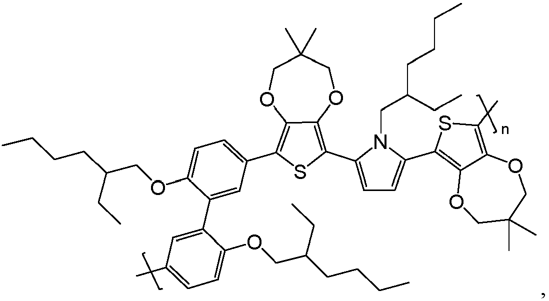 or 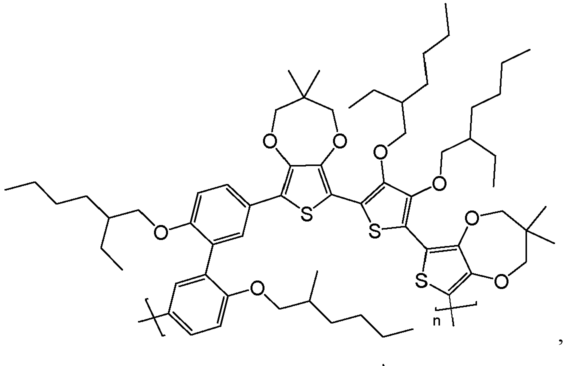 or 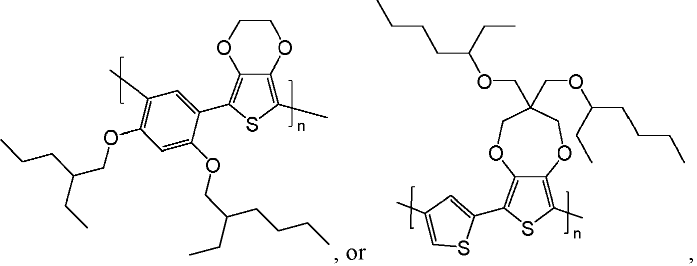 or 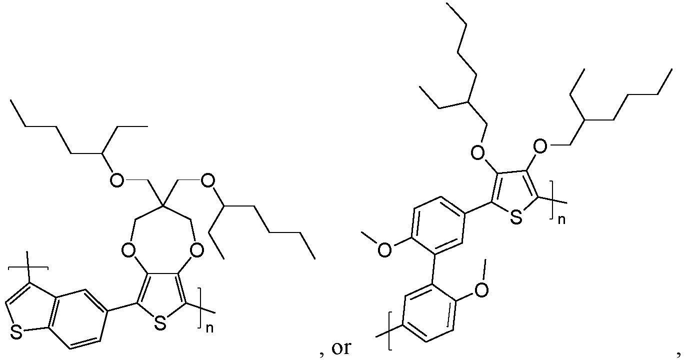 or 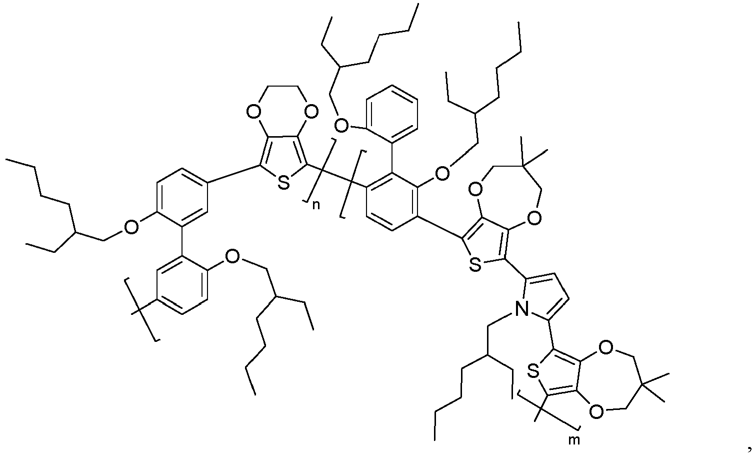 or 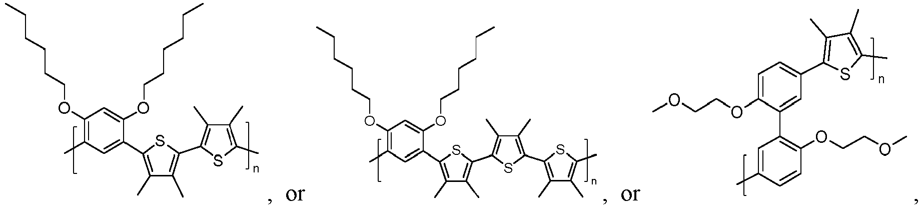 or 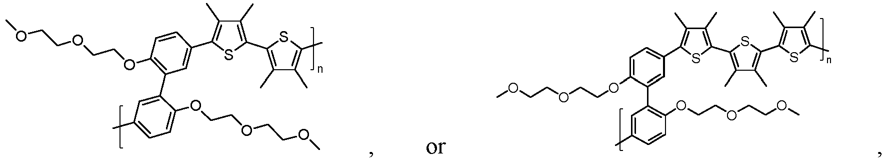 or 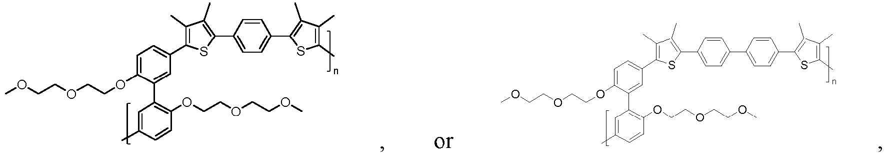 or 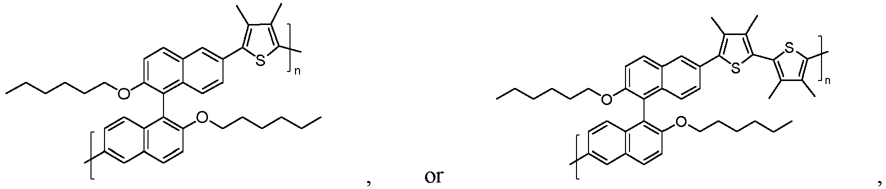 or 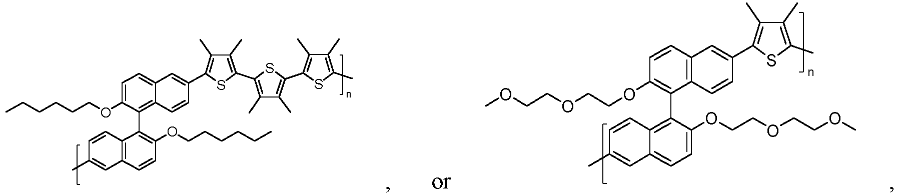 or 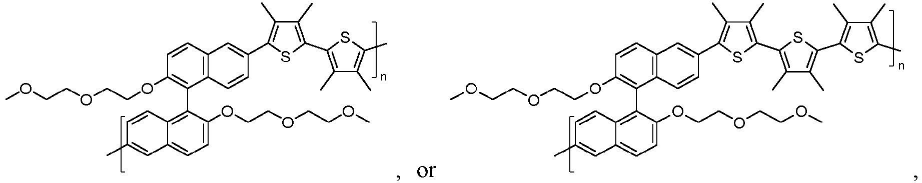 or 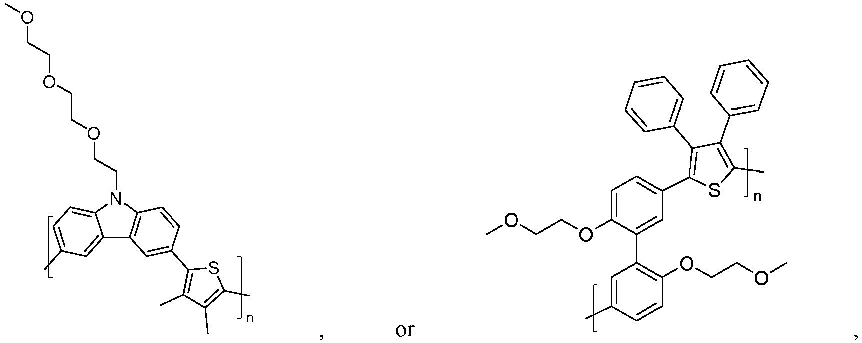 or 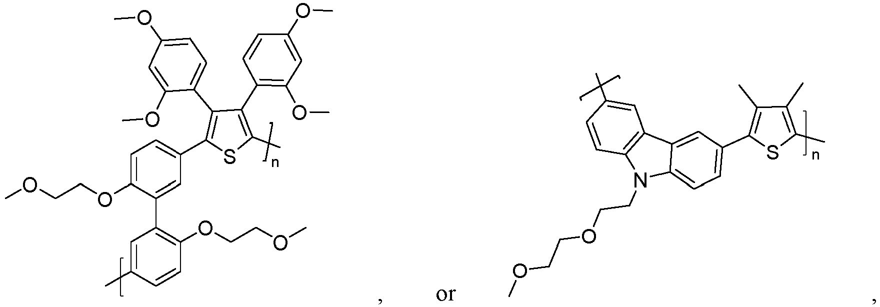 or 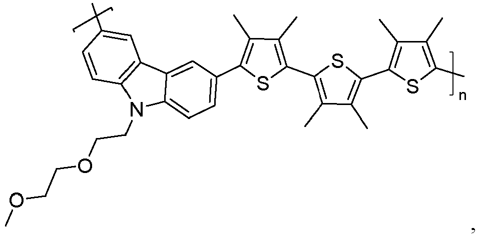 wherein n and m are integers greater than 0.

## Patentansprüche

1. Elektrochrome Vorrichtung, umfassend:
ein erstes isolierendes Substrat;
eine erste leitende Schicht, die über dem ersten isolierenden Substrat angeordnet ist;
eine elektrochrome Schicht, die über der ersten leitfähigen Schicht angeordnet ist, wobei die elektrochrome Schicht ein elektrochromes Polymer umfasst, das ein Polymergerüst aufweist, das einen oder mehrere meta-konjugierte Linker (MCLs) und eine oder mehrere aromatische Einheiten (Ars) umfasst, wobei jeder des einen oder mehrerer MCLs teilweise mit einem von der einen oder mehreren Ars an einer meta-Position des einen oder mehrerer MCLs konjugiert ist;
eine Elektrolytschicht, die über der elektrochromen Schicht angeordnet ist;
eine zweite leitende Schicht, die über der Elektrolytschicht angeordnet ist; und
ein zweites isolierendes Substrat, das über der zweiten leitenden Schicht angeordnet ist,
wobei eine Dicke der elektrochromen Schicht zwischen 10 nm und 1500 nm liegt, was zu einer Lichtdurchlässigkeit von 85 %-99,9 % bei einer Wellenlänge von 550 nm in einem neutralen Zustand der elektrochromen Schicht führt;
wobei die elektrochrome Vorrichtung bei einer Wellenlänge von 550 nm in einem gebleichten Zustand der Vorrichtung eine Lichtdurchlässigkeit von 60 % oder mehr aufweist;
wobei jeder von dem einen oder mehreren MCLs und die entsprechenden Meta-Positionen eine der folgenden Formeln umfassen: 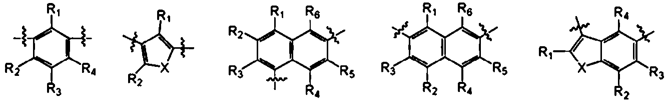 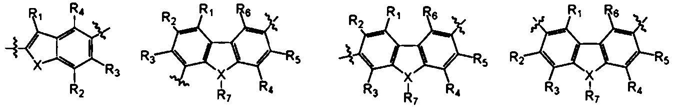 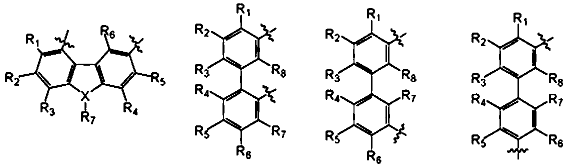 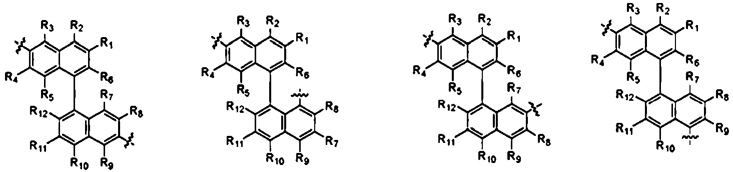 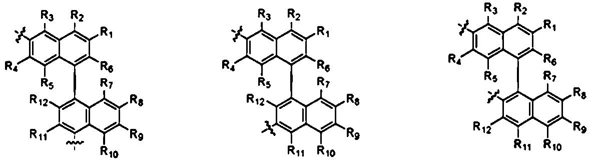 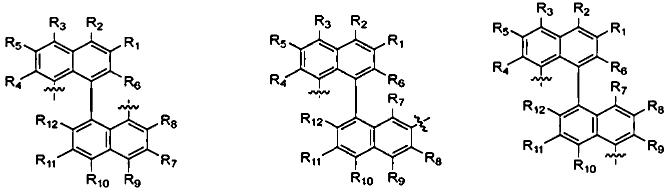 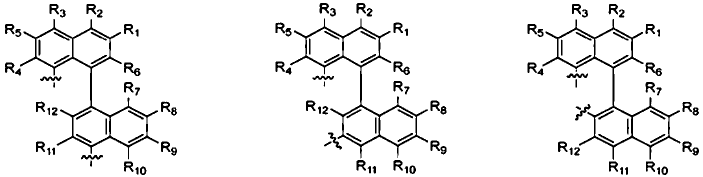 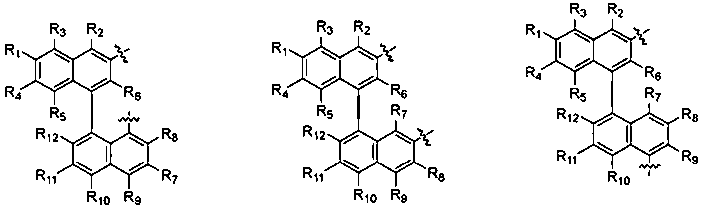 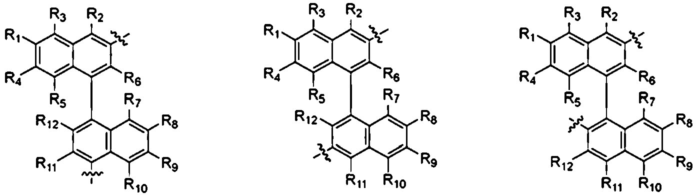 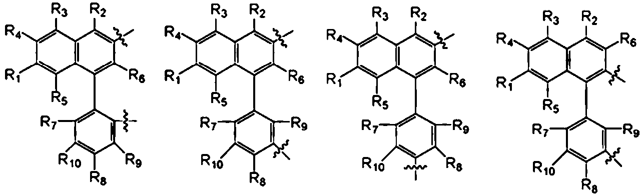 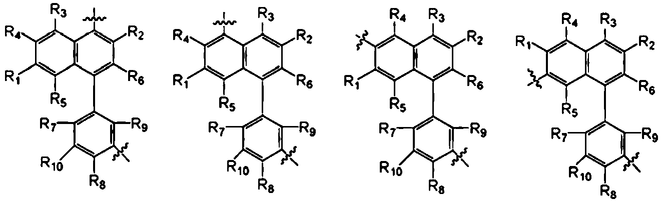 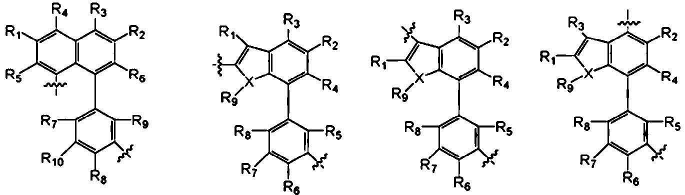 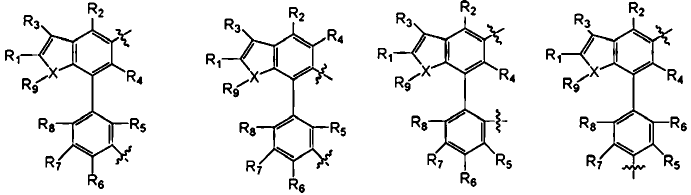 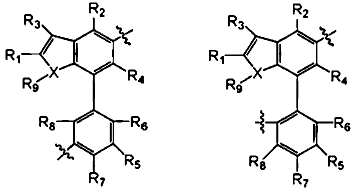 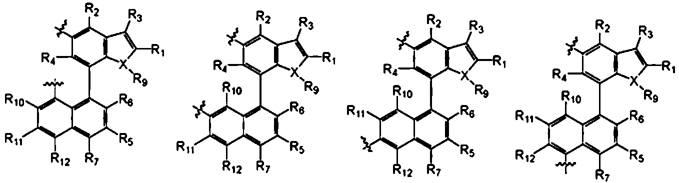 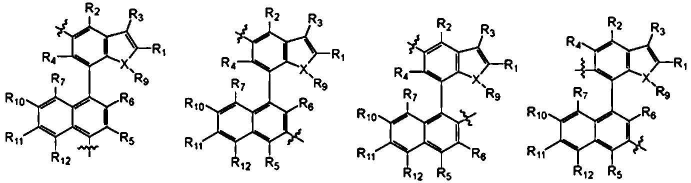 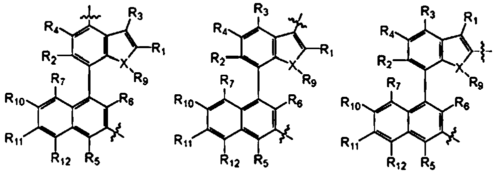
wobei X S, Se, N, C, oder O ist; jedes von R₁-R₁₂ unabhängig ausgewählt ist aus einem von Wasserstoff, C₁-C₃₀ Alkyl, C₂-C₃₀ Alkenyl, C₂-C₃₀ Alkinyl, C₂-C₃₀ Alkylcarbonyl, C₁-C₃₀ Alkoxy, C₃-C₃₀ Alkoxyalkyl, C₂-C₃₀ Alkoxycarbonyl, C₄-C₃₀ Alkoxycarbonylalkyl, C₁-C₃₀ Alkylthio, C₁-C₃₀ Aminylcarbonyl, C₄-C₃₀ Aminylalkyl, C₁-C₃₀ Alkylaminyl, C₁-C₃₀ Alkylsulfonyl, C₃-C₃₀ Alkylsulfonylalkyl, C₆-C₁₈ Aryl, C₃-C₁₅ Cycloalkyl, C₃-C₃₀ Cycloalkylaminyl, C₅-C₃₀ Cycloalkylalkylaminyl, C₅-C₃₀ Cycloalkylalkyl, C₅-C₃₀ Cycloalkylalkyloxy, C₁-C₁₂ Heterocyclyl, C₁-C₁₂ Heterocyclyloxy, C₁-C₃₀ Heterocyclylalkyloxy, C₁-C₃₀ Heterocyclylaminyl, C₅-C₃₀ Heterocyclylalkylaminyl, C₂-C₁₂ Heterocyclylcarbonyl, C₃-C₃₀ Heterocyclylalkyl, C₁-C₁₃ Heteroaryl, oder C₃-C₃₀ Heteroarylalkyl; und jede der Wellenlinien eine der meta-Positionen darstellt.
und
wobei jedes der einen oder mehreren Ars eine Thiophen-basierte Einheit, eine Furan-basierte Einheit, eine Selenophen-basierte Einheit oder eine Pyrrol-basierte Einheit mit einer der folgenden Formeln umfasst: 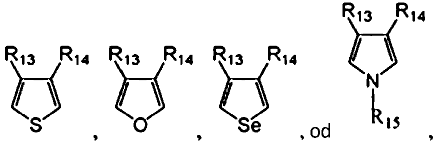
wobei jedes von R₁₃ R₁₄ und R₁₅ unabhängig ausgewählt ist aus einem von Wasserstoff, C₁-C₃₀ Alkyl, C₂-C₃₀ Alkenyl, C₂-C₃₀ Alkinyl, C₂-C₃₀ Alkylcarbonyl, C₁-C₃₀ Alkoxy, C₃-C₃₀ Alkoxyalkyl, C₂-C₃₀ Alkoxycarbonyl, C₄-C₃₀ Alkoxycarbonylalkyl, C₁-C₃₀ Alkylthio, C₁-C₃₀ Aminylcarbonyl, C₄-C₃₀ Aminylalkyl, C₁-C₃₀ Alkylaminyl, C₁-C₃₀ Alkylsulfonyl, C₃-C₃₀ Alkylsulfonylalkyl, C₆-C₁₈ Aryl, C₃-C₁₅ Cycloalkyl, C₃-C₃₀ Cycloalkylaminyl, C₅-C₃₀ Cycloalkylalkylaminyl, C₃-C₃₀ Cycloalkylalkyl, C₅-C₃₀ Cycloalkylalkyloxy, C₁-C₁₂ Heterocyclyl, C₁-C₁₂ Heterocyclyloxy, C₁-C₃₀ Heterocyclylalkyloxy, C₁-C₃₀ Heterocyclylaminyl, C₃-C₃₀ Heterocyclylalkylaminyl, C₂-C₁₂ Heterocyclylcarbonyl, C₃-C₃₀ Heterocyclylalkyl, C₁-C₁₃ Heteroaryl, oder C₃-C₃₀ Heteroarylalkyl.

2. Elektrochrome Vorrichtung nach Anspruch 1, wobei die elektrochrome Schicht eine Lichtdurchlässigkeit von 40% -0,1 % bei einer Wellenlänge von 550 nm im oxidierten Zustand der elektrochromen Schicht aufweist.

3. Elektrochrome Vorrichtung nach Anspruch 1, weiter umfassend eine Ionenspeicherschicht, wobei die Ionenspeicherschicht zwischen der Elektrolytschicht und der zweiten leitenden Schicht angeordnet ist und eine Lichtdurchlässigkeit von 80 % oder mehr bei einer Wellenlänge von 550 nm aufweist.

4. Elektrochrome Vorrichtung nach Anspruch 3, wobei die Ionenspeicherschicht ein oder mehrere Oxide von Metallelementen der Gruppen 4-12 oder ein Gemisch der Oxide oder eines der Oxide, das mit einem anderen Metalloxid oder einem Übergangsmetallkomplex dotiert ist, oder ein oder mehrere redoxaktive Polymere, die redoxaktive Nitroxyl-, Galvinoxyl-Radikalpolymere und konjugierte Polymere beinhalten, umfasst.

5. Elektrochrome Vorrichtung nach Anspruch 3, wobei die Ionenspeicherschicht Indiumzinnoxid-, (ITO)-Partikel umfasst und die Ionenspeicherschicht bei einer Wellenlänge von 550 nm eine Lichtdurchlässigkeit von 90 % oder mehr aufweist.

6. Elektrochrome Vorrichtung nach Anspruch 1, wobei mindestens eine von der ersten leitenden Schicht und der zweiten leitenden Schicht ITO, Aluminium-Zink-Oxid (AZO), fluor-dotiertes Zinnoxid (FTO), Silbernanodrähte, Graphen, Kohlenstoffnanoröhren, auf Metallgewebe basierende transparente leitende Elektroden oder Silbernanopartikel-Tinte oder ein organisches leitendes Polymer umfasst.

7. Elektrochrome Vorrichtung nach Anspruch 1, wobei die elektrochrome Schicht einen optischen Kontrast von 60 % oder mehr aufweist.

8. Elektrochrome Vorrichtung nach Anspruch 1, wobei die elektrochrome Vorrichtung einen optischen Kontrast von 60 % oder mehr aufweist.

9. Elektrochrome Vorrichtung nach Anspruch 1, wobei eine Farbe der elektrochromen Schicht in einem oxidierten Zustand durch Variieren der Konjugationslänge des einen oder mehrerer MCLs und der einen oder mehrerer Ars variiert wird.

10. Elektrochrome Vorrichtung nach Anspruch 1, wobei die elektrochrome Schicht eine Mischung der elektrochromen Polymere ohne Zwischenfarbe umfasst.

11. Elektrochrome Vorrichtung nach einem der vorstehenden Ansprüche, wobei die Thiophen-basierte Einheit eine der folgenden Formeln umfasst: 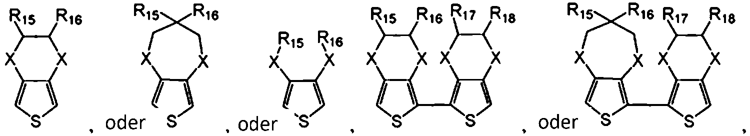 oder 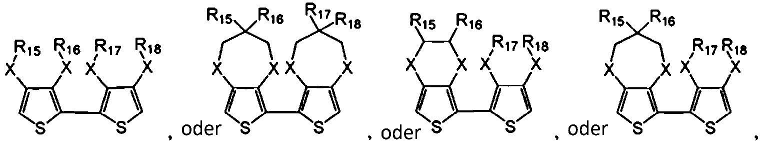 oder 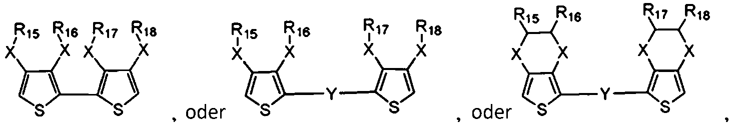 oder 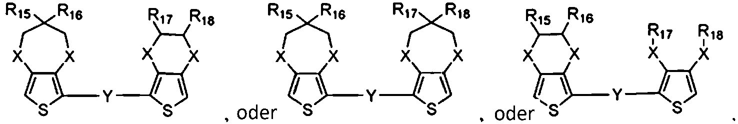 oder 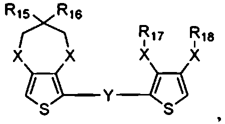 wobei X S, Se, N, C, oder O ist; jedes von R₁₅ - R₁₈ unabhängig ausgewählt ist aus einem von Wasserstoff, C₁-C₃₀ Alkyl, C₂-C₃₀ Alkenyl, C₂-C₃₀ Alkinyl, C₂-C₃₀ Alkylcarbonyl, C₁-C₃₀ Alkoxy, C₃-C₃₀ Alkoxyalkyl, C₂-C₃₀ Alkoxycarbonyl, C₄-C₃₀ Alkoxycarbonylalkyl, C₁-C₃₀ Alkylthio, C₁-C₃₀ Aminylcarbonyl, C₄-C₃₀ Aminylalkyl, C₁-C₃₀ Alkylaminyl, C₁-C₃₀ Alkylsulfonyl, C₃-C₃₀ Alkylsulfonylalkyl, C₆-C₁₈ Aryl, C₃-C₁₅ Cycloalkyl, C₃-C₃₀ Cycloalkylaminyl, C₅-C₃₀ Cycloalkylalkylaminyl, C₅-C₃₀ Cycloalkylalkyl, C₅-C₃₀ Cycloalkylalkyloxy, C₁-C₁₂ Heterocyclyl, C₁-C₁₂ Heterocyclyloxy, C₁-C₃₀ Heterocyclylalkyloxy, C₁-C₃₀ Heterocyclylaminyl, C₅-C₃₀ Heterocyclylalkylaminyl, C₂-C₁₂ Heterocyclylcarbonyl, C₃-C₃₀ Heterocyclylalkyl, C₁-C₁₃ Heteroaryl, oderr C₃-C₃₀ Heteroarylalkyl; Y eine oder mehrere von Arsen-Verbindungen, aromatischen Strukturen, kondensierten aromatischen Strukturen oder Kombinationen davon ist.

12. Elektrochrome Vorrichtung nach Anspruch 1, wobei das elektrochrome Polymer eine Formel umfasst von 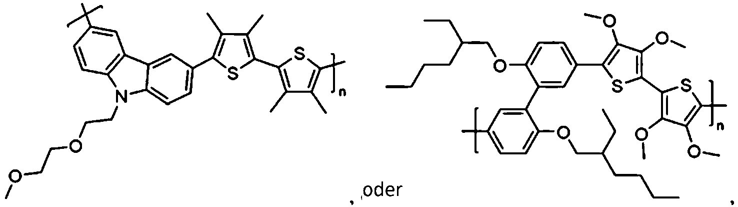 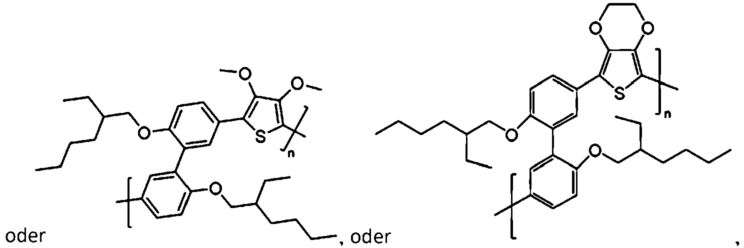 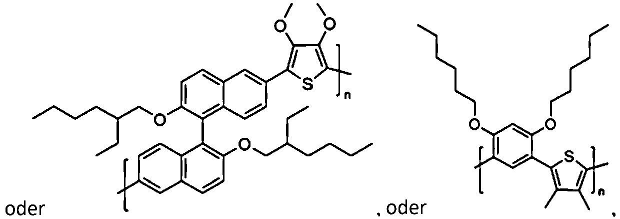 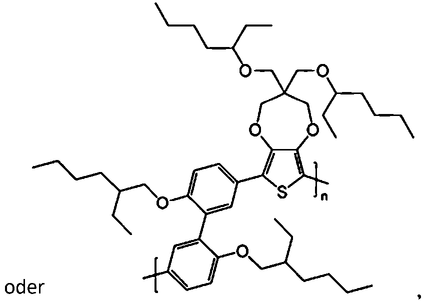 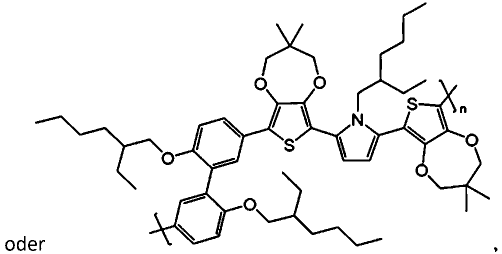 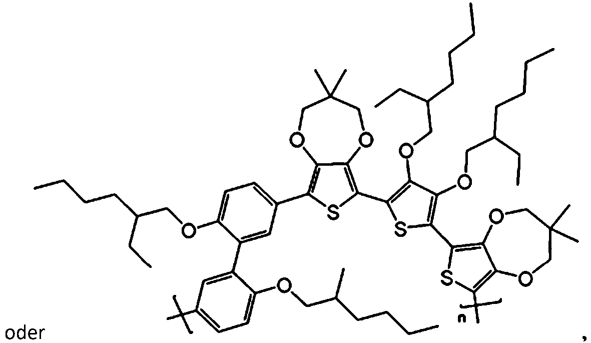 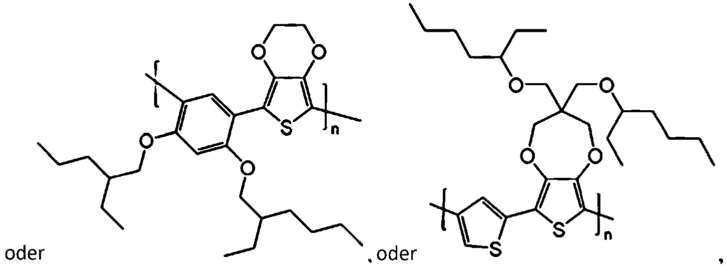 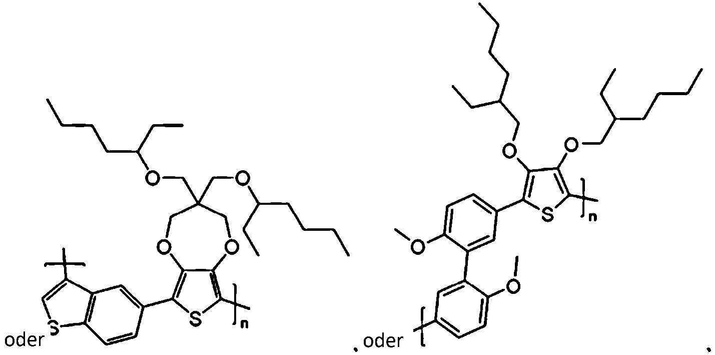 or 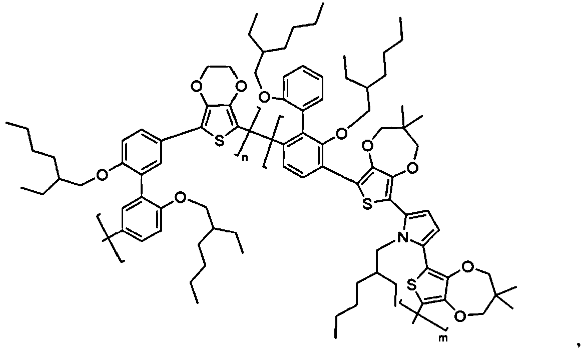 oder 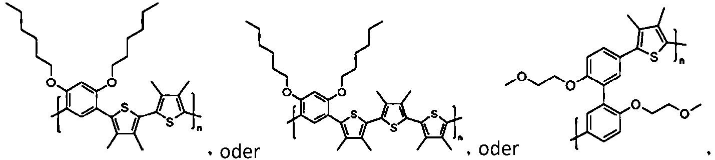 oder 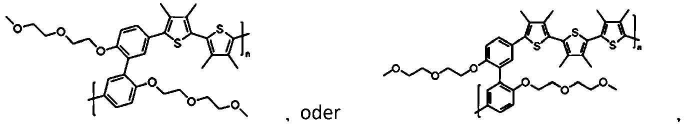 oder 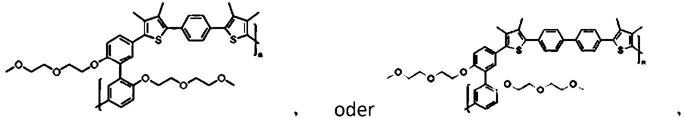 oder 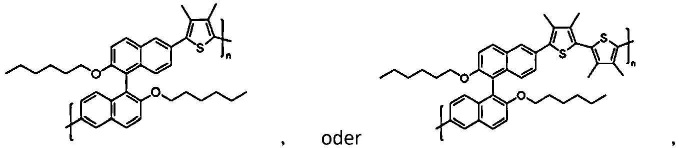 oder 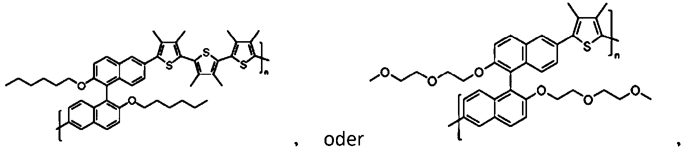 oder 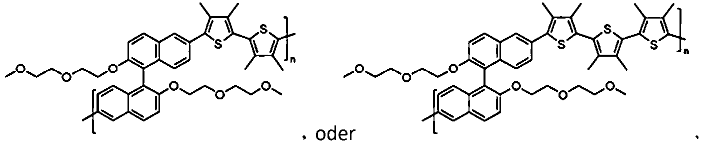 oder 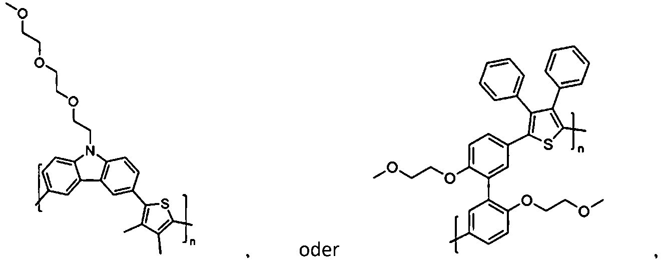 oder 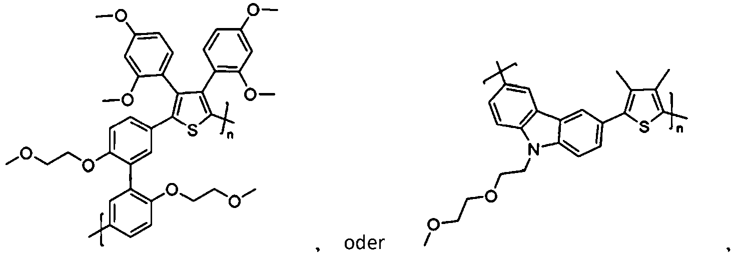 oder 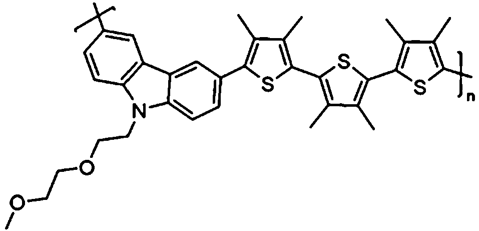 wobei n und m ganze Zahlen größer als 0 sind.

## Revendications

1. Dispositif électrochrome, comprenant :
un premier substrat isolant ;
une première couche conductrice disposée sur le premier substrat isolant ;
une couche électrochrome disposée sur la première couche conductrice, dans lequel la couche électrochrome comprend un polymère électrochrome présentant une chaîne principale de polymère comprenant un ou plusieurs coupleurs méta-conjugués (MCL) et un ou plusieurs fragments aromatiques (Ars), dans lequel chacun des un ou plusieurs MCL est partiellement conjugué avec l'un des un ou plusieurs Ars à une position méta des un ou plusieurs MCL ;
une couche d'électrolyte disposée sur la couche électrochrome ;
une seconde couche conductrice disposée sur la couche d'électrolyte ; et
un second substrat isolant disposé sur la seconde couche conductrice,
dans lequel une épaisseur de la couche électrochrome est de 10 nm à 1 500 nm, ce qui entraîne une transmittance de 85 % à 99,9 % à une longueur d'onde de 550 nm à un état neutre de la couche électrochrome ;
dans lequel le dispositif électrochrome présente une transmittance de 60 % ou plus à une longueur d'onde de 550 nm à un état blanchi du dispositif ;
dans lequel chacun des un ou plusieurs MCL et des positions méta correspondantes comprennent l'une des formules suivantes : 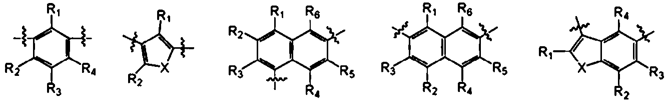 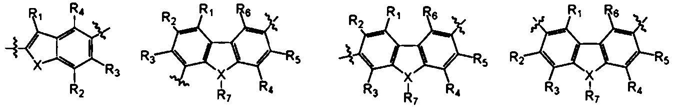 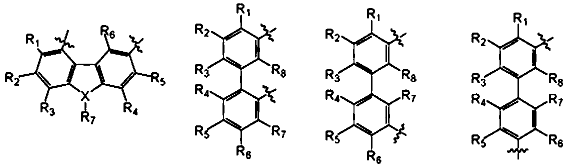 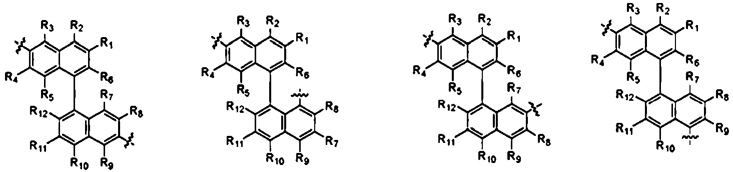 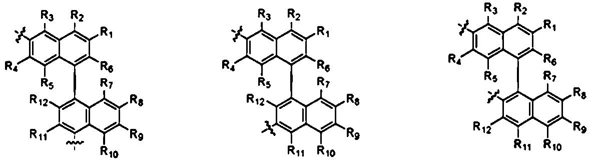 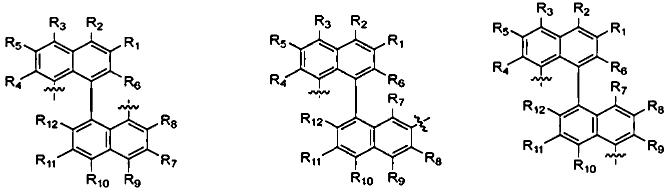 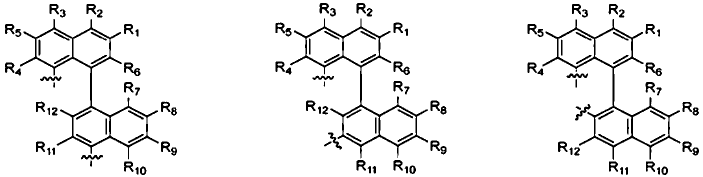 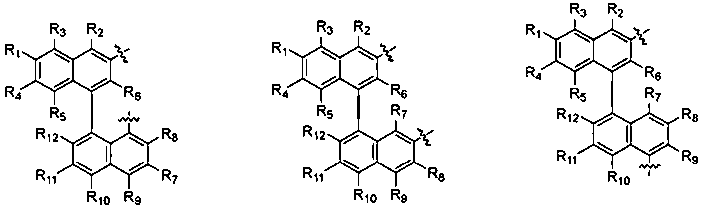 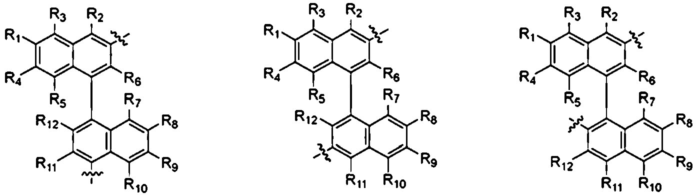 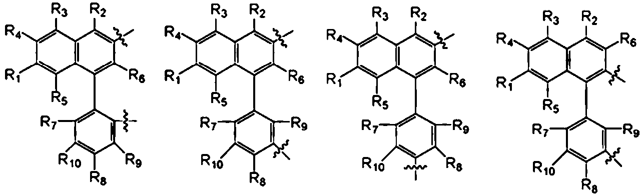 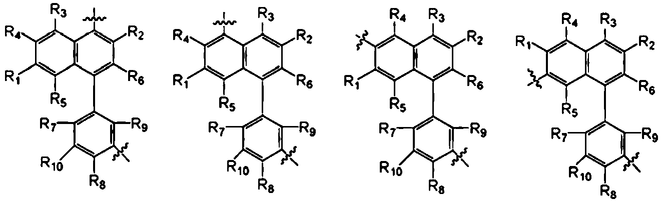 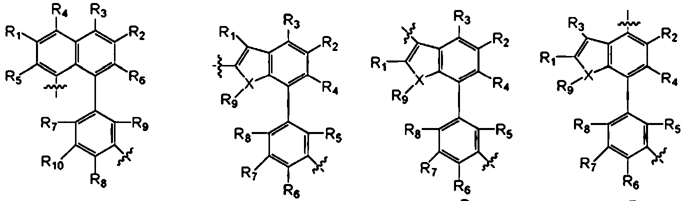 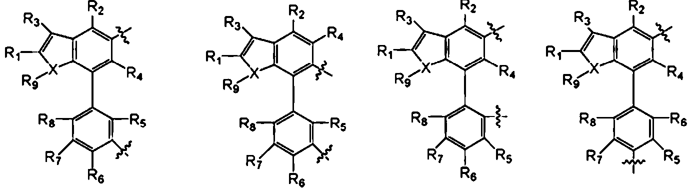 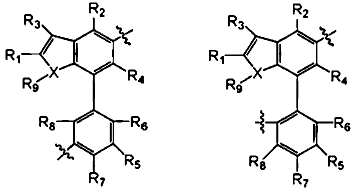 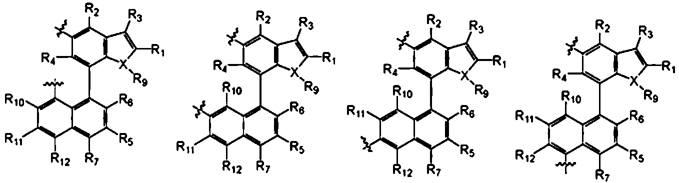 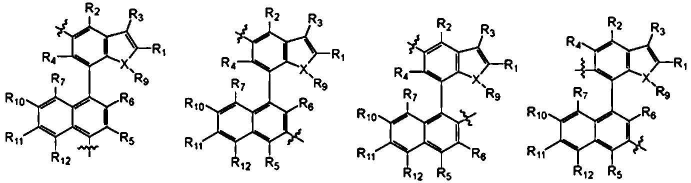 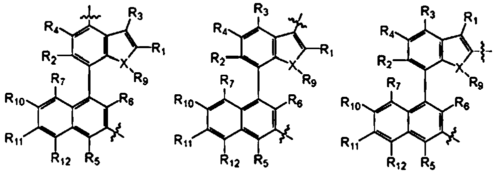
dans lesquelles X est S, Se, N, C ou O ; chacun de R₁-R₁₂ est choisi indépendamment parmi hydrogène, alkyle en C₁-C₃₀, alcényle en C₂-C₃₀, alcynyle en C₂-C₃₀, alkylcarbonyle en C₂-C₃₀, alcoxy en C₁-C₃₀, alcoxyalkyle en C₃-C₃₀, alcoxycarbonyle en C₂-C₃₀, alcoxycarbonylalkyle en C₄-C₃₀, alkylthio en C₁-C₃₀, aminylcarbonyle en C₁-C₃₀, aminylalkyle en C₄-C₃₀, alkylaminyle en C₁-C₃₀, alkylsulfonyle en C₁-C₃₀, alkylsulfonylalkyle en C₃-C₃₀, aryle en C₆-C₁₈, cycloalkyle en C₃-C₁₅, cycloalkylaminyle en C₃-C₃₀, cycloalkylalkylaminyle en C₅-C₃₀, cycloalkylalkyle en C₅-C₃₀, cycloalkylalkyloxy en C₅-C₃₀, hétérocyclyle en C₁-C₁₂, hétérocyclyloxy en C₁-C₁₂, hétérocyclylalkyloxy en C₁-C₃₀, hétérocyclylaminyle en C₁-C₃₀, hétérocyclylalkylaminyle en C₅-C₃₀, hétérocyclylcarbonyle en C₂-C₁₂, hétérocyclylalkyle en C₃-C₃₀, hétéroaryle en C₁-C₁₃ ou hétéroarylalkyle en C₃-C₃₀ ; et chacune des lignes ondulées représente l'une des positions méta
et
dans lesquelles chacun des un ou plusieurs Ars comprend une parmi une unité à base de thiophène, une unité à base de furane, une unité à base de sélénophène ou une unité à base de pyrrole avec l'une des formules suivantes : 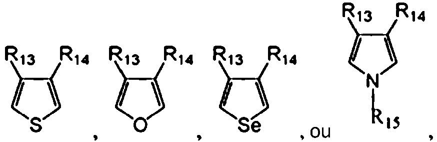
dans lesquelles chacun de R₁₃, R₁₄ et R₁₃ est choisi indépendamment parmi hydrogène, alkyle en C₁-C₃₀, alcényle en C₂-C₃₀, alcynyle en C₂-C₃₀, alkylcarbonyle en C₂-C₃₀, alcoxy en C₁-C₃₀, alcoxyalkyle en C₃-C₃₀, alcoxycarbonyle en C₂-C₃₀, alcoxycarbonylalkyle en C₄-C₃₀, alkylthio en C₁-C₃₀, aminylcarbonyle en C₁-C₃₀, aminylalkyle en C₄-C₃₀, alkylaminyle en C₁-C₃₀, alkylsulfonyle en C₁-C₃₀, alkylsulfonylalkyle en C₃-C₃₀, aryle en C₆-C₁₈, cycloalkyle en C₃-C₁₅, cycloalkylaminyle en C₃-C₃₀, cycloalkylalkylaminyle en C₅-C₃₀, cycloalkylalkyle en C₅-C₃₀, cycloalkylalkyloxy en C₅-C₃₀, hétérocyclyle en C₁-C₁₂, hétérocyclyloxy en C₁-C₁₂, hétérocyclylalkyloxy en C₁-C₃₀, hétérocyclylaminyle en C₁-C₃₀, hétérocyclylalkylaminyle en C₅-C₃₀, hétérocyclylcarbonyle en C₂-C₁₂, hétérocyclylalkyle en C₃-C₃₀, hétéroaryle en C₁-C₁₃ ou hétéroarylalkyle en C₃-C₃₀.

2. Dispositif électrochrome selon la revendication 1, dans lequel la couche électrochrome présente une transmittance de 40 % à 0,1 % à une longueur d'onde de 550 nm à un état oxydé de la couche électrochrome.

3. Dispositif électrochrome selon la revendication 1, comprenant en outre une couche de stockage d'ions, dans lequel la couche de stockage d'ions est disposée entre la couche d'électrolyte et la seconde couche conductrice et présente une transmittance de 80 % ou plus à une longueur d'onde de 550 nm.

4. Dispositif électrochrome selon la revendication 3, dans lequel la couche de stockage d'ions comprend un ou plusieurs oxydes d'éléments métalliques dans les groupes 4 à 12, ou un mélange des oxydes, ou l'un des oxydes dopé par un oxyde métallique différent, ou un complexe de métal de transition, ou un ou plusieurs parmi des polymères rédox-actifs incluant des polymères à radicaux nitroxyle, galvinoxyle et des polymères conjugués rédox-actifs.

5. Dispositif électrochrome selon la revendication 3, dans lequel la couche de stockage d'ions comprend des particules d'oxyde d'indium-étain (ITO), dans lequel la couche de stockage d'ions présente une transmittance de 90 % ou plus à une longueur d'onde de 550 nm.

6. Dispositif électrochrome selon la revendication 1, dans lequel au moins une parmi la première couche conductrice et la seconde couche conductrice comprend de l'ITO, de l'oxyde d'aluminium-zinc (AZO), de l'oxyde d'étain dopé au fluor (FTO), des nanofils d'argent, du graphène, un nanotube de carbone, des électrodes conductrices transparentes à base de treillis métallique, ou une encre à nanoparticules d'argent ou un polymère conducteur organique.

7. Dispositif électrochrome selon la revendication 1, dans lequel la couche électrochrome présente un contraste optique de 60 % ou plus.

8. Dispositif électrochrome selon la revendication 1, dans lequel le dispositif électrochrome présente un contraste optique de 60 % ou plus.

9. Dispositif électrochrome selon la revendication 1, dans lequel une couleur de la couche électrochrome à un état oxydé varie en faisant varier une longueur de conjugaison des un ou plusieurs MCL et des un ou plusieurs Ars.

10. Dispositif électrochrome selon la revendication 1, dans lequel la couche électrochrome comprend un mélange des polymères électrochromes sans couleur intermédiaire.

11. Dispositif électrochrome selon l'une quelconque des revendications précédentes, dans lequel l'unité à base de thiophène comprend l'une des formules suivantes : 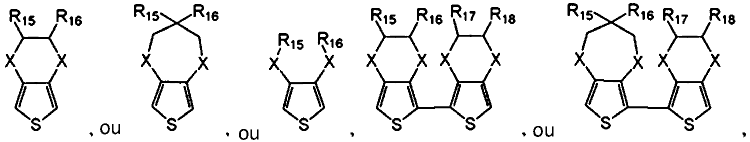 ou 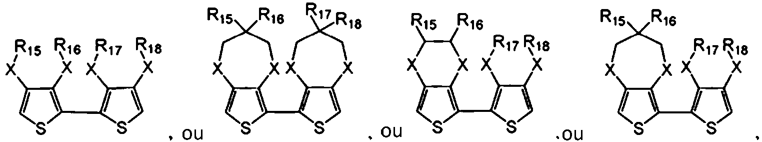 ou 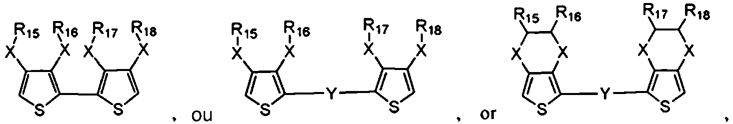 ou 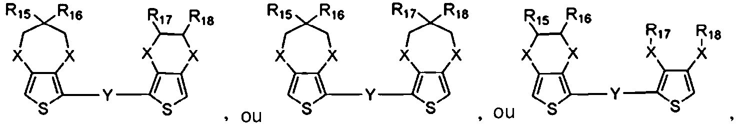 ou 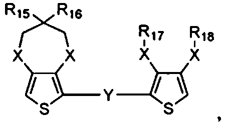 dans lesquelles X est S, Se, N, C ou O ; chacun de R₁₅-R₁₈ est choisi indépendamment parmi hydrogène, alkyle en C₁-C₃₀, alcényle en C₂-C₃₀, alcynyle en C₂-C₃₀, alkylcarbonyle en C₂-C₃₀, alcoxy en C₁-C₃₀, alcoxyalkyle en C₃-C₃₀, alcoxycarbonyle en C₂-C₃₀, alcoxycarbonylalkyle en C₄-C₃₀, alkylthio en C₁-C₃₀, aminylcarbonyle en C₁-C₃₀, aminylalkyle en C₄-C₃₀, alkylaminyle en C₁-C₃₀, alkylsulfonyle en C₁-C₃₀, alkylsulfonylalkyle en C₃-C₃₀, aryle en C₆-C₁₈, cycloalkyle en C₃-C₁₅, cycloalkylaminyle en C₃-C₃₀, cycloalkylalkylaminyleou en C₅-C₃₀, cycloalkylalkyle en C₅-C₃₀, cycloalkylalkyloxy en C₅-C₃₀, hétérocyclyle en C₁-C₁₂, hétérocyclyloxy en C₁-C₁₂, hétérocyclylalkyloxy en C₁-C₃₀, hétérocyclylaminyle en C₁-C₃₀, hétérocyclylalkylaminyle en C₅-C₃₀, hétérocyclylcarbonyle en C₂-C₁₂, hétérocyclylalkyle en C₃-C₃₀, hétéroaryle en C₁-C₁₃ ou hétéroarylalkyle en C₃-C₃₀; Y est l'un quelconque ouou plusieurs parmi des Ars, ou des structures aromatiques, ou des structures aromatiques fusionnées, ou une combinaison de ceux-ci.

12. Dispositif électrochrome selon la revendication 1, dans lequel le polymère électrochrome comprend une formule parmi 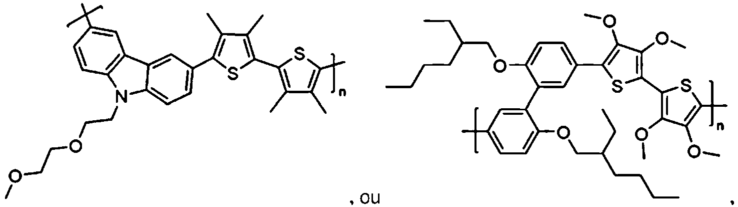 ou 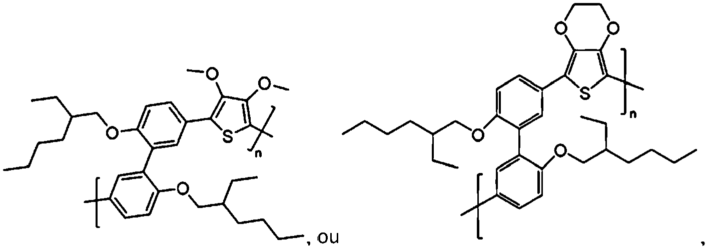 ou 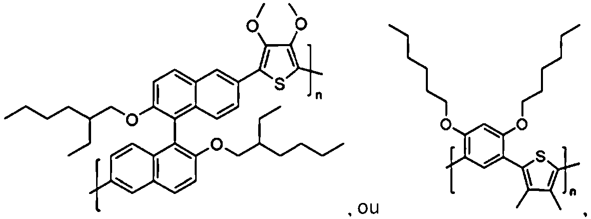 ou 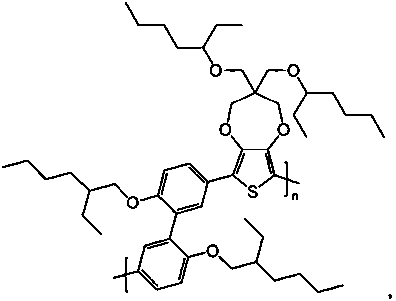 ou 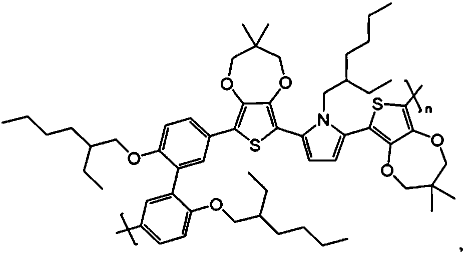 ou 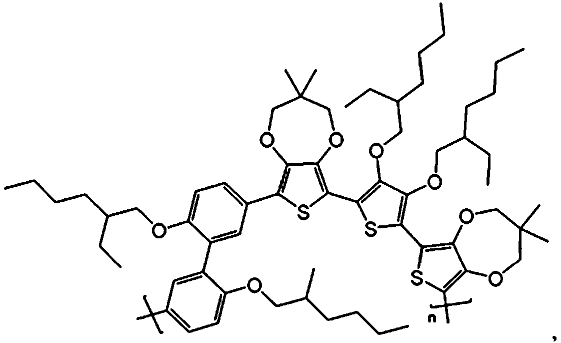 ou 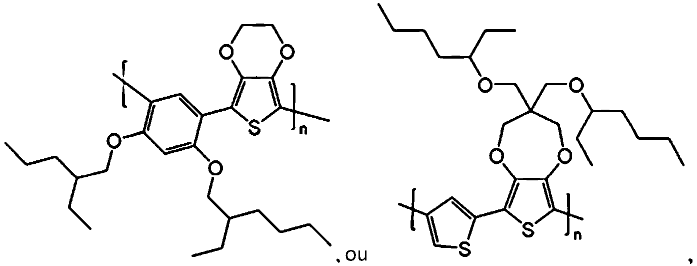 ou 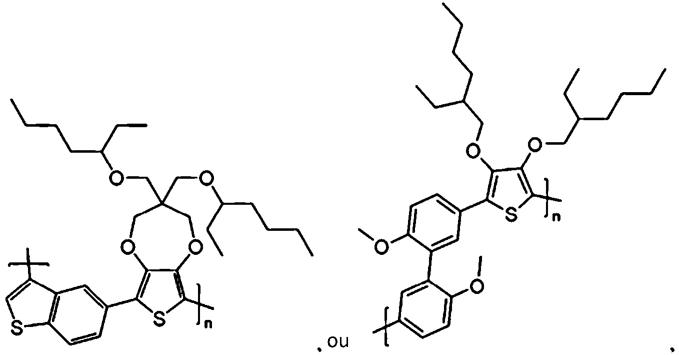 or 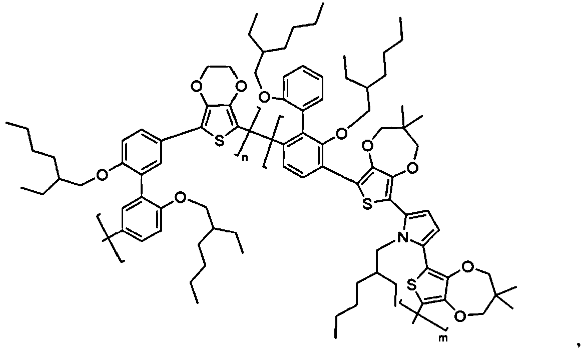 ou 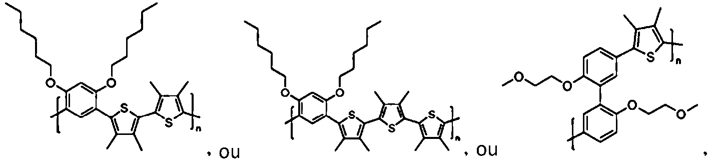 ou 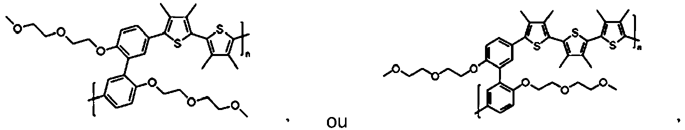 ou 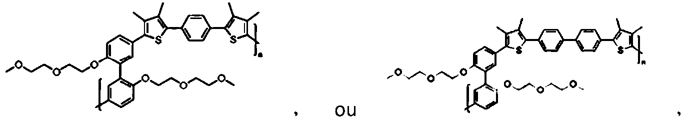 ou 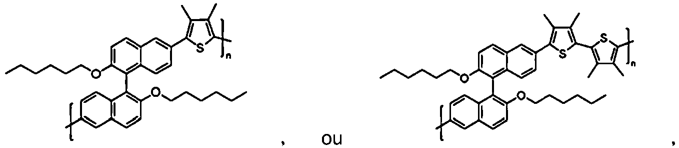 ou 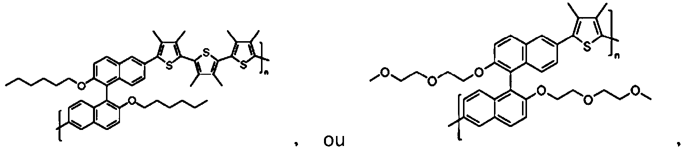 ou 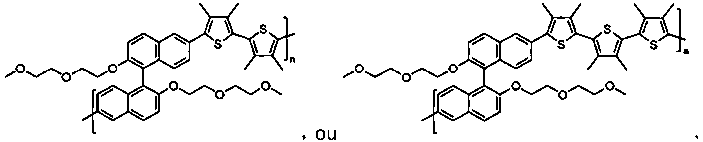 ou 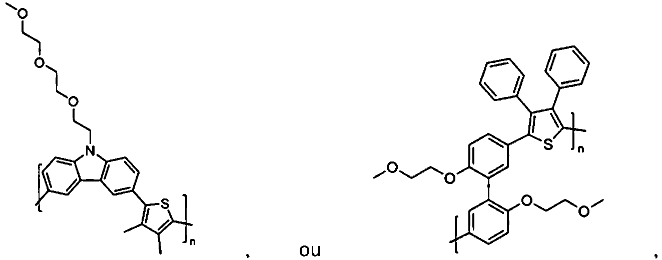 ou 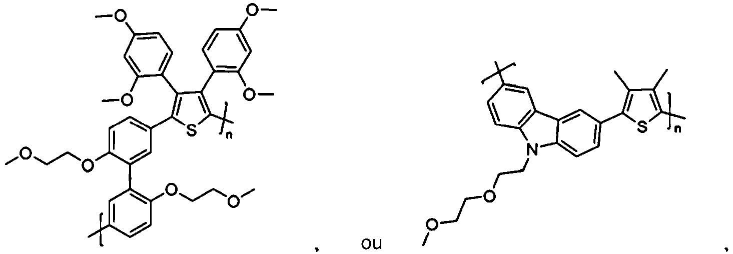 ou 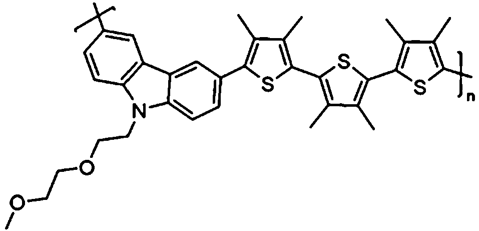 , dans lesquelles n et m sont des entiers supérieurs à 0.
